# EUROPEAN PATENT SPECIFICATION

(11) **EP 4 341 442 B1**
(45) Date of publication and mention of the grant of the patent: **11.11.2026**
(21) Application number: 22730193.4
(22) Date of filing: 20.05.2022
(51) Int. Cl.: C12Q 1/6886

(54) **MOLECULAR SIGNATURE FOR ASSESSING THE RESPONSIVENESS OF CANCER TO MITOCHONDRIA-TARGETED ANTIOXIDANTS**
MOLEKULARE SIGNATUR ZUR BEURTEILUNG DES ANSPRECHENS VON KREBS AUF AUF MITOCHONDRIEN GERICHTETE ANTIOXIDATIONSMITTEL
SIGNATURE MOLÉCULAIRE POUR ÉVALUER LA RÉACTIVITÉ D'UN CANCER À DES ANTIOXYDANTS CIBLANT LES MITOCHONDRIES

(30) Priority: 21.05.2021 EP 21175397
(43) Date of publication of application: 27.03.2024
(73) Proprietor: UNIVERSITE CATHOLIQUE DE LOUVAIN, 1348 Louvain-la-Neuve (BE)
(72) Inventor: SONVEAUX, Pierre, 1933 Sterrebeek (BE); DE MIRANDA CAPELOA, Tania, 1030 Schaerbeek (BE)
(74) Representative: Icosa
(86) International application number: PCT/EP2022/063789
(87) International publication number: WO 2022/243541

(56) References cited:
- US-A1- 2008 032 940
- PORPORATO PAOLO E. ET AL: "A Mitochondrial Switch Promotes Tumor Metastasis", CELL REPORTS, vol. 8, no. 3, 1 August 2014 (2014-08-01), US, pages 754 - 766, XP055858106, ISSN: 2211-1247, DOI: 10.1016/j.celrep.2014.06.043
- GRASSO DEBORA ET AL: "Mitochondria in cancer", CELL STRESS, vol. 4, no. 6, 8 June 2020 (2020-06-08), pages 114 - 146, XP055858097, DOI: 10.15698/cst2020.06.221
- GONZALEZ YANIRA ET AL: "Atg7-and Keap1-dependent autophagy protects breast cancer cell lines against mitoquinone-induced oxidative stress", ONCOTARGET, IMPACT JOURNALS LLC, UNITED STATES, vol. 5, no. 6, 30 March 2014 (2014-03-30), pages 1526 - 1537, XP009531080, ISSN: 1949-2553, DOI: 10.18632/ONCOTARGET.1715
- CHENG MENG-HSUAN ET AL: "BA6 Induces Apoptosis via Stimulation of Reactive Oxygen Species and Inhibition of Oxidative Phosphorylation in Human Lung Cancer Cells", OXIDATIVE MEDICINE AND CELLULAR LONGEVITY, vol. 2019, 7 May 2019 (2019-05-07), US, pages 1 - 21, XP055857965, ISSN: 1942-0900, Retrieved from the Internet <URL:https://downloads.hindawi.com/journals/omcl/2019/6342104.pdf> DOI: 10.1155/2019/6342104
- DANHIER PIERRE ET AL: "Cancer metabolism in space and time: Beyond the Warburg effect", BIOCHIMICA ET BIOPHYSICA ACTA. BIOENERGETICS, AMSTERDAM, NL, vol. 1858, no. 8, 4 February 2017 (2017-02-04), pages 556 - 572, XP085107703, ISSN: 0005-2728, DOI: 10.1016/J.BBABIO.2017.02.001
- QU JIAGUI ET AL: "Melatonin treatment increases the transcription of cell proliferation-related genes prior to inducing cell death in C6 glioma cells in vitro", ONCOLOGY LETTERS, vol. 6, no. 2, 18 June 2013 (2013-06-18), GR, pages 347 - 352, XP055955984, ISSN: 1792-1074, DOI: 10.3892/ol.2013.1413
- KOÇAK NADIR ET AL: "Effects of melatonin on apoptosis and cell differentiation in MCF-7 derived cancer stem cells", CELLULAR AND MOLECULAR BIOLOGY, vol. 64, no. 12, 30 September 2018 (2018-09-30), FR, pages 56 - 61, XP055956017, ISSN: 0145-5680, Retrieved from the Internet <URL:https://cellmolbiol.org/index.php/CMB/article/download/1727/1315> DOI: 10.14715/cmb/2018.64.12.12
- ANONYMOUS: "GeneAnnot results for the Gene Symbol CHMP5 for HG-U95, HG-U133 and HG-U133 Plus 2.0", 1 October 2015 (2015-10-01), XP055217523, Retrieved from the Internet <URL:http://genecards.weizmann.ac.il/cgi-bin/geneannot/GA_search.pl> [retrieved on 20151001]

## Description

### FIELD OF INVENTION

The present invention relates to the field of personalized medicine, and in particular to molecular signatures allowing the identification of individuals with cancer as being susceptible to respond to a treatment.

### BACKGROUND OF INVENTION

Breast cancer is the second cause of death in women. According to the WHO (Word Health Organization), 2.09 million cases were detected in 2018 from which 627 000 died. To date, metastasis is the main cause of death for breast cancer patients, and, despite great clinical advances, there is no effective drug that can prevent it.

Metastatic cells have been demonstrated to be metabolically different than other cancer cells. Depending on the cancer type, cancer cell invasion can be promoted by an increase in glucose and lactate metabolism, an increase in fatty acid metabolism and/or changes in mitochondrial metabolism. For example, increasing mitochondrial reactive oxygen species (mtROS) production and downstream activation of the SRC/PYK2 pathway promotes cancer cell migration, invasion and metastasis (Porporato et al., 2014). mtROS-induced metastasis involves mitochondrial superoxide production, superoxide-mediated activation of SRC kinase activating the downstream part of the transforming growth factor-β (TGF-β) pathway, and, ultimately, activation of focal adhesion kinase (FAK) family member PYK2, which remodels cell cytoskeleton during cancer metastasis (Porporato et al., 2014).

The role of enhanced reactive oxygen species (ROS) levels in solid tumor metastasis was also demonstrated in cancer stem cells (CSCs). More specifically, ROS signaling was shown to facilitate CSC self-renewal, and to potentiate the epithelial-to-mesenchymal transition (EMT) and invasiveness of cancer cells. CSCs or tumor-initiating cells (TICs) are a highly tumorigenic subpopulation. These cells play an important role in tumor propagation and cancer metastasis due to their quiescent properties as well as to their self-renewal and differentiation potential.

Some antioxidants, such as muscadine grape skin extract (MSKE), have demonstrated not only to be effective in decreasing ROS levels in prostate cancer models, but also to impair cell migration and EMT markers expression (Burton et al., 2014). However, general antioxidants cannot be used for cancer treatments as they produce variable effects, sometimes promoting tumor growth, and interfere with immunity. Preferentially, the use of mitochondria-targeted antioxidants, such as, *e.g.,* MitoQ and MitoTEMPO, has proven to be a good strategy to repress the migratory, invasive and metastatic phenotypes of cancer cells (Porporato et al., 2014). Interestingly, MitoQ successfully passed Phase I clinical trials and is now tested in Phase II and III trials in pathologies others than cancer (see, *e.g*., ClinicalTrials.gov Identifiers: NCT04267926). SKQ1, belonging to the SkQ class of mitochondria-targeted antioxidants, is also currently tested in clinical trials, but for applications other than cancer treatment.

MitoQ consists in a lipophilic cation (tetraphenylphosphonium [TPP]) linked to an antioxidant component (quinone) by a 10-carbon alkyl chain, which can rapidly cross biological membranes and concentrate up to 100-fold in mitochondria (Murphy and Smith, 2007). It is able to access the membrane core of mitochondria acting as a chain-breaking antioxidant, which further allows the recycling of MitoQ to its ubiquinol form via reduction by electron transfer chain (ETC) Complex II. MitoQ is currently mainly used as a food supplement, but due to its promising protective effects observed in *in vivo* experiments in mitochondria-related diseases like Parkinson or Alzheimer's disease, MitoQ was also tested and proven to be safe in humans (NCT00329056, NCT02597023).

There is need to provide the state of the art with means to identify individuals with cancer that are susceptible to respond to a treatment with mitochondria-targeted antioxidants, such as, *e.g.,* MitoQ and SKQ1.

There is also a need to provide the state of the art with means to identify individuals with metastatic cancer that are susceptible to respond to a treatment with mitochondria-targeted antioxidants, such as, *e.g.,* MitoQ and SKQ1.

### SUMMARY

A method for identifying an individual with cancer, in particular breast cancer, as being susceptible to respond to a treatment by at least one mitochondria-targeted antioxidant, preferably MitoQ or a functional derivative thereof (*e.g.,* SKQ1), the method comprising:
a) assessing the expression levels of at least three biomarkers selected from the group consisting of SLC7A11, SERPINE1, PSAT1, PHGDH-1, TXNRD1, VIM, SNAI1, ZEB1, NANOG, SOX2, PCK2, G6PD, ASNS, NUPR1 and SLC6A9, in samples obtained from said individual before and after treatment with said at least one mitochondria-targeted antioxidant, preferably MitoQ or SKQ1 or a functional derivative thereof; and
b) comparing the expression levels of the at least three biomarkers in the samples obtained after treatment with said mitochondria-targeted antioxidant with their respective reference levels in the samples obtained before treatment with said at least one mitochondria-targeted antioxidant, preferably MitoQ or SKQ1 or a functional derivative thereof;
wherein a significant variation of the expression levels of at least three biomarkers as compared to their respective reference levels represents a molecular signature indicative of the individual with cancer, in particular breast cancer, as being susceptible to respond to a treatment by the at least one mitochondria-targeted antioxidant, preferably MitoQ or SKQ1 or a functional derivative thereof.

In some embodiments, the significant variation comprises a variation of at least 1.2-fold of the expression levels of at least three biomarkers as compared to their respective reference levels, and optionally a statistical relevance of P < 0.05. In one embodiment, the statistical relevance is measured by Student *t* test, Mann-Whitney U test or another appropriate statistical test, preferably by Student *t* test.

In certain embodiments, the at least three biomarkers include SLC7A11, and/or SERPINE1 and/or PSAT1, preferably SLC7A11, SERPINE1 and PSAT1.

In some embodiments, the at least three biomarkers include SLC7A11, and/or SERPINE1, and/or PSAT1, and/or PHGDH-1 and/or TXNRD1, preferably SLC7A11, SERPINE1, PSAT1, PHGDH-1 and TXNRD1.

In certain embodiments, the biomarkers are SLC7A11, SERPINE1, PSAT1, PHGDH-1, TXNRD1, VIM, SNAI1, ZEB1, NANOG, SOX2, PCK2, G6PD, ASNS, NUPR1 and SLC6A9.

In some embodiments, assessing the expression levels of the at least three biomarkers is performed at the nucleic acid level, preferably at the RNA level, more preferably at the mRNA level.

In certain embodiments, the cancer is a metastatic cancer or a cancer susceptible to undergo metastasis, in particular a metastatic breast cancer or a breast cancer susceptible to undergo metastasis.

A further aspect of the invention relates to the use of the expression levels of at least three biomarkers selected from the group consisting of SLC7A11, SERPINE1, PSAT1, PHGDH-1, TXNRD1, VIM, SNAI1, ZEB1, NANOG, SOX2, PCK2, G6PD, ASNS, NUPR1 and SLC6A9 as a molecular signature for identifying an individual with cancer, in particular breast cancer, as being susceptible to respond to a treatment by at least one mitochondria-targeted antioxidant, preferably MitoQ or SKQ1 or a functional derivative thereof.

In some embodiments, the molecular signature comprises:
- at least three biomarkers including SLC7A11, and/or SERPINE1, and/or PSAT1, preferably biomarkers SLC7A11, SERPINE1 and PSAT1; or
- at least three biomarkers including SLC7A11, and/or SERPINE1, and/or, PSAT1 and/or PHGDH-1 and/or TXNRD1, preferably biomarker SLC7A11, SERPINE1, PSAT1, PHGDH-1and TXNRD1; or
- biomarkers being SLC7A11, SERPINE1, PSAT1, PHGDH-1, TXNRD1, VIM, SNAI1, ZEB1, NANOG, SOX2, PCK2, G6PD, ASNS, NUPR1 and SLC6A9.

In certain embodiments, the expression levels of the at least three biomarkers are compared to their respective reference levels, and wherein a significant variation of the expression levels of the at least three biomarkers as compared to their respective reference levels is indicative of the individual with cancer being susceptible to respond to a treatment by the at least one mitochondria-targeted antioxidant, preferably MitoQ or SKQ1 or a functional derivative thereof.

In some embodiments, the cancer is a metastatic cancer or a cancer susceptible to undergo metastasis, in particular a metastatic breast cancer or a breast cancer susceptible to undergo metastasis.

The invention also pertains to a mitochondria-targeted antioxidant, preferably MitoQ or SKQ1 or a functional derivative thereof, for use in a method for preventing and/or treating cancer, in particular breast cancer, in an individual identified by the method as defined herein.

In another aspect, the invention relates to a mitochondria-targeted antioxidant, preferably MitoQ or SKQ1 or a functional derivative thereof, for use in a method for preventing and/or treating metastasis of cancer, in particular breast cancer, in an individual identified by the method as defined herein.

The invention further relates to a mitochondria-targeted antioxidant, preferably MitoQ or SKQ1 or a functional derivative thereof, for use in a method for preventing and/or treating cancer recurrence before, concomitantly or after a surgery intended to remove all or part of the tumor, in particular breast cancer recurrence, in an individual identified by the method as defined herein.

In some embodiments, the antioxidant, preferably MitoQ or SKQ1 or a functional derivative thereof, is further combined with another cancer treatment, preferably selected in the group consisting of chemotherapy, radiotherapy, hormonal therapy, immunotherapy, anti-angiogenic therapy, a surgery intended to remove all or part of the tumor, in particular the breast tumor, at least another mitochondria-targeted antioxidant, and any combination thereof.

Another aspect of the invention relates to a kit for identifying an individual with cancer, in particular breast cancer, as being susceptible to respond to a treatment by at least one mitochondria-targeted antioxidant, preferably MitoQ or SKQ1 or a functional derivative thereof, comprising means for determining the expression levels of at least three biomarkers selected from the group consisting of SLC7A11, SERPINE1, PSAT1, PHGDH-1, TXNRD1, VIM, SNAI1, ZEB1, NANOG, SOX2, PCK2, G6PD, ASNS, NUPR1 and SLC6A9.

### DEFINITIONS

In the present invention, the following terms have the following meanings:

"About" preceding a figure encompasses plus or minus 10%, or less, of the value of said figure. It is to be understood that the value to which the term "about" refers is itself also specifically, and preferably, disclosed.

"Comprise" is intended to mean "contain", "encompass" and "include". In some embodiments, the term "comprise" also encompasses the term "consist of".

**"Mitochondria-targeted antioxidant"** refers to an antioxidant that can accumulate inside mitochondria and scavenge and/or inactivate one or several reactive oxygen species (ROS). Non-limitative examples of mitochondria-targeted antioxidants suitable for implementing the invention are disclosed by, *e.g.,* Jiang *et al.* (2020) and Fock and Parnova (2021).

**"Functional derivative",** when referred to the mitochondria-targeted antioxidant according to the invention, is intended to refer to a derivate of a mitochondria-targeted antioxidant that shares an equivalent biological physiological function, while having a similar structure. The term **"having a similar structure"** is intended to mean that the derivate of the mitochondria-targeted antioxidant differs from the reference mitochondria-targeted antioxidant in that it possesses one or more substituent(s).

**"Expression level"** refers to the synthesis of a significant detectable level of a biomarker of interest, at the nucleic acid (RNA) level and/or the polypeptide level. By extension, **"expressing"** or **"expression"** also refers to the level itself.

**"Biomarker"** refers to a molecule, preferably a DNA sequence, a RNA sequence, a protein, a glycoprotein or a lipoprotein that is expressed or not expressed, in particular differentially expressed or not differentially expressed, by a given cell or a population of cells, and which expression level may be measured by suitable techniques (e.g., RT-PCR, RNA sequencing, ELISA, FACS, western blot, proteomics, immunofluorescence staining, protein activity), in order to characterize said cell or population of cells. In some embodiments, the cells or the population of cells are originating from a sample of an individual, in particular a blood sample or a biopsy.

**"Molecular signature"** refers to a combination of DNAs, RNAs, proteins, genetic variants and/or other variables that display differential levels of expression in certain circumstances and provides an indication about the biological behavior towards said circumstances. As used herein, **"molecular signature"** is therefore indicative of a biological condition, a particular prognosis, or a particular response of an individual to a treatment.

**"Metastasis"** refers to the process by which cancer cells spread to other parts of the body from where the cancer originally started (referred to as **"primary cancer").** In practice, metastasis may be the consequence of pleural effusion; lymph nodes or blood vessels colonization; and the dissemination of cancer cells from the primary cancer site/localization to any other site/localization, such as, but not exclusively, the liver, brain, bones, lungs, skin, eyes, the peritoneum and/or the peritoneal cavity.

**"Treating"** or **"treatment"** or **"alleviation"** refers to both therapeutic treatment and prophylactic or preventative measures, wherein the object is to prevent or slow down (lessen) cancer, in particular breast cancer. Those in need of treatment include those already with cancer, in particular breast cancer, as well as those prone to develop cancer or those in whom cancer is to be prevented. An individual is successfully "treated" for cancer, in particular breast cancer, if, after receiving a therapeutic amount of the mitochondria-targeted antioxidant, preferably MitoQ or SKQ1 or a functional derivative thereof , alone or in combination with another treatment, the individual shows observable and/or measurable reduction in or absence of one or more of the symptoms associated with cancer, in particular breast cancer; reduced morbidity and mortality, and improvement in quality of life issues. The above parameters for assessing successful treatment and improvement of cancer, in particular breast cancer, are readily measurable by routine procedures familiar to physician or authorized personnel.

**"Preventing"** refers to keeping from happening, and/or lowering the chance of the onset, of at least one adverse effect or symptom of cancer, in particular breast cancer, and/or of a disorder or condition associated with a deficiency in or absence of an organ, tissue or cell function.

**"Therapeutically efficient amount"** refers to the level or the amount of the active agent, in particular a mitochondria-targeted antioxidant, more particularly MitoQ or SKQ1 or a functional derivative thereof, which is aimed at, without causing significant negative or unacceptable adverse side effects to the target, (1) delaying or preventing the onset of cancer, in particular breast cancer; (2) delaying or preventing the recurrence or relapse of cancer, in particular breast cancer; (3) slowing down or stopping the progression, aggravation, or deterioration of one or more symptoms of cancer, in particular breast cancer; (4) bringing about ameliorations of the symptoms of cancer, in particular breast cancer; (5) reducing the severity or incidence of cancer, in particular breast cancer; or (6) curing cancer, in particular breast cancer. A therapeutically effective amount may be administered prior to the onset of cancer, in particular breast cancer, for a prophylactic or preventive action. Alternatively, or additionally, the therapeutically effective amount may be administered after the onset of cancer, in particular breast cancer, for a therapeutic action. In one embodiment, a therapeutically effective amount of the composition is an amount that is effective in reducing at least one symptom of cancer, in particular breast cancer.

**"Pharmaceutically acceptable carrier"** refers to a carrier, or vehicle, that does not produce any or produces acceptable adverse, allergic or other unwanted reactions when administered to an animal individual, preferably a human individual. It includes any and all solvents, dispersion media, coatings, antibacterial and antifungal agents, isotonic and absorption delaying agents and the like. For human administration, preparations should meet sterility, pyrogenicity, general safety, quality and purity standards as required by regulatory Offices, such as, *e.g.,* the Food and Drugs Administration (FDA) in the United States of America or the European Medicines Agency (EMA) in the European Union.

**"Individual"** is intended to refer to an animal individual, preferably a mammalian individual, more preferably a human individual. Among the non-human mammalian individuals of interest, one may non-limitatively mention pets, such as dogs, cats, guinea pigs; animals of economic importance such as cattle, sheep, goats, horses, monkeys. In one embodiment, an individual may be a "patient", *i.e.* a warm-blooded animal, more preferably a human, who/which is awaiting the receipt of, or is receiving medical care or was/is/will be the object of a medical procedure, or is monitored for the development of a disease, disorder or condition. In one embodiment, the individual is an adult (for example a human subject above the age of 18). In another embodiment, the individual is a child (for example a human subject below the age of 18). In one embodiment, the individual is a male. In another embodiment, the individual is a female.

### DETAILED DESCRIPTION

The inventors identified a set of specific biomarkers that are differentially expressed in tumors upon treatment of individual with breast cancer with the mitochondria-targeted antioxidant MitoQ, and with the MitoQ analogue SKQ1. Determining the molecular signature on the basis of these biomarkers allows identifying the individuals with breast cancer being susceptible to response positively to the treatment.

This invention relates to a method for identifying an individual with cancer, in particular breast cancer, as being susceptible to respond to a treatment by at least one mitochondria-targeted antioxidant, preferably MitoQ or SKQ1 or a functional derivative thereof, the method comprising:
a) assessing the expression levels of at least three biomarkers selected from the group consisting of SLC7A11, SERPINE1, PSAT1, PHGDH-1, TXNRD1, VIM, SNAI1, ZEB1, NANOG, SOX2, PCK2, G6PD, ASNS, NUPR1 and SLC6A9, in samples obtained from said individual before and after treatment with said at least one mitochondria-targeted antioxidant; and
b) comparing the expression levels of the at least three biomarkers in the samples obtained after treatment with said mitochondria-targeted antioxidant with their respective reference levels in the samples obtained before treatment with said at least one mitochondria-targeted antioxidant;
wherein a significant variation of the expression levels of at least three biomarkers as compared to their respective reference levels represents a molecular signature indicative of the individual with cancer, in particular breast cancer, as being susceptible to respond to a treatment by the at least one mitochondria-targeted antioxidant, preferably MitoQ or SKQ1 or a functional derivative thereof.

As used herein, the term "a treatment by at least one mitochondria-targeted antioxidant" includes a treatment by one mitochondria-targeted antioxidant, a concomitant treatment by two mitochondria-targeted antioxidants or more *(i.e.,* the two mitochondria-targeted antioxidants or more are administered concomitantly), and a sequential treatment by two mitochondria-targeted antioxidants or more *(i.e.,* the two mitochondria-targeted antioxidants or more are administered sequentially).

In some embodiments, the method comprises the following steps:
a) assessing the expression levels of at least three biomarkers selected from the group consisting of SLC7A11, SERPINE1, PSAT1, PHGDH-1, TXNRD1, VIM, SNAI1, ZEB1, NANOG, SOX2, PCK2, G6PD, ASNS, and NUPR1, in samples obtained from said individual before and after treatment with said at least one mitochondria-targeted antioxidant, preferably MitoQ or SKQ1 or a functional derivative thereof; and
b) comparing the expression levels of the at least three biomarkers in the samples obtained after treatment with said at least one mitochondria-targeted antioxidant with their respective reference levels in the samples obtained before treatment with said at least one mitochondria-targeted antioxidant, preferably MitoQ or SKQ1 or a functional derivative thereof;
wherein a significant variation of the expression levels of at least three biomarkers as compared to their respective reference levels represents a molecular signature indicative of the individual with cancer, in particular breast cancer, as being susceptible to respond to a treatment by the at least one mitochondria-targeted antioxidant, preferably MitoQ or SKQ1 or a functional derivative thereof.

In certain embodiments, the method is performed *in vitro* or *ex vivo.*

In some embodiments, the method of the instant invention is for assisting the identification of an individual with cancer, in particular breast cancer, as being susceptible to respond to a treatment by at least one mitochondria-targeted antioxidant, preferably MitoQ or SKQ1. In practice, the method disclosed herein is for predicting the responsiveness of an individual with cancer, in particular breast cancer, to a treatment by at least one mitochondria-targeted antioxidant, preferably MitoQ or SKQ1 or a functional derivative thereof.

As used herein the term "mitochondria-targeted antioxidant" refers to an antioxidant that can accumulate inside mitochondria and scavenge and/or inactivate and/or prevents the production of one or more reactive oxygen species (ROS).

As used herein, the term "Reactive oxygen species" or "ROS" is intended to refer to the highly reactive molecules that are derived from oxygen (O₂) and can be produced by cells. ROS notably encompass the superoxide ion (O₂·⁻), the hydroxyl radical (HO·) and hydrogen peroxide (H₂O₂).

Non-limitative examples of mitochondria-targeted antioxidants that are suitable for the invention are disclosed by, *e.g.,* Jiang *et al.* (2020) and Fock and Parnova (2021).

In some embodiments, the mitochondria-targeted antioxidant is selected in the group consisting of MitoQ, MitoTEMPO, MitoTEMPOL, MitoE, MitoVitE, MitoSOD, MitoSNO, SKQ1, SKQR1, SKQ2, SKQ3, SKQ4, SKQ5, SKQBerb, SKQPalm, C12TPP, melatonin, dimethyl malonate, methylene blue, Mn-porphyrin-oligopeptide conjugate, M40401, SS20, SS31, XJB-5-125, XJB-5-131 and XJB-5-197. In some embodiments, the mitochondria-targeted antioxidant is a SKQ compound.

In certain embodiments, the mitochondria-targeted antioxidant is selected in the group consisting of MitoQ, SKQ1, MitoTEMPO. In certain embodiments, the mitochondria-targeted antioxidant is MitoQ or SKQ1. In some embodiments, the mitochondria-targeted antioxidant is MitoQ. In some embodiments, the mitochondria-targeted antioxidant is SKQ1.

In practice, a specific mitochondria-targeted antioxidant may be substituted by one more chemical group(s) so as to generate a functional derivative thereof. In some embodiments, the one more chemical group(s) comprises a O, S, N, F, Cl or Br atom. In certain embodiments, the one more chemical group(s) comprises a C₁-C₁₀ alkyl group, a C₁-C₁₀ aryl group, a carboxylic group, an amine group, an amide group, a sulfide group, a sulfoxide group, and ester group, an ether group and the like.

As used herein, the expression "at least three biomarkers" encompasses 3, 4, 5, 6, 7, 8, 9, 10, 11, 12, 13, 14 and 15 biomarkers.

In some embodiments, step a) comprises assessing the expression level of 3, 4, 5, 6, 7, 8, 9, 10, 11, 12, 13, 14 or 15 biomarkers selected from the group consisting of SLC7A11, SERPINE1, PSAT1, PHGDH-1, TXNRD1, VIM, SNAI1, ZEB1, NANOG, SOX2, PCK2, G6PD, ASNS, NUPR1 and SLC6A9. In certain embodiments, step a) comprises assessing the expression level of biomarkers SLC7A11, SERPINE1, PSAT1, PHGDH-1, TXNRD1, VIM, SNAI1, ZEB1, NANOG, SOX2, PCK2, G6PD, ASNS, NUPR1 and SLC6A9.

In some embodiments, step a) comprises assessing the expression level of 3, 4, 5, 6, 7, 8, 9, 10, 11, 12, 13, 14 or 15 biomarkers selected from the group consisting of SLC7A11, SERPINE1, PSAT1, PHGDH-1, TXNRD1, VIM, SNAI1, ZEB1, NANOG, SOX2, PCK2, G6PD, ASNS, and NUPR1. In certain embodiments, step a) comprises assessing the expression level of biomarkers SLC7A11, SERPINE1, PSAT1, PHGDH-1, TXNRD1, VIM, SNAI1, ZEB1, NANOG, SOX2, PCK2, G6PD, ASNS, and NUPR1.

In practice, SLC7A11, with Entrez Gene ID No. 23657, also refers non-limitatively to the Solute Carrier Family 7 Member 11, XCT, Solute Carrier Family 7 (Anionic Amino Acid Transporter Light Chain, Xc- System) Member 11, Calcium Channel Blocker Resistance Protein CCBR1, Amino Acid Transport System Xc-, Cystine/Glutamate Transporter, Solute Carrier Family 7 (Cationic Amino Acid Transporter, Y+ System) Member 11 and CCBR1.

SERPINE1, with Entrez Gene ID No. 5054, also refers non-limitatively to the Serpin Family A Member 1, Alpha-1-Antitrypsin, AAT, Serpin Peptidase Inhibitor Clade A (Alpha-1 Antiproteinase, Antitrypsin) Member 1, Protease Inhibitor 1 (Anti-Elastase) Alpha-1-Antitrypsin, Alpha-1 Protease Inhibitor, Alpha-1-Antiproteinase, Serpin A1, Alpha1AT, A1AT, A1A, PI1, PI, Serine (Or Cysteine) Proteinase Inhibitor Clade A (Alpha-1 Antiproteinase, Antitrypsin) Member 1, Alpha-1-Antitrypsin Short Transcript Variant 1C4, Alpha-1-Antitrypsin Short Transcript Variant 1C5, Serpin Peptidase Inhibitor Clade A Member 1, Epididymis Secretory Sperm Binding Protein, Alpha-1-Antitrypsin Null, PRO2275 and NNIF.

PSAT1, with Entrez Gene ID No. 29968, also refers non-limitatively to the Phosphoserine Aminotransferase 1, PSA, Phosphohydroxythreonine Aminotransferase, Phosphoserine Aminotransferase, EC 2.6.1.52, PSAT, Endometrial Progesterone-Induced Protein, PSATD, EPIP and NLS2.

PHGDH-1, with Entrez Gene ID No. 26227, also refers non-limitatively to the Phosphoglycerate Dehydrogenase-1, Inc-PHGDH-1, NONHSAG002595.2, HSALNG0006501 and Lnc-PHGDH-1.

TXNRD1, with Entrez Gene ID No. 7296, also refers non-limitatively to the Thioredoxin Reductase 1, GRIM-12, Gene Associated With Retinoic And Interferon-Induced Mortality 12 Protein, Cytoplasmic Thioredoxin Reductase 1, KM-102-Derived Reductase-Like Factor, Thioredoxin Reductase TR1, EC 1.8.1.9, TXNR, TR, Testis Tissue Sperm-Binding Protein Li 46a, Thioredoxin Reductase GRIM-12, Oxidoreductase, EC 1.8.1, TRXR1, KDRF and TR1.

VIM, with Entrez Gene ID No. 7431, also refers non-limitatively to the Vimentin and Epididymis Secretory Sperm Binding Protein.

SNAI1, with Entrez Gene ID No. 6615, also refers non-limitatively to the Snail Family Transcriptional Repressor 1, SNAH, Snail Family Zinc Finger 1, Zinc Finger Protein SNAI1, Protein Snail Homolog 1, Protein Sna, SLUGH2, SNAIL1, SNAIL, SNA, Snail 1 (Drosophila Homolog) Zinc Finger Protein, Snail Homolog 1 (Drosophila), Snail 1 Zinc Finger Protein, Snail 1 Homolog, Snail Homolog 1 and DJ710H13.1.

ZEB1, with Entrez Gene ID No. 6935, also refers non-limitatively to the Zinc Finger E-Box Binding Homeobox 1, AREB6, Transcription Factor 8 (Represses Interleukin 2 Expression), Posterior Polymorphous Corneal Dystrophy 3, Negative Regulator Of IL2, FECD6, TCF8, BZP, Zinc Finger Homeodomain Enhancer-Binding Protein, Delta-Crystallin Enhancer Binding Factor 1, NIL-2-A Zinc Finger Protein, Transcription Factor 8, DELTAEF1, NIL-2-A, ZFHX1A, PPCD3, ZFHEP, TCF-8 and ZEB.

NANOG, with Entrez Gene ID No. 79923, also refers non-limitatively to the Nanog Homeobox, Homeobox Transcription Factor Nanog, Homeobox Protein NANOG, Homeobox Transcription Factor Nanog-Delta 48, FLJ12581, FLJ40451 and HNanog.

SOX2, with Entrez Gene ID No. 6657, also refers non-limitatively to the SRY-Box Transcription Factor 2, SRY (Sex Determining Region Y)-Box 2, Transcription Factor SOX-2, SRY-Related HMG-Box Gene 2, Transcription Factor SOX2, MCOPS3, ANOP3.

PCK2, with Entrez Gene ID No. 5106, also refers non-limitatively to the Mitochondrial Phosphoenolpyruvate Carboxykinase 2, PEPCK2, Mitochondrial Phosphoenolpyruvate Carboxykinase [GTP], EC 4.1.1.32, PEPCK-M, PEPCK, Epididymis Secretory Sperm Binding Protein, Phosphopyruvate Carboxylase and PEP Carboxykinase.

G6PD, with Entrez Gene ID No. 2539, also refers non-limitatively to the Glucose-6-Phosphate Dehydrogenase, Glucose-6-Phosphate 1-Dehydrogenase, EC 1.1.1.49, G6PD1 and Epididymis Secretory Sperm Binding Protein.

ASNS, with Entrez Gene ID No. 440, also refers non-limitatively to the Glutamine-Hydrolyzing Asparagine Synthetase, Glutamine-Dependent Asparagine Synthetase, EC 6.3.5.4, TS11, TS11 Cell Cycle Control Protein, Cell Cycle Control Protein TS11, Asparagine Synthetase and ASNSD.

NUPR1, with Entrez Gene ID No. 26471, also refers non-limitatively to the Nuclear Protein 1, Transcriptional Regulator, Candidate Of Metastasis 1, COM1, Nuclear Protein 1, Protein P8 and P8.

SLC6A9, with Entrez Gene ID No. 6536, also refers non-limitatively to the Solute Carrier Family 6 Member 9, Sodium- and chloride-dependent glycine transporter 1, GlyT1.

In certain embodiments, the biomarkers are human biomarkers.

In certain embodiments, the at least three biomarkers include SLC7A11, and/or SERPINE1 and/or PSAT1, preferably SLC7A11, SERPINE1 and PSAT1.

In some embodiments, the at least three biomarkers include SLC7A11, and/or SERPINE1, and/or PSAT1, and/or PHGDH-1, and/or TXNRD1, preferably SLC7A11, SERPINE1, PSAT1, PHGDH-1 and TXNRD1.

In some embodiments, the at least three biomarkers include SLC7A11, SERPINE1, PSAT1, PHGDH-1 and TXNRD1, and further include VIM, and/or SNAI1, and/or ZEB1, and/or NANOG, and/or SOX2, and/or PCK2, and/or G6PD, and/or ASNS, and/or NUPR1 and/or SLC6A9.

In some embodiments, the at least three biomarkers include SLC7A11, SERPINE1, PSAT1, PHGDH-1 and TXNRD1, and further include VIM, and/or SNAI1, and/or ZEB1, and/or NANOG, and/or SOX2, and/or PCK2, and/or G6PD, and/or ASNS, and/or NUPR1.

In certain embodiments, the biomarkers are SLC7A11, SERPINE1, PSAT1, PHGDH-1, TXNRD1, VIM, SNAI1, ZEB1, NANOG, SOX2, PCK2, G6PD, ASNS, NUPR1 and SLC6A9.

In certain embodiments, the biomarkers are SLC7A11, SERPINE1, PSAT1, PHGDH-1, TXNRD1, VIM, SNAI1, ZEB1, NANOG, SOX2, PCK2, G6PD, ASNS, and NUPR1.

In practice, step a) comprises the assessment of the expression levels of at least three biomarkers in samples obtained from said individual before and after treatment with at least one mitochondria-targeted antioxidant.

As used herein, the expression "before treatment" means that the sample may be collected from about 6 h to about 10 days prior to the initiation of the treatment, preferably from about 12 h and 72 h. Within the scope of the instant invention, the expression "from about 6 h to about 10 days" includes 6 h, 12 h, 24 h (1 day), 48 h (2 days), 72 h (3 days), 4 days, 5 days, 6 days, 7 days, 8 days, 9 days and 10 days. In practice the initiation of the treatment encompasses the time course preceding the first uptake of the at least one mitochondria-targeted antioxidant.

Illustratively, the treatment may consist of administering a dose ranging from about 50 mg to about 100 mg of mitochondria-targeted antioxidant per day, preferably about 80 mg per day. In practice, the daily dose of mitochondria-targeted antioxidant may be administered as a single dose, or divided in two or more uptakes. Illustratively, the daily dose of 80 mg/day may be administered as 2 doses of 40 mg (40 mg bid (*bis in die* (twice a day)). Within the scope of the instant invention, the expression "from about 50 mg to about 100 mg" includes 50, 55, 60, 65, 70, 75, 80, 85, 90, 95 and 100 mg.

In practice, the treatment may extend from about 5 days to about 30 days, preferably from about 10 days to about 21 days. Within the scope of the instant invention, the expression "from about 5 days to about 30 days" includes 5, 6, 7, 8, 9, 10, 11, 12, 13, 14, 15, 16, 17, 18, 19, 20, 21, 22, 23, 24, 25, 26, 27, 28, 29 and 30 days. In some embodiments, the treatment may be or is to be performed, once, or repeated twice, three times, four times, or more if applicable. In certain embodiments, the treatment may be or is to be performed lifelong.

As used herein, the expression "after treatment" means that the sample may be collected from about 6 h to about 10 days after the end of the treatment, preferably from about 12 h and 72 h. Within the scope of the instant invention, the expression "from about 6 h to about 10 days" includes 6 h, 12 h, 24 h (1 day), 48 h (2 days), 72 h (3 days), 4 days, 5 days, 6 days, 7 days, 8 days, 9 days and 10 days. In practice, the end of the treatment encompasses the time course following the first or the last uptake of the mitochondria-targeted antioxidant. In one embodiment, the treatment consists of one uptake *(i.e.,* one dose) of the at least one mitochondria-targeted antioxidant.

In practice, assessing the expression levels of the at least three biomarkers may be performed at the nucleic acid level and/or the protein level.

In some embodiments, assessing the expression levels of the at least three biomarkers is performed at the nucleic acid level, preferably at the RNA level, more preferably at the mRNA level.

Illustratively, assessing the expression levels of the at least three biomarkers at the mRNA level may be performed by any suitable method known from the state of the art, or a method adapted thereof, such as, *e.g.,* by RT-PCR, RT-qPCR, Northern blot, hybridization techniques, and the like. In practice, total RNA may be extracted from the sample by any suitable method known from the state of the art, or a method adapted thereof, or by the means of commercial kit, such as, *e.g.,* the Mammalian RNA Isolation Kit from BioVision^{®}, RNeasy Mini Kit from Qiagen^{®}, and the like.

In certain embodiments, the expression levels of the biomarkers may be assessed by the means of PCR or qPCR primers that are specific to the above disclosed biomarkers.

Illustratively, non-limitative examples of suitable PCR or qPCR primers that are specific to the biomarkers according to the invention are depicted in **Table 1** and **Table 2** below.

**Table 1: Forward primers used in Q-PCR**

| **Gene name** | **Sequence of Forward 5' - 3' primer** | **SEQ ID NO:** |
|---|---|---|
| **Human genes** | | |
| *VIM* | CGGGAGAAATTGCAGGAGGA | **1** |
| *SNAI1* | AATCCAGAGTTTACCTTCCAGCA | **2** |
| *ZEB1* | GCCCAAACTGCAAGAAACGC | **4** |
| *NANOG* | AATACCTCAGCCTCCAGCAGATG | **8** |
| *SOX2* | CGAGTGGAAACTTTTGTCGGA | **9** |
| *PHGDH-1* | CACGACAGGCTTGCT GAATGA | **12** |
| *SLC7A11* | GCGTGGGCATGTCTCTGAC | **13** |
| *SERPINE1* | CACAAATCAGACGGCAGCACT | **14** |
| *TXNRD1* | CCACTGGTGAAAGACCACGTT | **16** |
| *PSAT1* | GGCCAGTTCAGTGCTGTCC | **17** |
| *PCK2* | TGCCAGGCTGGAAAGTGGAGTGT | **18** |
| *G6PD* | AACAGAGTGAGCCCTTCTTAA | **19** |
| *ASNS* | CACTCCGCGACTCCCTTTT | **20** |
| *SLC6A9* | GAGGATCAGCCCCATGTTCA | **21** |
| *NUPR1* | AGGACTTATTCCCGCTGACTGA | **22** |

| **Mouse genes** | | |
|---|---|---|
| *PHGDH-1* | ATGGCCTTCGCAAATCTGC | **24** |
| *SLC7A11* | ATGCAGTGGCAGTGACCTTT | **25** |
| *SERPINE1* | TCATCAATGACTGGGTGGAA | **26** |
| *PSAT1* | ACGCCAAAGGAGACGAAGCT | **27** |
| *TXNRD1* | GTGGCGACTTGGCTAATC | **28** |

**Table 2: Reverse primers used in Q-PCR**

| **Gene name** | **Sequence of Reverse 5'- 3' primer** | **SEQ ID NO:** |
|---|---|---|
| **Human genes** | | |
| *VIM* | AAGGTCAAGACGTGCCAGAG | **30** |
| *SNAI1* | TCCCAGATGAGCATTGGCAG | **31** |
| *ZEB1* | GTCGCCCATTCACAGGTATCA | **33** |
| *NANOG* | TGCGTCACACCATTGCTATTCTTC | **37** |
| *SOX2* | TGTGCAGCGGCTCGCAG | **38** |
| *PHGDH-1* | CTTCCGTAAACACGTCC AGTG | **41** |
| *SLC7A11* | GCTGGTAATGGACCAAAGACTTC | **42** |
| *SERPINE1* | CATCGGGCGTGGTGAACTC | **43** |
| *TXNRD1* | AGGAGAAAAGATCATCACTGCTGAT | **45** |
| *PSAT1* | GCTCCTGTCACCACATAGTCA | **46** |
| *PCK2* | GCAACCCCAAAGAAGCCGTTCTCA | **47** |
| *G6PD* | GGAGGCTGCATCATCGTACT | **48** |
| *ASNS* | ACCATTTCCACGGATGCAA | **49** |
| *SLC6A9* | AAGGCGATGCAGATGACCAC | **50** |
| *NUPR1* | TGCCGTGCGTGTCTATTTATTG | **51** |

| ***Mouse genes*** | | |
|---|---|---|
| *PHGDH-1* | AGTTCAGCTATCAGCTCCTCC | **53** |
| *SLC7A11* | GGCAACAAAGATCGGAACTG | **54** |
| *SERPINE1* | TGCTGGCCTCTAAGAAAGGA | **55** |
| *PSAT1* | ATGTTGAGTTCTACCGCCTTGTC | **56** |
| *TXNRD1* | ACCAGGAGAGACACTCAC | **57** |

In certain embodiments, assessing the expression levels of the at least three biomarkers is performed at the protein level. Illustratively, assessing the expression levels of the at least three biomarkers at the polypeptide level may be performed by any suitable method known from the state of the art, or a method adapted thereof, such as, *e.g.,* by an immunohistochemistry, immunofluorescence, FACS, ELISA, an enzymatic assay, and the like.

In some embodiments, the expression levels of the biomarkers may be assessed by the means of antibodies that specifically bind to the biomarkers.

Illustratively, non-limitative examples of suitable antibodies that specifically binds to the biomarkers according to the invention are depicted in **Table 3** below.

**Table 3: Antibodies used in western blotting and IHC**

| **Biomarker** | **Antibody** | **Reference** |
|---|---|---|
| *SNAI1* | rabbit anti-SNAIL | Cell signaling^{®}, #3879 |
| ZEB1 | rabbit anti-ZEB 1 | Cell signaling^{®}, #3396 |
| VIM | rabbit anti-VIMENTIN | Cell signaling^{®}, D21H3, #5741 |
| SOX2 | rabbit anti-SOX2 | Cell signaling^{®}, #3579 |
| TXNRD1 | rabbit anti-TRXR1 | Cell signaling^{®}, #15140 |
| G6PD | rabbit anti-G6PD | Sigma-Aldrich^{®}, HPA000247 |
| SLC6A9 | rabbit anti-SLC6A9 | Sigma-Aldrich^{®}, HPA013977 |
| PHGDH-1 | rabbit anti-PHGDH1 | Atlas antibodies^{®}, HPA024031 |
| ASNS | rabbit anti-ASNS | Sigma-Aldrich^{®}, HPA029318 |
| PCK2 | rabbit anti-PCK2 | Atlas antibodies^{®}, HPA051162 |
| PSAT1 | rabbit anti-PSAT1 | Origen^{®}, TA307984 |
| SERPINE1 | rabbit anti-SERPIN1 | Atlas antibodies^{®}, HPA050039 |
| NANOG | rabbit anti-NANOG | Cell signaling^{®}, #3580 |
| SLC7A11 | rabbit anti- SLC7A11 | Cell signaling^{®}, #12691 |
| NUPR1 | rabbit anti-NUPR1 | Thermo Fisher Scientific^{®}, #PA1-4177 |

### Significant variation

In some embodiments, the significant variation comprises a variation of at least about 1.2-fold of the expression levels of at least three biomarkers as compared to their respective reference levels, and optionally a statistical relevance of P < 0.05. In some embodiment, the statistical relevance is measured by Student t test or Mann-Whitney U test or any another appropriate statistical test, preferably by Student t test.

Within the scope of the instant invention, the expression "at least about 1.2-fold" encompasses at least about 1.2, 1.5, 2.0, 2.5, 3.0, 3.5, 4.0, 4.5, 5.0, 5.5, 6.0, 6.6, 7.0, 7.5, 8.0, 8.5, 9.0, 9.5, 10.0, or more.

In some embodiments, the significant variation comprises a variation of at least about 1.5-fold of the expression levels of at least three biomarkers as compared to their respective reference levels, and optionally a statistical relevance of P < 0.05.

In some embodiments, the significant variation comprises a variation of at least about 2.0-fold of the expression levels of at least three biomarkers as compared to their respective reference levels, and optionally a statistical relevance of P < 0.05.

In practice, the expression levels of at least three biomarkers after the treatment by the at least one mitochondria-targeted antioxidant is compared to their respective reference levels after the treatment by the at least one mitochondria-targeted antioxidant.

In some embodiments, the variation includes an increase in the expression level of a biomarker as compared to the corresponding reference level. In practice, the expression levels of biomarkers SLC7A11, SERPINE1, PSAT1, PHGDH-1, TXNRD1, PCK2, G6PD, ASNS, NUPR1 and SLC6A9 are increased as compared to the corresponding reference levels. In certain embodiments, biomarkers SLC7A11, SERPINE1, PSAT1, PHGDH-1, TXNRD1, PCK2, G6PD, ASNS, NUPR1 and SLC6A9 are upregulated as compared to their corresponding reference levels.

In some embodiments, the variation includes an increase in the expression level of a biomarker as compared to the corresponding reference level. In practice, the expression levels of biomarkers SLC7A11, SERPINE1, PSAT1, PHGDH-1, TXNRD1, PCK2, G6PD, ASNS, and NUPR1 are increased as compared to the corresponding reference levels. In certain embodiments, biomarkers SLC7A11, SERPINE1, PSAT1, PHGDH-1, TXNRD1, PCK2, G6PD, ASNS, and NUPR1 are upregulated as compared to their corresponding reference levels.

In some embodiments, the expression level of SLC7A11 in a sample obtained from an individual after treatment by at least one mitochondria-target antioxidant, is upregulated as compared to a reference level represented by the expression level of SLC7A11 in a sample obtained from an individual before treatment by at least one mitochondria-target antioxidant.

In certain embodiments, the expression level of SERPINE1 in a sample obtained from an individual after treatment by at least one mitochondria-target antioxidant, is upregulated as compared to a reference level represented by the expression level of SERPINE1 in a sample obtained from an individual before treatment by at least one mitochondria-target antioxidant.

In certain embodiments, the expression level of PSAT1 in a sample obtained from an individual after treatment by at least one mitochondria-target antioxidant, is upregulated as compared to a reference level represented by the expression level of PSAT1 in a sample obtained from an individual before treatment by at least one mitochondria-target antioxidant.

In certain embodiments, the expression level of PHGDH-1 in a sample obtained from an individual after treatment by at least one mitochondria-target antioxidant, is upregulated as compared to a reference level represented by the expression level of PHGDH-1 in a sample obtained from an individual before treatment by at least one mitochondria-target antioxidant.

In some embodiments, the expression level of TXNRD1 in a sample obtained from an individual after treatment by at least one mitochondria-target antioxidant, is upregulated as compared to a reference level represented by the expression level of TXNRD1 in a sample obtained from an individual before treatment by at least one mitochondria-target antioxidant.

In some embodiments, the expression level of PCK2 in a sample obtained from an individual after treatment by at least one mitochondria-target antioxidant, is upregulated as compared to a reference level represented by the expression level of PCK2 in a sample obtained from an individual before treatment by at least one mitochondria-target antioxidant.

In certain embodiments, the expression level of G6PD in a sample obtained from an individual after treatment by at least one mitochondria-target antioxidant, is upregulated as compared to a reference level represented by the expression level of G6PD in a sample obtained from an individual before treatment by at least one mitochondria-target antioxidant.

In some embodiments, the expression level of ASNS in a sample obtained from an individual after treatment by at least one mitochondria-target antioxidant, is upregulated as compared to a reference level represented by the expression level of ASNS in a sample obtained from an individual before treatment by at least one mitochondria-target antioxidant.

In certain embodiments, the expression level of NUPR1 in a sample obtained from an individual after treatment by at least one mitochondria-target antioxidant, is upregulated as compared to a reference level represented by the expression level of NUPR1 in a sample obtained from an individual before treatment by at least one mitochondria-target antioxidant.

In certain embodiments, the expression level of SLC6A9 in a sample obtained from an individual after treatment by at least one mitochondria-target antioxidant, is upregulated as compared to a reference level represented by the expression level of SLC6A9 in a sample obtained from an individual before treatment by at least one mitochondria-target antioxidant.

In some embodiments, the variation includes a decrease in the expression level of a biomarker as compared to the corresponding reference level. In practice, the expression levels of biomarkers VIM, SNAI1, ZEB1, NANOG and SOX2 are decreased as compared to the corresponding reference levels. In certain embodiments, biomarkers VIM, SNAI1, ZEB1, NANOG and SOX2 are downregulated as compared to their corresponding reference levels.

In some embodiments, the expression level of VIM in a sample obtained from an individual after treatment by at least one mitochondria-target antioxidant, is downregulated as compared to a reference level represented by the expression level of VIM in a sample obtained from an individual before treatment by at least one mitochondria-target antioxidant.

In certain embodiments, the expression level of SNAI1 in a sample obtained from an individual after treatment by at least one mitochondria-target antioxidant, is downregulated as compared to a reference level represented by the expression level of SNAI1 in a sample obtained from an individual before treatment by at least one mitochondria-target antioxidant.

In some embodiments, the expression level of ZEB1 in a sample obtained from an individual after treatment by at least one mitochondria-target antioxidant, is downregulated as compared to a reference level represented by the expression level of ZEB1 in a sample obtained from an individual before treatment by at least one mitochondria-target antioxidant.

In certain embodiments, the expression level of NANOG in a sample obtained from an individual after treatment by at least one mitochondria-target antioxidant, is downregulated as compared to a reference level represented by the expression level of NANOG in a sample obtained from an individual before treatment by at least one mitochondria-target antioxidant.

In some embodiments, the expression level of SOX2 in a sample obtained from an individual after treatment by at least one mitochondria-target antioxidant, is downregulated as compared to a reference level represented by the expression level of SOX2 in a sample obtained from an individual before treatment by at least one mitochondria-target antioxidant.

In some embodiments, the sample obtained the said individual is a biological sample. As used herein, the term "biological sample" encompasses a biopsy, blood sample, feces sample, urine sample, saliva sample, and the like

. In certain embodiments, the sample is a blood sample. In some embodiments, the sample is a biopsy, in particular a biopsy of the organ affected by the cancer to be treated, more particularly, a breast cancer to be treated. In certain embodiments, the sample comprise cancer cells from the cancer to be treated, more particularly, a breast cancer to be treated.

In certain embodiments, the sample is a fresh sample. In some embodiments, the sample has been cryopreserved, in particular at a temperature ranging from about -80°C to about 4°C. Within the scope of the invention, the expression "from about -80°C to about 4°C" includes about -80°C, -70°C, -60°C, -50°C, -40°C, -30°C, -20°C, -10°C, - 5°C, 0°C, 1°C, 2°C, 3°C and 4°C. In some embodiments, the sample has been persevered in paraffin, or a like. In some embodiments, the sample has been preserved in formaldehyde, or a like.

In practice, the sample is collected within the good practice in veterinary or human medicine.

In some embodiments, the sample was previously taken from the individual. In other words, the methods of the invention do not comprise a step of obtaining a sample from the individual. In certain embodiments, the methods of the invention are non-invasive methods. In some embodiments, the methods of the invention are performed *in vivo* or *ex vivo.*

In certain embodiments, the individual with cancer is a mammal. In some embodiments, the mammalian individual is a non-human mammalian individual, in particular selected in the group consisting of dogs, cats, rats, mice, horses, cattle, sheep, goats, pigs, and the like. In some embodiments, the mammalian individual is a human individual. In certain embodiments, the human individual is a female. In some embodiments, the human individual is a male.

In practice, the individual with cancer may have been previously diagnosed with cancer by authorized personnel skilled in the art.

In some embodiments, the individual with cancer is or may be under cancer treatment.

In some embodiments, said cancer is a blood cancer or a solid cancer.

In certain embodiments, the cancer is a blood cancer. As used herein, the term "blood cancer", also referred to as "hematologic cancer", encompasses any cancer involving uncontrolled proliferation of blood cells, in particular white blood cells. Blood cancers includes leukemia, lymphoma (Hodgkin and non-Hodgkin lymphomas) and myeloma.

In some embodiments, said cancer is a blood cancer selected in the group comprising or consisting of Hodgkin's disease, immunoblastic lymphadenopathy, lymphoma, chronic lymphocytic leukemia, acute leukemia, myeloma and the like.

In certain embodiments, the cancer is a solid cancer. As used herein, the term "solid cancer" encompasses any cancer (also referred to as "malignancy" or "tumor") that forms a discrete tumor mass, as opposed to blood cancer.

In some embodiments, the solid cancer is selected in the group comprising or consisting of bladder cancer, a bone cancer, a brain cancer, a breast cancer, a cancer of the central nervous system, a cancer of the cervix, a cancer of the upper aero digestive tract, a colorectal cancer, an endometrial cancer, a germ cell cancer, a glioblastoma, a kidney cancer, a laryngeal cancer, a liver cancer, a lung cancer, a nephroblastoma (Wilms tumor), a neuroblastoma, an esophageal cancer, an osteosarcoma, an ovarian cancer, a pancreatic cancer, a pleural cancer, a prostate cancer, a retinoblastoma, a skin cancer (including a melanoma), a small intestine cancer, a soft tissue sarcoma, a stomach cancer, a testicular cancer and a thyroid cancer.

In certain embodiment, the solid cancer is breast cancer. The term "breast cancer" as used herein, refers to histologically or cytologically confirmed cancer of the breast. In some embodiments, the breast cancer is a carcinoma. In some embodiments, the breast cancer is an adenocarcinoma. In some embodiments, the breast cancer is a sarcoma. In some embodiments, the breast cancer is a hormone receptor-positive (HR+) breast cancer. In some embodiments, the HR+ breast cancer is an estrogen receptor-positive (ER+) breast cancer. In some embodiments, the ER+ breast cancer is luminal A breast cancer. In some embodiments, the ER+ breast cancer is luminal B breast cancer.

In some embodiments, the breast cancer is a non-invasive breast cancer, in particular a ductal carcinoma in situ or a lobular carcinoma in situ. In certain embodiment, the breast cancer is an invasive breast cancer, in particular selected in the group comprising or consisting of invasive ductal carcinoma, invasive lobular carcinoma, Paget's disease of the nipple, inflammatory breast cancer, Phyllodes tumor of the breast, locally advanced breast cancer and metastatic breast cancer.

In certain embodiment, the breast cancer is a HR+ breast cancer or a hormone receptor-negative (HR-) breast cancer. In one embodiment, the hormone receptor positive breast cancer is an ER+ breast cancer and/or a progesterone receptor-positive (PR+) breast cancer. In one embodiment, the hormone receptor negative breast cancer is a triple negative breast cancer (TNBC).

In some embodiments, the breast cancer is a human epidermal growth factor receptor 2-positive (HER2+) breast cancer. In some embodiments, the breast cancer is a human epidermal growth factor receptor 2-negative (HER2-) breast cancer.

In some embodiments, the breast cancer is a cancer with a combined expression of receptors, e.g., hormone receptor-positive and HER2 negative (HR+ HER2-).

In some embodiments, the breast cancer is a cancer of Group 1 (luminal A), Group 2 (luminal B), Group 3 (HER2+) or Group 4 (basal-like). Group 1 includes tumors that are ER+ and progesterone- receptor-positive (PR+) positive, but negative for HER2 (HR+ HER2-). Group 2 includes tumors that are ER+, PR-and HER2+. Group 3 includes tumors that are ER- and PR-, but HER2+. Group 4, which is also called TNBC, includes tumors that are ER-, PR- and HER2+.

In some embodiments, the breast cancer is a HER2+ breast cancer or a TNBC. In some embodiments, the breast cancer is HER2+ breast cancer, preferably HER2+ breast adenocarcinoma. In some embodiments, the breast cancer is a TNBC.

In some embodiments, the type of tissue where breast cancer arises is milk ducts, milk-productive lobules or connective tissues.

In some embodiments, the breast cancer is a metastatic or a locally advanced breast cancer. The term "locally advanced breast cancer" refers to cancer that has spread from where it started in the breast to nearby tissue or lymph nodes, but not to other parts of the body.

In certain embodiments, the cancer is a metastatic cancer or a cancer susceptible to undergo metastasis, in particular a metastatic breast cancer or a breast cancer susceptible to undergo metastasis.

The term "metastatic breast cancer" refers to a cancer that has spread from the breast to other parts of the body, such as the bones, liver, lungs, or brain. Metastatic breast cancer may also be referred to as stage IV breast cancer. As used herein, the term "cancer susceptible to undergo metastasis" refers to an invasive cancer for which cancer cells may detach from the primary tumor and propagate in other organs, where they can grow to form secondary tumors, also referred to as metastases.

In one aspect, the invention also relates to the use of the expression levels of at least three biomarkers selected from the group consisting of SLC7A11, SERPINE1, PSAT1, PHGDH-1, TXNRD1, VIM, SNAI1, ZEB1, NANOG, SOX2, PCK2, G6PD, ASNS, NUPR1 and SLC6A9 as a molecular signature for identifying an individual with cancer, in particular breast cancer, as being susceptible to respond to a treatment by at least one mitochondria-targeted antioxidant, preferably MitoQ or SKQ1 or a functional derivative thereof.

In one aspect, the invention also relates to the use of the expression levels of at least three biomarkers selected from the group consisting of SLC7A11, SERPINE1, PSAT1, PHGDH-1, TXNRD1, VIM, SNAI1, ZEB1, NANOG, SOX2, PCK2, G6PD, ASNS, and NUPR1 as a molecular signature for identifying an individual with cancer, in particular breast cancer, as being susceptible to respond to a treatment by at least one mitochondria-targeted antioxidant, preferably MitoQ or SKQ1 or a functional derivative thereof.

In some embodiments, the molecular signature comprises:
a. at least three biomarkers including SLC7A11, and/or SERPINE1, and/or PSAT1, preferably biomarkers SLC7A11, SERPINE1 and PSAT1; or
b. at least three biomarkers including SLC7A11, and/or SERPINE1, and/or PSAT1, and/or PHGDH-1 and/or TXNRD1, preferably biomarker SLC7A11, SERPINE1, PSAT1, PHGDH-1 and TXNRD1; or
c. biomarkers being SLC7A11, SERPINE1, PSAT1, PHGDH-1, TXNRD1, VIM, SNAI1, ZEB1, NANOG, SOX2, PCK2, G6PD, ASNS, NUPR1 and SLC6A9.

In some embodiments, the molecular signature comprises:
a. at least three biomarkers including SLC7A11, and/or SERPINE1, and/or PSAT1, preferably biomarkers SLC7A11, SERPINE1 and PSAT1; or
b. at least three biomarkers including SLC7A11, and/or SERPINE1, and/or PSAT1, and/or PHGDH-1 and/or TXNRD1, preferably biomarker SLC7A11, SERPINE1, PSAT1, PHGDH-1 and TXNRD1; or
c. biomarkers being SLC7A11, SERPINE1, PSAT1, PHGDH-1, TXNRD1, VIM, SNAI1, ZEB1, NANOG, SOX2, PCK2, G6PD, ASNS, and NUPR1.

In some embodiments, the use is performed *in vitro* or *ex vivo.*

In certain embodiments, the expression levels of the at least three biomarkers are compared to their respective reference levels, and wherein a significant variation of the expression levels of the at least three biomarkers as compared to their respective reference levels is indicative of the individual with cancer being susceptible to respond to a treatment by the at least one mitochondria-targeted antioxidant, preferably MitoQ or SKQ1 or a functional derivative thereof.

In some embodiments, the expression levels of the at least three biomarkers are assessed before and after treatment of the individual by the at least one mitochondria-targeted antioxidant, preferably MitoQ or SKQ1 or a functional derivative thereof.

In certain embodiments, the reference levels are represented by the expression levels of the at least three biomarkers assessed before treatment of the individual by the at least one mitochondria-targeted antioxidant, preferably MitoQ or SKQ1 or a functional derivative thereof.

In some embodiments, the cancer is a metastatic cancer or a cancer susceptible to undergo metastasis, in particular a metastatic breast cancer or a breast cancer susceptible to undergo metastasis.

In some aspects, the invention further relates to a method for identifying an individual with cancer, in particular breast cancer, as being susceptible to respond to a treatment by at least one mitochondria-targeted antioxidant, preferably MitoQ or SKQ1 or a functional derivative thereof, and for treating said individual, the method comprising:
a) assessing the expression levels of at least three biomarkers selected from the group consisting of SLC7A11, SERPINE1, PSAT1, PHGDH-1, TXNRD1, VIM, SNAI1, ZEB1, NANOG, SOX2, PCK2, G6PD, ASNS, NUPR1 and SLC6A9, in samples obtained from said individual before and after treatment with said at least one mitochondria-targeted antioxidant; and
b) comparing the expression levels of the at least three biomarkers in the samples obtained after treatment with said mitochondria-targeted antioxidant with their respective reference levels in the samples obtained before treatment with said at least one mitochondria-targeted antioxidant;
c) identifying an individual with cancer, in particular breast cancer, as being susceptible to respond to a treatment by said at least one mitochondria-targeted antioxidant, when a significant variation of the expression levels of at least three biomarkers as compared to their respective reference levels is observed at step b); and
d) treating the individual being susceptible to respond to a treatment by said at least one mitochondria-targeted antioxidant identified at step c), by administering a therapeutically efficient amount of said at least one mitochondria-targeted antioxidant.

In some aspects, the invention further relates to a method for identifying an individual with cancer, in particular breast cancer, as being susceptible to respond to a treatment by at least one mitochondria-targeted antioxidant, preferably MitoQ or SKQ1 or a functional derivative thereof, and for treating said individual, the method comprising:
a) assessing the expression levels of at least three biomarkers selected from the group consisting of SLC7A11, SERPINE1, PSAT1, PHGDH-1, TXNRD1, VIM, SNAI1, ZEB1, NANOG, SOX2, PCK2, G6PD, ASNS, and NUPR1, in samples obtained from said individual before and after treatment with said at least one mitochondria-targeted antioxidant; and
b) comparing the expression levels of the at least three biomarkers in the samples obtained after treatment with said at least one mitochondria-targeted antioxidant with their respective reference levels in the samples obtained before treatment with said mitochondria-targeted antioxidant;
c) identifying an individual with cancer, in particular breast cancer, as being susceptible to respond to a treatment by said at least one mitochondria-targeted antioxidant, when a significant variation of the expression levels of at least three biomarkers as compared to their respective reference levels is observed at step b); and
d) treating the individual being susceptible to respond to a treatment by a mitochondria-targeted antioxidant identified at step c), by administering a therapeutically efficient amount of said at least one mitochondria-targeted antioxidant.

Another aspect of the invention relates to a mitochondria-targeted antioxidant, preferably MitoQ or SKQ1 or a functional derivative thereof, for use in a method for preventing and/or treating cancer, in particular breast cancer, in an individual identified by the method according to the instant invention. Another aspect of the invention relates to at least one mitochondria-targeted antioxidant, preferably MitoQ or SKQ1 or a functional derivative thereof, for use in a method for preventing and/or treating cancer, in particular breast cancer, in an individual identified by the method according to the instant invention.

In a further aspect, the invention relates to the use of at least one mitochondria-targeted antioxidant, preferably MitoQ or SKQ1 or a functional derivative thereof, for preventing and/or treating cancer, in particular breast cancer, in an individual identified by the method according to the instant invention.

One aspect of the invention relates to a method for preventing and/or treating cancer, in particular breast cancer, in an individual identified by the method according to the instant invention, comprising the administration of a therapeutically efficient amount of at least one mitochondria-targeted antioxidant, preferably MitoQ or SKQ1 or a functional derivative thereof.

A further aspect of the invention relates to at least one mitochondria-targeted antioxidant, preferably MitoQ or SKQ1 or a functional derivative thereof, for use in a method for preventing and/or treating metastasis of cancer, in particular breast cancer, in an individual identified by the method according to the instant invention.

In some embodiments, the at least one mitochondria-targeted antioxidant, preferably MitoQ or SKQ1 or a functional derivative thereof, is for use in a method for preventing metastasis of cancer, in particular breast cancer, in an individual identified by the method according to the instant invention.

One aspect of the invention relates to the use of at least one mitochondria-targeted antioxidant, preferably MitoQ or SKQ1 or a functional derivative thereof, for preventing and/or treating metastasis of cancer, in particular breast cancer, in an individual identified by the method according to the instant invention.

In some embodiments, the use of at least one mitochondria-targeted antioxidant, preferably MitoQ or SKQ1 or a functional derivative thereof, is for use preventing metastasis of cancer, in particular breast cancer, in an individual identified by the method according to the instant invention.

Another aspect of the invention relates to a method for preventing and/or treating metastasis of cancer, in particular breast cancer, in an individual identified by the method according to the instant invention, comprising the administration of a therapeutically efficient amount of at least one mitochondria-targeted antioxidant, preferably MitoQ or SKQ1 or a functional derivative thereof. In some embodiments, the method is for preventing metastasis of cancer, in particular breast cancer, in an individual identified by the method according to the instant invention.

In one aspect, the invention pertains to at least one mitochondria-targeted antioxidant, preferably MitoQ or SKQ1 or a functional derivative thereof, for use in a method for preventing and/or treating cancer recurrence before, concomitantly or after a surgery intended to remove all or part of the tumor, in particular breast cancer recurrence, in an individual identified by the method according to the instant invention.

The invention also relates to the use of at least one mitochondria-targeted antioxidant, preferably MitoQ or SKQ1 or a functional derivative thereof, for preventing and/or treating cancer recurrence before, concomitantly or after a surgery intended to remove all or part of the tumor, in particular breast cancer recurrence, in an individual identified by the method according to the instant invention.

In one aspect, the invention relates to a method for preventing and/or treating cancer recurrence before, concomitantly or after a surgery intended to remove all or part of the tumor, in particular breast cancer recurrence, in an individual identified by the method according to the instant invention, comprising the administration of a therapeutically efficient amount of at least one mitochondria-targeted antioxidant, preferably MitoQ or SKQ1 or a functional derivative thereof.

As used herein, the term "recurrence", also referred to as "relapse", is intended to refer to the process by which cancer is found after treatment, and after a period of time when the cancer could not be detected.

In some embodiments, the at least one mitochondria-targeted antioxidant, preferably MitoQ or SKQ1 or a functional derivative thereof, may be formulated as a pharmaceutical composition.

In certain embodiments, the pharmaceutical composition is to be administered to individual with cancer, in particular breast cancer, identified by the method according to the instant invention.

A further aspect of the invention relates to a pharmaceutical composition comprising (i) at least one mitochondria-targeted antioxidant, preferably MitoQ or SKQ1 or a functional derivative thereof, and (ii) a pharmaceutically acceptable carrier, for use in a method for preventing and/or treating cancer, in particular breast cancer, in an individual identified by the method according to the instant invention.

In some embodiments, a suitable pharmaceutically acceptable carrier according to the invention includes any and all conventional solvents, dispersion media, fillers, solid carriers, aqueous solutions, coatings, antibacterial and antifungal agents, isotonic and absorption delaying agents, and the like. In certain embodiments, suitable pharmaceutically acceptable carriers may include, water, saline, phosphate buffered saline, dextrose, glycerol, ethanol and a mixture thereof. In some embodiments, pharmaceutically acceptable carriers may further comprise minor amounts of auxiliary substances such as wetting or emulsifying agents, preservatives or buffers, which enhance the shelf life or effectiveness of the cells. The preparation and use of pharmaceutically acceptable carriers is well known in the art.

In some embodiments, the at least one mitochondria-targeted antioxidant, preferably MitoQ or SKQ1 or a functional derivative thereof, or the pharmaceutical composition may be administered to an individual in need thereof by any suitable route, *i.e.,* by an oral administration, a topical administration or a parenteral administration, *e.g.,* by injection, including a sub-cutaneous administration, a venous administration, an arterial administration, in intra-muscular administration, an intra-ocular administration and an intra-auricular administration. In certain embodiment, the at least one mitochondria-targeted antioxidant, preferably MitoQ or SKQ1 or a functional derivative thereof, or the pharmaceutical composition may be administered to an individual in need thereof by oral administration.

In some embodiments, the administration of the at least one mitochondria-targeted antioxidant, preferably MitoQ or SKQ1 or a functional derivative thereof, or the pharmaceutical composition by injection may be directly performed in the target tissue of interest, in particular in order to avoid spreading of the mitochondria-targeted antioxidant.

Within the scope of the instant invention, the therapeutically effective amount of the at least one mitochondria-targeted antioxidant, preferably MitoQ or SKQ1 or a functional derivative thereof, to be administered may be determined by a physician or an authorized person skilled in the art and can be suitably adapted within the time course of the treatment.

In certain embodiments, the therapeutically effective amount to be administered may depend upon a variety of parameters, including the material selected for administration, whether the administration is in single or multiple doses, and the individual's parameters including age, physical conditions, size, weight, gender, and the severity of the cancer to be treated.

In certain embodiments, a therapeutically effective amount of the at least one mitochondria-targeted antioxidant, preferably MitoQ or SKQ1 or a functional derivative thereof, agent may range from about 0.001 mg to about 3,000 mg, per dosage unit, preferably from about 0.05 mg to about 100 mg, per dosage unit.

Within the scope of the instant invention, the expression "from about 0.001 mg to about 3,000 mg" includes, from about 0.001 mg, 0.002 mg, 0.003 mg, 0.004 mg, 0.005 mg, 0.006 mg, 0.007 mg, 0.008 mg, 0.009 mg, 0.01 mg, 0.02 mg, 0.03 mg, 0.04 mg, 0.05 mg, 0.06 mg, 0.07 mg, 0.08 mg, 0.09 mg, 0.1 mg, 0.2 mg, 0.3 mg, 0.4 mg, 0.5 mg, 0.6 mg, 0.7 mg, 0.8 mg, 0.9 mg, 1 mg, 2 mg, 3 mg, 4 mg, 5 mg, 6 mg, 7 mg, 8 mg, 9 mg, 10 mg, 20 mg, 30 mg, 40 mg, 50 mg, 60 mg, 70 mg, 80 mg, 90 mg, 100 mg, 150 mg, 200 mg, 250 mg, 300 mg, 350 mg, 400 mg, 450 mg, 500 mg, 550 mg, 600 mg, 650 mg, 700 mg, 750 mg, 800 mg, 850 mg, 900 mg, 950 mg, 1,000 mg, 1,100 mg, 1,150 mg, 1,200 mg, 1,250 mg, 1,300 mg, 1,350 mg, 1,400 mg, 1,450 mg, 1,500 mg, 1,550 mg, 1,600 mg, 1,650 mg, 1,700 mg, 1,750 mg, 1,800 mg, 1,850 mg, 1,900 mg, 1,950 mg, 2,000 mg, 2,100 mg, 2,150 mg, 2,200 mg, 2,250 mg, 2,300 mg, 2,350 mg, 2,400 mg, 2,450 mg, 2,500 mg, 2,550 mg, 2,600 mg, 2,650 mg, 2,700 mg, 2,750 mg, 2,800 mg, 2,850 mg, 2,900 mg, 2,950 mg and 3,000 mg per dosage unit.

**In** certain embodiments, the at least one mitochondria-targeted antioxidant, preferably MitoQ or SKQ1 or a functional derivative thereof, may be at dosage levels sufficient to deliver from about 0.001 mg/kg to about 100 mg/kg, from about 0.01 mg/kg to about 50 mg/kg, preferably from about 0.1 mg/kg to about 40 mg/kg, preferably from about 0.5 mg/kg to about 30 mg/kg, from about 0.01 mg/kg to about 10 mg/kg, from about 0.1 mg/kg to about 10 mg/kg, and more preferably from about 1 mg/kg to about 25 mg/kg, of subject body weight per day. Within the scope of the instant invention, the expression "from about 0.001 mg/kg to about 100 mg/kg" includes about 0.001 mg/kg, 0.002 mg/kg, 0.003 mg/kg, 0.004 mg/kg, 0.005 mg/kg, 0.006 mg/kg, 0.007 mg/kg, 0.008 mg/kg, 0.009 mg/kg, 0.01 mg/kg, 0.02 mg/kg, 0.03 mg/kg, 0.04 mg/kg, 0.05 mg/kg, 0.06 mg/kg, 0.07 mg/kg, 0.08 mg/kg, 0.09 mg/kg, 0.1 mg/kg, 0.2 mg/kg, 0.3 mg/kg, 0.4 mg/kg, 0.5 mg/kg, 0.6 mg/kg, 0.7 mg/kg, 0.8 mg/kg, 0.9 mg/kg, 1 mg/kg, 2 mg/kg, 3 mg/kg, 4 mg/kg, 5 mg/kg, 6 mg/kg, 7 mg/kg, 8 mg/kg, 9 mg/kg, 10 mg/kg, 20 mg/kg, 30 mg/kg, 40 mg/kg, 50 mg/kg, 60 mg/kg, 70 mg/kg, 80 mg/kg, 90 mg/kg and 100 mg/kg.

In some embodiments, the at least one mitochondria-targeted antioxidant, preferably MitoQ or SKQ1 or a functional derivative thereof, is further combined with another cancer treatment, preferably selected in the group consisting of chemotherapy, radiotherapy, hormonal therapy, immunotherapy, anti-angiogenic therapy, a surgery intended to remove all or part of the tumor, in particular the breast tumor, and another mitochondria-targeted antioxidant, and any combination thereof.

As used herein, the term "chemotherapy" refers to a drug treatment that uses chemicals to kill fast-growing cells, in particular cancer cells.

Non-limitative examples of chemotherapy agents include acalabrutinib, alectinib, alemtuzumab, anastrozole, avapritinib, avelumab, belinostat, bevacizumab, bleomycin, blinatumomab, bosutinib, brigatinib, carboplatin, carmustine, cetuximab, chlorambucil, cisplatin, copanlisib, cytarabine, daunorubicin, decitabine, dexamethasone, docetaxel, doxorubicin, encorafenib, epirubicin, erdafitinib, etoposide, everolimus, exemestane, fludarabine, 5-fluorouracil (5-FU), gemcitabine, ifosfamide, imatinib Mesylate, leuprolide, lomustine, mechlorethamine, melphalan, methotrexate, mitomycin, nelarabine, paclitaxel, pamidronate, panobinostat, pralatrexate, prednisolone, ofatumumab, rituximab, temozolomide, topotecan, tositumomab, trastuzumab, vandetanib, vincristine, vorinostat, zanubrutinib, and the like.

In certain embodiments, the chemotherapy agent is selected in a group consisting of doxorubicin, 5-FU, cisplatin, paclitaxel, gemcitabine, epirubicin and the like.

In one embodiment, the chemotherapy agent is doxorubicin. In one embodiment, the chemotherapy agent is cisplatin.

Without wanting to be bound to a theory, the inventors observed that the antioxidant, preferably MitoQ or SKQ1 or a functional derivative thereof, as defined herein, may prevent the cancer cell migration and invasion (prometastatic traits) that may occur with certain prometastatic chemotherapy agents, such as anthracycline drugs, in particular doxorubicin.

As used herein, the term "radiotherapy" refers to a therapy that uses radiation to kill cancer cells or reduce the size/volume of the tumor.

In some embodiments, the radiotherapy relies upon external beam radiation or internal beam radiation, as used in the field.

As used herein, the term "hormonal therapy" refers to a therapy that uses drugs to stop or slow down the growth of tumors, mainly by blocking the body's ability to produce hormones or by interfering with effects of hormones on cancer cells.

Illustratively, non-limitative examples of hormonal therapy include luteinizing hormone-releasing hormone (LHRH) agonists, such as leuprolide, goserelin, triptorelin, and histrelin; androgen receptor antagonists, such as flutamide, enzalutamide, apalutamide, bicalutamide, and nilutamide; other androgen-blocking drugs, such as abiraterone, prednisone, and ketoconazole; estrogen receptor modulators, such as tamoxifen, toremifene, and fulvestrant; drugs that lower estrogen effects, such as aromatase inhibitors letrozole, anastrozole, and exemestane; progesterone-like drugs, such as megestrol acetate; testosterone-like drugs, such as androgens; and estrogen-like drugs, such as estradiol.

As used herein, the term "immunotherapy" refers to a therapy aiming at inducing and/or enhancing an immune response towards a specific target, in particular towards cancer cells.

As used herein, examples of immunotherapies include, without being limited to, vaccination, such as preventive and therapeutic vaccination; adoptive transfer of immune cells, in particular of T cells (such as alpha beta (αβ) T cells or gamma delta T cells) or natural killer (NK) cells; checkpoint inhibitors; checkpoint agonists; antibodies.

In some embodiments, the immunotherapy is a cancer immunotherapy. As used herein, the term "cancer immunotherapy" refers to an immunotherapy used for the treatment of a cancer, said immunotherapy modulating the immune response of a subject with the aim of inducing and/or stimulating the immune response of the subject towards cancer cells. In some embodiments, the cancer immunotherapy comprises, or consists of, the adoptive transfer of immune cells (ACT), in particular of T cells (such as αβ T cells or γδ T cells), NK cells or NK T cells. In some embodiments, the cancer immunotherapy comprises, or consists of, the administration of a checkpoint inhibitor.

In certain embodiments, said immunotherapy comprises ACT, a checkpoint inhibitor, vaccination, the like, and a combination thereof.

As used herein, an adoptive transfer of cells or adoptive cell therapy (or ACT) is defined as the transfer, for example as an infusion, of immune cells to a subject. As a cancer treatment, the adoptive transfer of immune cells to a subject aims at enhancing the subject immune response towards the cancer cells.

In certain embodiments, the transferred immune cells are T cells or natural killer (NK) cells. In some embodiments, the transferred immune cells are T cells, in particular CD8+ T cells, and/or NK cells.

In one embodiment, the transferred immune cells are T cells, in particular effector T cells. Examples of effector T cells include CD4+ T cells and CD8+ T cells. In one embodiment, the transferred immune cells are αβ T cells. In another embodiment, the transferred immune cells are γδ T cells. In one embodiment, the transferred immune cells are CD4+ T cells, CD8+ T cells, or NK T cells, preferably the transferred T cells are CD8+ T cells.

In certain embodiments, the transferred immune cells as described hereinabove are antigen-specific immune cells. In one embodiment, the transferred immune cells as described hereinabove are tumor-specific immune cells, in other words the transferred immune cells as described hereinabove specifically recognize cancer cells or tumor cells through an antigen specifically and/or abundantly expressed by said cancer cells or tumor cells. In one embodiment, the transferred immune cells as described hereinabove are tumor-specific effector T cells, in particular tumor-specific CD8+ effector T cells, more particularly tumor-specific cytotoxic CD8+ T cells; or tumor-specific NK cells.

Examples of tumor-specific antigens, i.e., antigens that are specifically and/or abundantly expressed by cancer cells include, include without being limited to, neoantigens (also referred to as new antigens or mutated antigens), 9D7, ART4, β-catenin, BING-4, Bcr-abl, BRCA1/2, calcium-activated chloride channel 2, CDK4, CEA (carcinoembryonic antigen), CML66, Cyclin B1, CypB, EBV (Epstein-Barr virus) associated antigens (such as LMP-1, LMP-2, EBNA1 and BARF 1), EGFRvIII, Ep-CAM, EphA3, fibronectin, Gp100/pmel17, Her2/neu, HPV (human papillomavirus) E6, HPV E7, hTERT, IDH1, IDH2, immature laminin receptor, MC1R, Melan-A/MART-1, MART-2, mesothelin, MUC1, MUC2, MUM-1, MUM-2, MUM-3, NY-ESO-1/LAGE-2, p53, PRAME, prostate-specific antigen (PSA), PSMA (prostate-specific membrane antigen), Ras, SAP-1, SART-I, SART-2, SART-3, SSX-2, survivin, TAG-72, telomerase, TGF-βRII, TRP-1/-2, tyrosinase, WT1, antigens of the BAGE family, antigens of the CAGE family, antigens of the GAGE family, antigens of the MAGE family, antigens of the SAGE family, and antigens of the XAGE family.

As used herein, neoantigens (also referred to as new antigens or mutated antigens) correspond to antigens derived from proteins that are affected by somatic mutations or gene rearrangements acquired by the tumors. Neoantigens may be specific to each individual subject and thus provide targets for developing personalized immunotherapies. Examples of neoantigens include for example, without being limited to, the R24C mutant of CDK4, the R24L mutant of CDK4, KRAS mutated at codon 12, mutated p53, the V600E mutant of BRAF and the R132H mutant of IDH1.

In one embodiment, the transferred immune cells as described hereinabove are specific for a tumor antigen selected from the group comprising or consisting of the class of CTAs (cancer/testis antigens, also known as MAGE-type antigens), the class of neoantigens and the class of viral antigens.

As used herein, the class of CTAs corresponds to antigens encoded by genes that are expressed in tumor cells but not in normal tissues except in male germline cells.

Examples of CTAs include, without being limited to, MAGE-A1, MAGE-A3, MAGE-A4, MAGE-C2, NY-ESO-1, PRAME and SSX-2.

As used herein, the class of viral antigens corresponds to antigens derived from viral oncogenic proteins. Examples of viral antigens include, without being limited to, HPV (human papillomavirus) associated antigens such as E6 and E7.

In one embodiment, the transferred immune cells as described hereinabove are autologous immune cells, in particular autologous T cells. In another embodiment, the transferred immune cells as described hereinabove are allogenic (or allogenous) immune cells, in particular allogenic NK cells.

For example, autologous T cells can be generated *ex vivo* either by expansion of antigen-specific T cells isolated from the subject or by redirection of T cells of the subject through genetic engineering.

In one embodiment, the immune cells to be infused are modified *ex vivo,* in particular with RNA interference (also known as RNAi), before being infused to the subject.

In some embodiments, said immune cells are selected in the group comprising T cells, in particular CD8+ T cells and chimeric antigen receptor (CAR) T cells; NK cells, in particular CAR NK cells; the like; and combination thereof.

CARs are synthetic receptors consisting of a targeting moiety that is associated with one or more signaling domains in a single fusion molecule or in several molecules. In general, the binding moiety of a CAR consists of an antigen-binding domain of a single-chain antibody (scFv), comprising the light and variable fragments of a monoclonal antibody joined by a flexible linker. Binding moieties based on receptor or ligand domains have also been used successfully. The signaling domains for first generation CARs are usually derived from the cytoplasmic region of the CD3zeta or the Fc receptor gamma chains.

Thus, in one embodiment, the transferred T cells as described hereinabove are CAR T cells. The expression of a CAR allows the T cells to be redirected against a selected antigen, such as an antigen expressed at the surface of cancer cells. In one embodiment, the transferred CAR T cells recognize a tumor-specific antigen.

In another embodiment, the transferred NK cells as described hereinabove are CAR NK cells. The expression of a CAR allows the NK cells to be redirected against a selected antigen, such as an antigen expressed at the surface of cancer cells. In one embodiment, the transferred CAR NK cells recognize a tumor-specific antigen.

Examples of tumor-specific antigens are mentioned hereinabove.

In one embodiment, the CAR immune cells as described hereinabove are autologous CAR immune cells, in particular autologous CAR T cells. In another embodiment, the CAR immune cells as described hereinabove are allogenic (or allogenous) CAR immune cells, in particular allogenic CAR NK cells.

As used herein, a checkpoint inhibitor therapy is defined as the administration of at least one checkpoint inhibitor to the subject.

Checkpoint inhibitors (CPI, that may also be referred to as immune checkpoint inhibitors or ICI) block the interactions between inhibitory receptors expressed on T cells and their ligands. As a cancer treatment, checkpoint inhibitor therapy aims at preventing the activation of inhibitory receptors expressed on T cells by ligands expressed by the tumor cells. Checkpoint inhibitor therapy thus aims at preventing the inhibition of T cells present in the tumor, i.e., tumor infiltrating T cells, and thus at enhancing the subject immune response towards the tumor cells.

Examples of checkpoint inhibitors include, without being limited to, inhibitors of the cell surface receptor PD-1 (programmed cell death protein 1), also known as CD279 (cluster of differentiation 279); inhibitors of the ligand PD-L1 (programmed death-ligand 1), also known as CD274 (cluster of differentiation 274) or B7-H1 (B7 homolog 1); inhibitors of the cell surface receptor CTLA4 or CTLA-4 (cytotoxic T-lymphocyte-associated protein 4), also known as CD152 (cluster of differentiation 152); inhibitors of IDO (indoleamine 2,3-dioxygenase) and inhibitors of TDO (tryptophan 2,3-dioxygenase); inhibitors of LAG-3 (lymphocyte-activation gene 3), also known as CD223 (cluster differentiation 223); inhibitors of TIM-3 (T-cell immunoglobulin and mucin-domain containing-3), also known as HAVCR2 (hepatitis A virus cellular receptor 2) or CD366 (cluster differentiation 366); inhibitors of TIGIT (T cell immunoreceptor with Ig and ITIM domains), also known as VSIG9 (V-Set And Immunoglobulin Domain-Containing Protein 9) or VSTM3 (V-Set And Transmembrane Domain-Containing Protein 3); inhibitors of BTLA (B and T lymphocyte attenuator), also known as CD272 (cluster differentiation 272); inhibitors of CEACAM-1 (carcinoembryonic antigen-related cell adhesion molecule 1) also known as CD66a (cluster differentiation 66a).

In one embodiment, the at least one checkpoint inhibitor is selected from the group comprising or consisting of inhibitors or PD-1, inhibitors of PD-L1, inhibitors of CTLA-4 and any mixtures thereof.

In one embodiment, the at least one checkpoint inhibitor is selected from the group comprising or consisting of pembrolizumab, nivolumab, cemiplimab, tislelizumab, spartalizumab, ABBV-181, JNJ-63723283, BI 754091, MAG012, TSR-042, AGEN2034, avelumab, atezolizumab, durvalumab, LY3300054, ipilimumab, tremelimumab, and any mixtures thereof.

In one embodiment, the at least one checkpoint inhibitor is an inhibitor of PD-1, also referred to as an anti-PD-1. Inhibitors of PD-1 may include antibodies targeting PD-1, in particular monoclonal antibodies, and non-antibody inhibitors such as small molecule inhibitors. Examples of inhibitors of PD-1 include, without being limited to, pembrolizumab, nivolumab, cemiplimab, tislelizumab, spartalizumab, ABBV-181, JNJ-63723283, BI 754091, MAG012, TSR-042, and AGEN2034.

In one embodiment, the at least one checkpoint inhibitor is an inhibitor of PD-L1, also referred to as an anti-PD-L1. Inhibitors of PD-L1 may include antibodies targeting PD-L1, in particular monoclonal antibodies, and non-antibody inhibitors such as small molecule inhibitors. Examples of inhibitors of PD-L1 include, without being limited to, avelumab, atezolizumab, durvalumab and LY3300054.

In one embodiment, the at least one checkpoint inhibitor is an inhibitor of CTLA-4, also referred to as an anti-CTLA-4. Inhibitors of CTLA-4 may include antibodies targeting CTLA-4, in particular monoclonal antibodies, and non-antibody inhibitors such as small molecule inhibitors. Examples of inhibitors of CTLA-4 include, without being limited to, ipilimumab and tremelimumab.

In one embodiment, the at least one checkpoint inhibitor is an inhibitor of IDO or an inhibitor of TDO, also referred to as an anti-IDO or anti-TDO, respectively. Examples of inhibitors of IDO include, without being limited to, 1-methyl-D-tryptophan (also known as indoximod), epacadostat (also known as INCB24360), navoximod (also known as IDO-IN-7 or GDC-0919), linrodostat (also known as BMS-986205), PF-06840003 (also known as EOS200271), TPST-8844, and LY3381916.

As used herein, an antibody therapy is defined as the administration of at least one antibody to the subject.

As used herein, "antibody therapy" may include the administration of monoclonal antibodies, polyclonal antibodies, multiple-chain antibodies, single-chain antibodies, single-domain antibodies, antibody fragments, antibody domains, antibody mimetics or multi-specific antibodies such as bispecific antibodies.

Examples of antibodies include, without being limited to, tumor-specific antibodies, in particular tumor-specific monoclonal antibodies, (such as antibodies targeting cell surface markers of cancer cells or tumor cells, antibodies targeting proteins involved in the growth or spreading of cancer cells or tumor cells).

Examples of antibodies include anti-CD137 antibodies and anti-OX40 antibodies as described hereinabove; anti-PD-1 antibodies (such as pembrolizumab, nivolumab, cemiplimab, tislelizumab, and spartalizumab), anti-PD-L1 antibodies (such as avelumab, atezolizumab, and durvalumab) and anti-CTLA-4 antibodies (such as ipilimumab and tremelimumab) as described hereinabove; and anti-HER2 antibodies (such as trastuzumab).

As used herein, the term "vaccination" refers to the use of a preparation comprising a substance or a group of substances (*i.e.,* a vaccine) meant to induce and/or enhance in a subject a targeted immune response towards cancer cells. Prophylactic vaccination is used to prevent a subject from ever having a particular disease or to only have a mild case of the disease. Therapeutic vaccination is intended to treat a particular disease in a subject, for example cancers. For example, therapeutic anti-cancer vaccines may comprise a tumor-associated antigen or tumor-associated antigens, aiming at inducing and/or enhancing a cell-mediated immune response, in particular a T cell immune response, directed towards the cancer cells expressing said tumor-associated antigen(s).

As used herein, a "therapeutic vaccine" is defined as the administration of at least one tumor-specific antigen (*e.g*., synthetic long peptides or SLP), or of the nucleic acid encoding said tumor-specific antigen; the administration of recombinant viral vectors selectively entering and/or replicating in tumor cells; the administration of tumor cells; and/or the administration of immune cells (*e.g.*, dendritic cells) engineered to present tumor-specific antigens and trigger an immune response against these antigens.

As a cancer treatment, therapeutic vaccines aim at enhancing the subject immune response towards the tumor cells.

Examples of therapeutic vaccines aiming at enhancing the subject immune response towards the tumor cells include, without being limited to, viral-vector based therapeutic vaccines such as adenoviruses (*e.g*., oncolytic adenoviruses), vaccinia viruses (*e.g.,* modified vaccinia Ankara (MVA)), alpha viruses (*e.g.,* Semliki Forrest Virus (SFV)), measles virus, Herpes simplex virus (HSV), and coxsackievirus; synthetic long peptide (SLP) vaccines; RNA-based vaccines, and dendritic cell vaccines.

As used herein, the term "anti-angiogenic therapy" refers to a therapy that use anti-angiogenic drugs to inhibit growth of new blood vessels, in particular blood vessels from a tumor.

Non limitative examples of anti-angiogenic drugs include bevacizumab (commercially available as Avastin^{®}), lenalidomide, sunitinib, axitinib, cabozantinib, everolimus, lenvatinib mesylate, pazopanib, regorafenib, sorafenib, thalidomide, ramucirumab, vandetanib and ziv-aflibercept.

In one embodiment, the mitochondria-targeted antioxidant of the invention is further combined with at least another mitochondria-targeted antioxidant.

In certain embodiments, said chemotherapy, radiotherapy, hormonal therapy, immunotherapy, anti-angiogenic therapy, surgery or other(s) mitochondria-targeted antioxidant(s) is to be administered separately or concomitantly with the mitochondria-targeted antioxidant, preferably MitoQ or SKQ1 or a functional derivative thereof. In a particular embodiment, the mitochondria-targeted antioxidant of the invention, preferably MitoQ or SKQ1 or a functional derivative thereof is to be administered separately or concomitantly with at least another mitochondria-targeted antioxidant.

One aspect of the invention relates to a kit for identifying an individual with cancer, in particular breast cancer, as being susceptible to respond to a treatment by at least one mitochondria-targeted antioxidant, preferably MitoQ or SKQ1 or a functional derivative thereof, comprising means for determining the expression levels of at least three biomarkers selected from the group consisting of SLC7A11, SERPINE1, PSAT1, PHGDH-1, TXNRD1, VIM, SNAI1, ZEB1, NANOG, SOX2, PCK2, G6PD, ASNS, NUPR1 and SLC6A9.

One aspect of the invention relates to a kit for identifying an individual with cancer, in particular breast cancer, as being susceptible to respond to a treatment by at least one mitochondria-targeted antioxidant, preferably MitoQ or SKQ1 or a functional derivative thereof, comprising means for determining the expression levels of at least three biomarkers selected from the group consisting of SLC7A11, SERPINE1, PSAT1, PHGDH-1, TXNRD1, VIM, SNAI1, ZEB1, NANOG, SOX2, PCK2, G6PD, ASNS, and NUPR1.

In some embodiments, the means for determining the expression levels of the biomarkers include antibodies that specifically bind to the biomarkers, and/or PCR or qPCR primers that specifically hybridize with the mRNA of the biomarkers.

Another aspect of the invention relates to the use of a kit for identifying an individual with cancer, in particular breast cancer, as being susceptible to respond to a treatment by at least one mitochondria-targeted antioxidant, preferably MitoQ or SKQ1 or a functional derivative thereof, the kit comprising means for determining the expression levels of at least three biomarkers selected from the group consisting of SLC7A11, SERPINE1, PSAT1, PHGDH-1, TXNRD1, VIM, SNAI1, ZEB1, NANOG, SOX2, PCK2, G6PD, ASNS and NUPR1, and optionally SLC6A9.

### BRIEF DESCRIPTION OF THE DRAWINGS

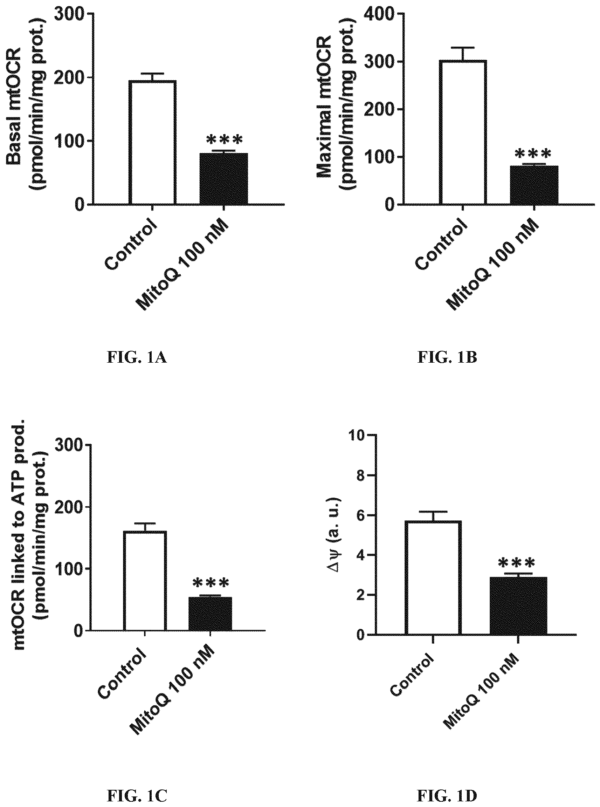

**Figure 1A-E** is a set of graphs showing that MitoQ selectively represses mitochondrial superoxide production by MDA-MB-231 human breast cancer cells. **(A-C)** MDA-MB-231 human breast cancer cells were treated ± MitoQ 100 nM for 48 h. **(A)** basal, **(B)** maximal and **(C)** ATP-linked mitochondrial oxygen consumption rates (mtOCRs) measured using Seahorse oximetry (n = 17-18). **(D)** Mitochondrial potential (Δψ) measured using JC-10 on a SpectraMax i3 spectrophotometer (n = 16). **(E)** Mitochondrial superoxide production was measured using electron paramagnetic resonance (EPR) with MitoTEMPO-H as a specific mitochondrial superoxide sensor ± PEG-SOD2 (n = 4). All data are shown as means ± SEM. * P < 0.05, *** P < 0.001 compared to control; by Student t test.

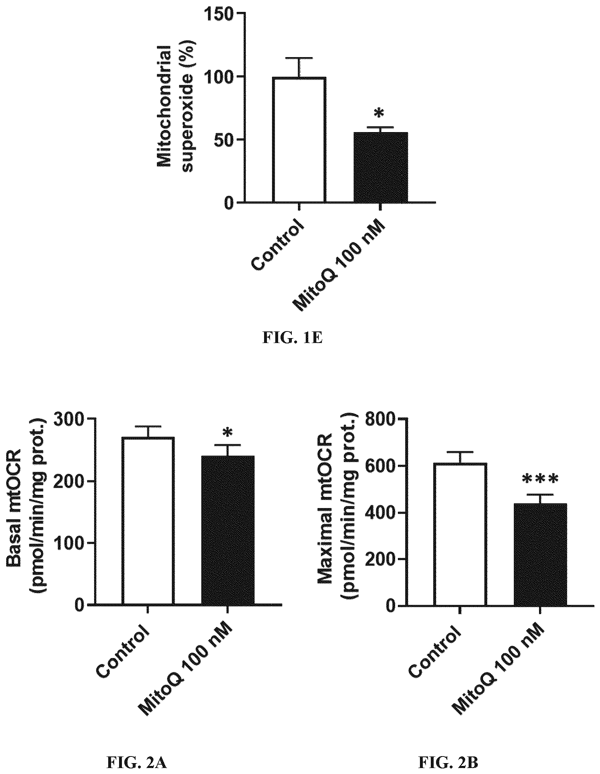

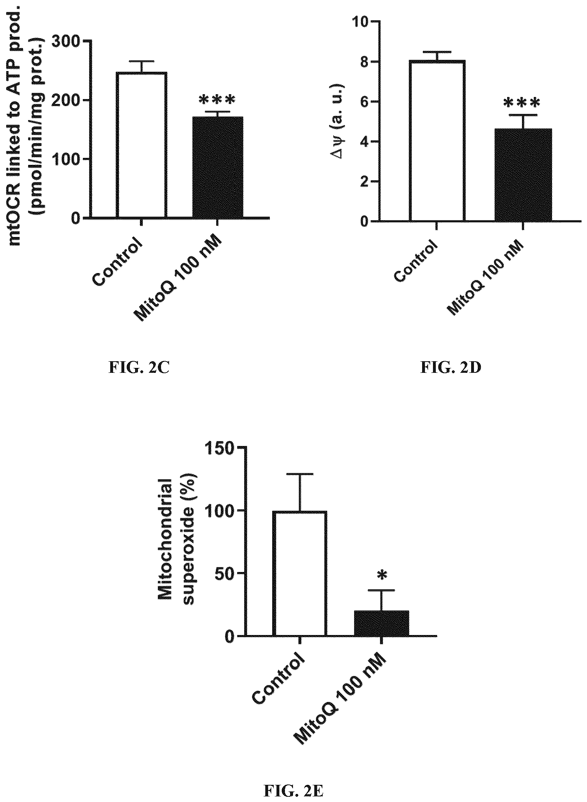

**Figure 2A-E** is a set of graphs showing that MitoQ selectively represses mitochondrial superoxide production by SkBr3 human breast cancer cells. SkBr3 human breast cancer cells were treated ± MitoQ 100 nM for 48h. **(A-C)** as in **Figure 1A-C** (n = 36-41). **(D)** as in **Figure 1D** (n = 16). **(E)** As in **Figure 1E** (n = 6). All data are shown as means ± SEM. * P < 0.05, *** P < 0.001 compared to control; by Student t test.

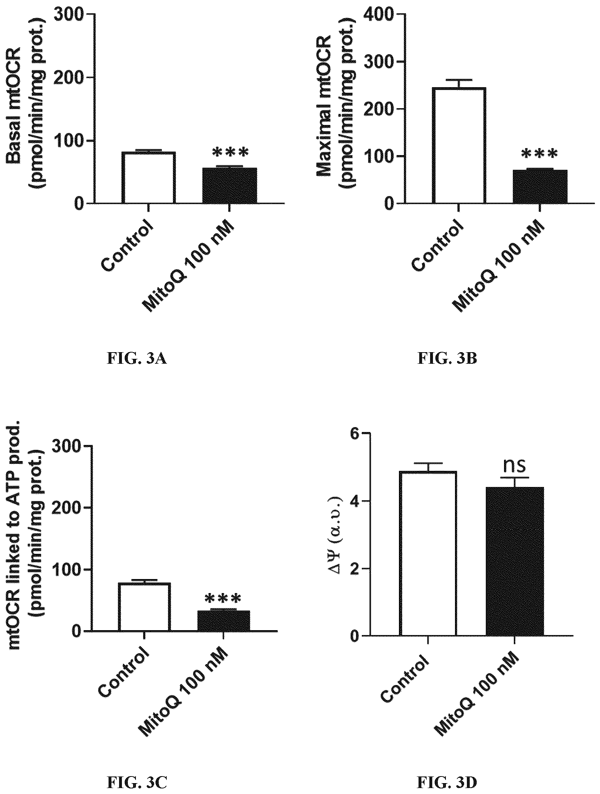

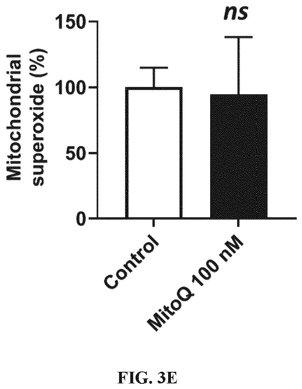

**Figure 3A-E** is a set of graphs showing that MitoQ does not affect the Mitochondrial potential (Δψ) of MCF10A normal breast epithelial cells. **(A-C)** MCF10A normal breast epithelial cells were treated ± MitoQ 100 nM for 48h. **(A-C)** as in **Figure 1A-C** (n = 19-23). **(D)** as in **Figure 1D** (n = 8). **(E)** As in **Figure 1E** (n = 3). All data are shown as means ± SEM. *** P < 0.001, ns = non-significant, compared to control; by Student t test.

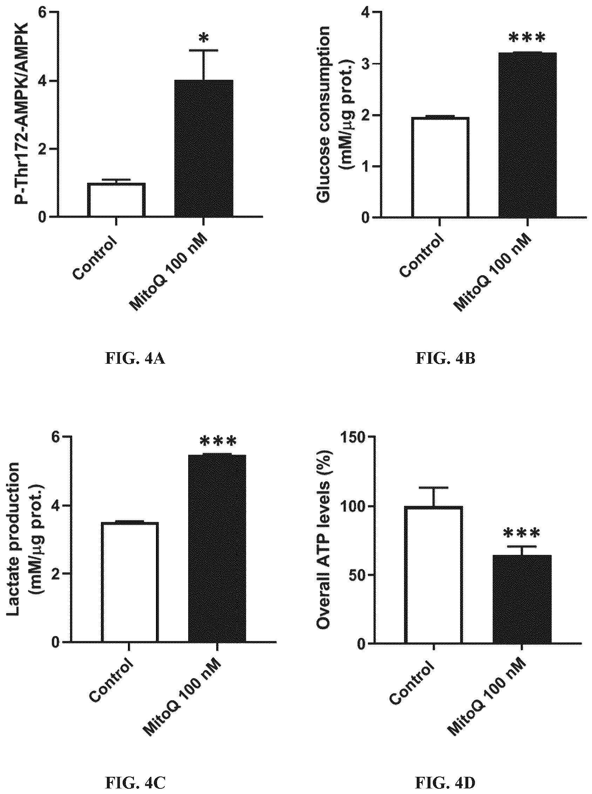

**Figure 4A-F** is a set of graphs showing that MitoQ selectively represses ATP production by MDA-MB-231 human breast cancer cells. MDA-MB-231 cells were treated ± mitoQ 100 nM for 48h. **(A)** Phospho-Thr172-AMPK (P-T172-AMPK)/total AMPK with representative western blot (n = 4). **(B)** Glucose consumption, **(C)** lactate production determined using enzymatic assays on a CMA600 analyzer (n = 3 all). **(D)** Total ATP cell content (n = 8). **(E)** Cell cycle analysis in MDA-MB-231 cells (n = 3-4) by flow cytometry using propidium iodide (10,000 events analyzed per experiment). **(F)** Direct MDA-MB-231 cell counting on a SpectraMax i3 spectrophotometer at the indicated time points after treatment with increasing doses of MitoQ (0 to 500 nM) (n = 4). All data are shown as means ± SEM. * P < 0.05, *** P < 0.001 compared to control; by Student t test **(A-E)** or 2-way ANOVA with Tukey's post-hoc test **(F).**

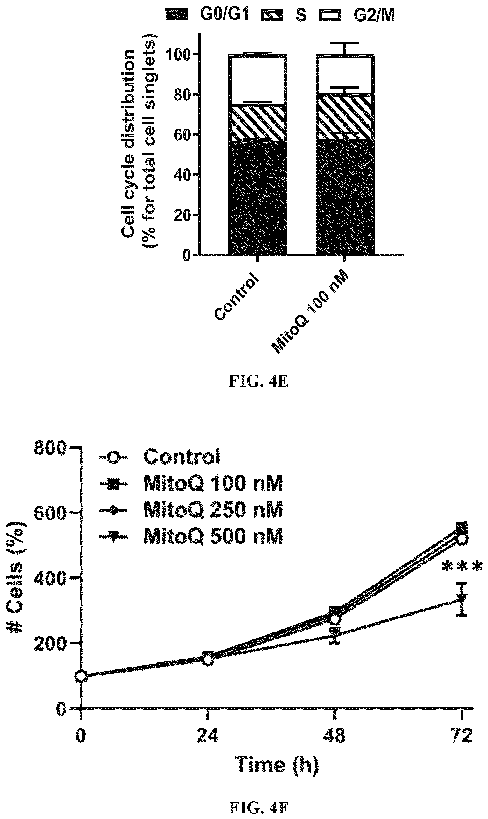

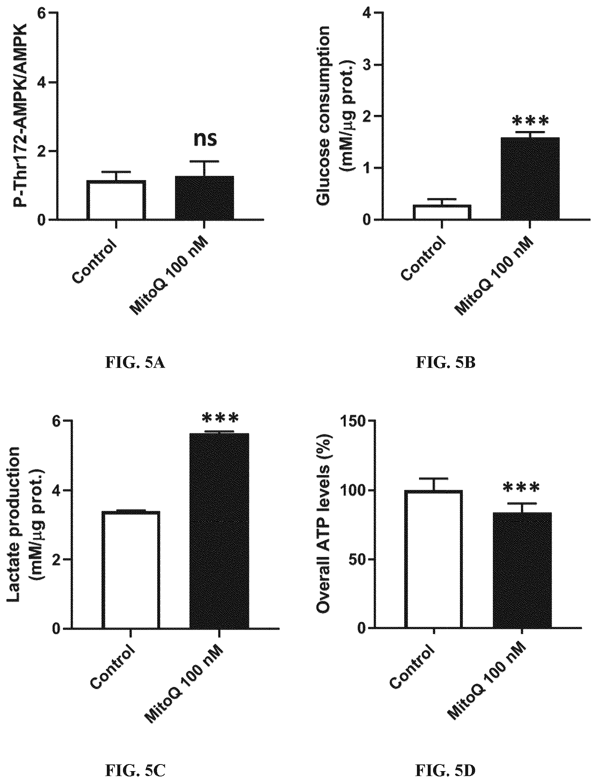

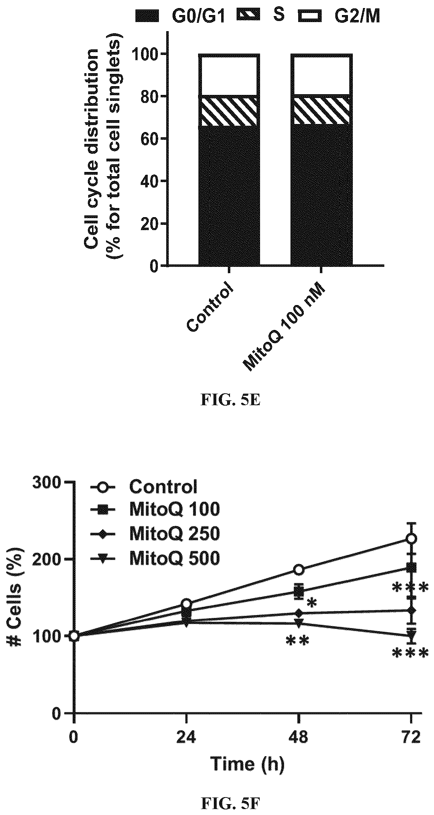

**Figure 5A-F** is a set of graphs showing that MitoQ selectively repressed ATP production by SkBr3 human breast cancer cells. SkBr3 cells were treated ± MitoQ 100 nM for 48h. **(A)** As in **Figure 4A** (n = 10). **(B-C)** As in **Figure 4B-C** (n = 3-6). **(D)** Total ATP cell content (n = 8). **(E)** Cell cycle analysis in SkBr3 cells (right, n = 2) by flow cytometry using propidium iodide (10,000 events analyzed per experiment). **(F)** As in **Figure 4F** (n = 6). All data are shown as means ± SEM. * P < 0.05, ** P < 0.01, *** P < 0.001, ns= non-significant, compared to control; by Student t test (A-E) or 2-way ANOVA with Tukey's post-hoc test (F).

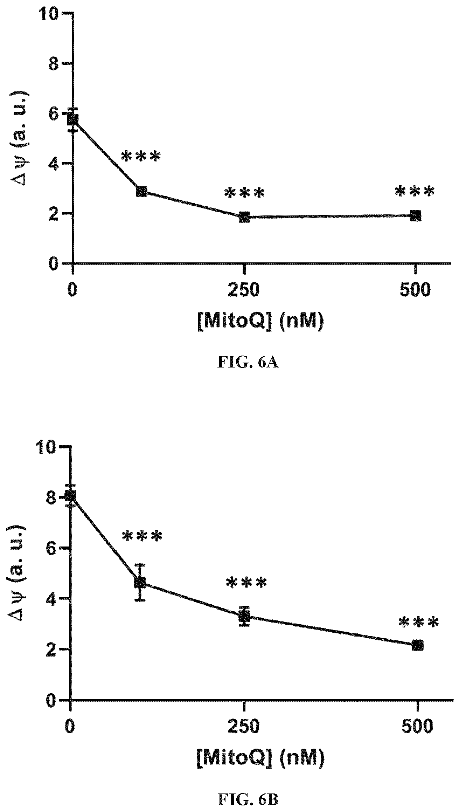

**Figure 6A-B** is a set of graphs showing that MitoQ dose-dependently depolarizes mitochondria in human breast cancer cells. Human breast cancer cells were treated for 48 h with the indicated doses of MitoQ. Mitochondrial potential (Δψ) measured in MDA-MB-231 **(A)** and SkBr3 **(B)** cells using JC-10 on a SpectraMax i3 spectrophotometer (n = 16 all). All data are shown as means ± SEM. *** P < 0.001 compared to control, by 2-way ANOVA with Tukey's post-hoc test.

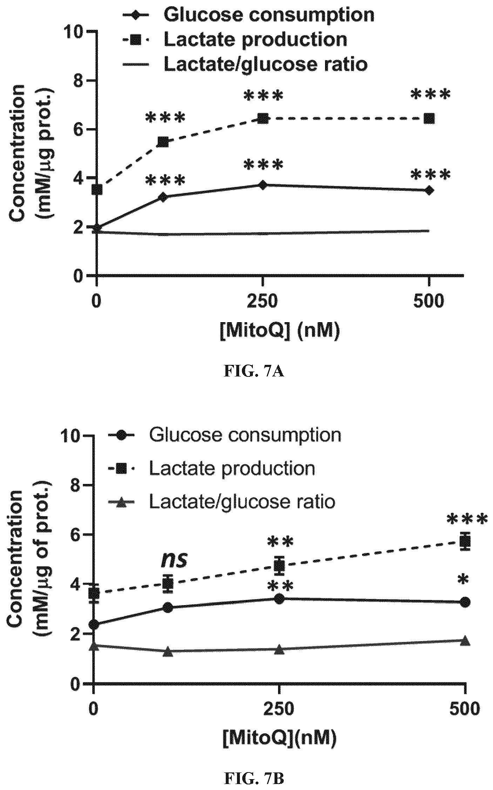

**Figure 7A-B** is a set of graphs showing that MitoQ dose-dependently induces a glycolytic switch in human breast cancer cells. Human breast cancer cells were treated for 48 h with the indicated doses of MitoQ. Glucose consumption, lactate production and the lactate/glucose ratio measured in MDA-MB-231 **(A)** (n = 3) and SkBr3 **(B)** (n = 5-6) cells using enzymatic assays on a CMA600 analyzer. All data are shown as means ± SEM. * P < 0.05, ** P < 0.01, *** P < 0.001, ns= non-significant, compared to control, by 2-way ANOVA with Tukey's post-hoc test.

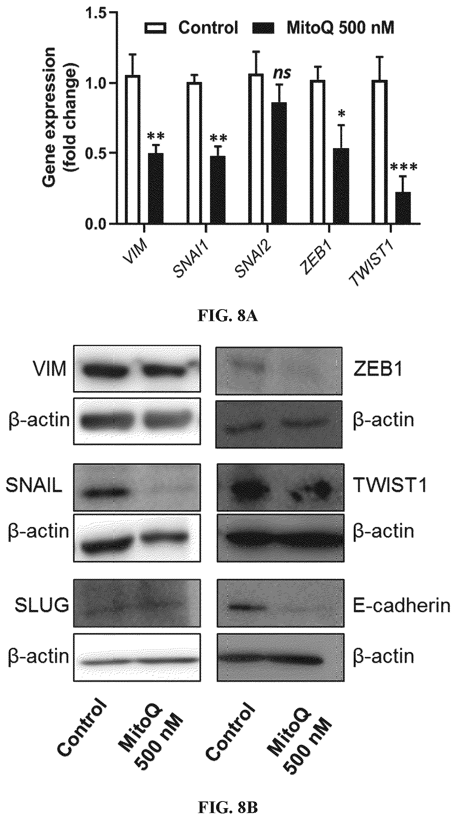

**Figure 8A-D** is a set of graphs showing that MitoQ partially reverts EMT in human breast cancer cells. MDA-MB-231 cells **(A-B)** and SkBr3 cells **(C-D)** were treated for 48 h with ± 500 nM MitoQ. **(A/C)** mRNA expression of EMT markers vimentin (VIM), SNAIL (SNAI1), SLUG (SNAI2), ZEB1 and TWIST1 in MDA-MB-231 cancer cells (n = 6-9) and in in SkBr3 cells (n = 3-9). **(B/D)** Representative Western blots of the corresponding proteins with β-actin as a loading control. All data are shown as means ± SEM. * P < 0.05, ** P < 0.01, *** P < 0.001, ns= non-significant, compared to control; by Student t test.

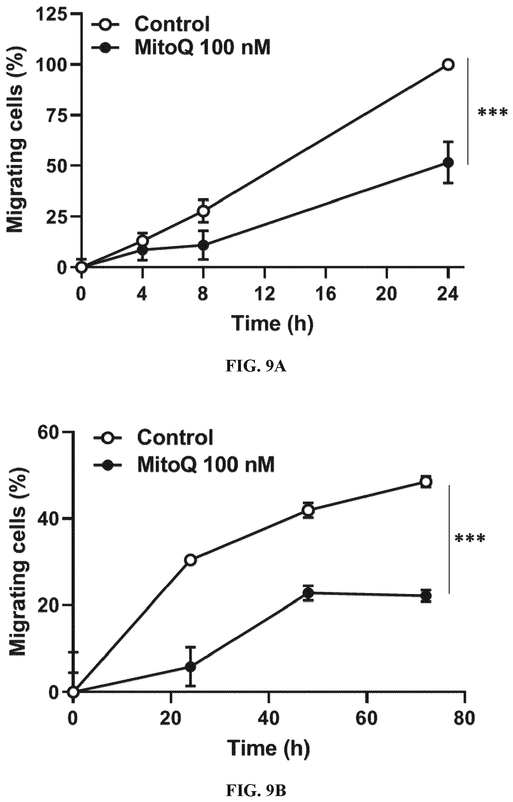

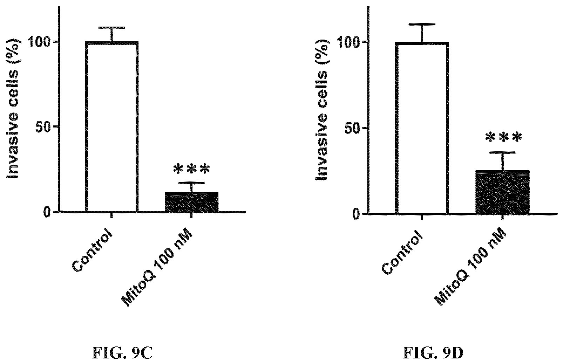

**Figure 9A-D** is a set of graphs showing that MitoQ inhibits the *in vitro* migration and invasion of human breast cancer cells. MDA-MB-231 cells **(A/C)** and SkBr3 cells **(B/D)** were treated for 48 h ± MitoQ (100 nM). MDA-MB-231 **(A)** (n = 6) and SkBr3 **(B)** (n = 3-8) cancer cell migration in a scratch test assay. MDA-MB-231 **(C)** and SkBr3 **(D)** cell invasion in a Boyden chamber assay. All data are shown as means ± SEM. *** P < 0.001 compared to control; by Student t test **(C-D)** or 2-way ANOVA **(A-B).**

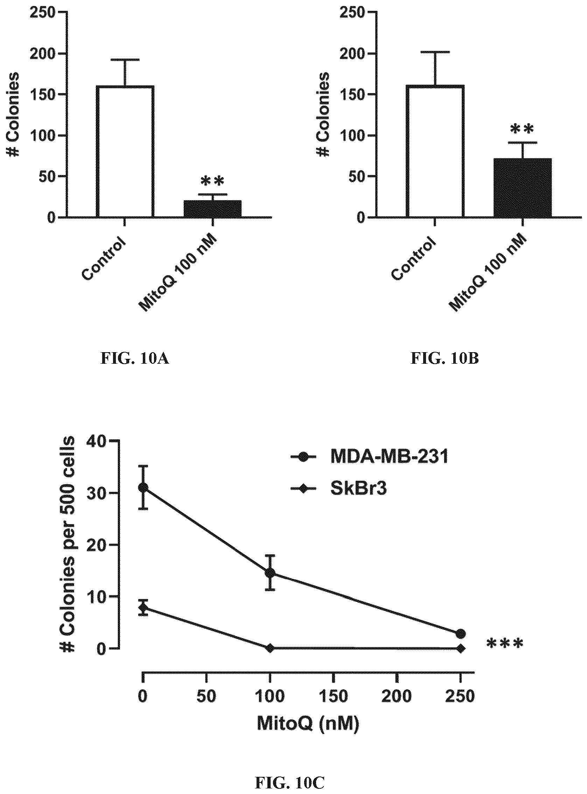

**Figure 10A-C** is a set of graphs showing that MitoQ represses breast cancer clonogenicity. Cells were pretreated for 48 h with the indicated doses of mitoQ. **(A-B)** Clonogenic assay using adherent MDA-MB-231 **(A)** (n = 6) and SkBr3 **(B)** (n = 9) cells. **(C)** Clonogenic assay on soft agar using MDA-MB-231 and SkBr3 cells (n = 16 all). All data are shown as means ± SEM. ** P < 0.01, *** P < 0.001 compared to control; by Student t test (A-B) or 2-way ANOVA (C).

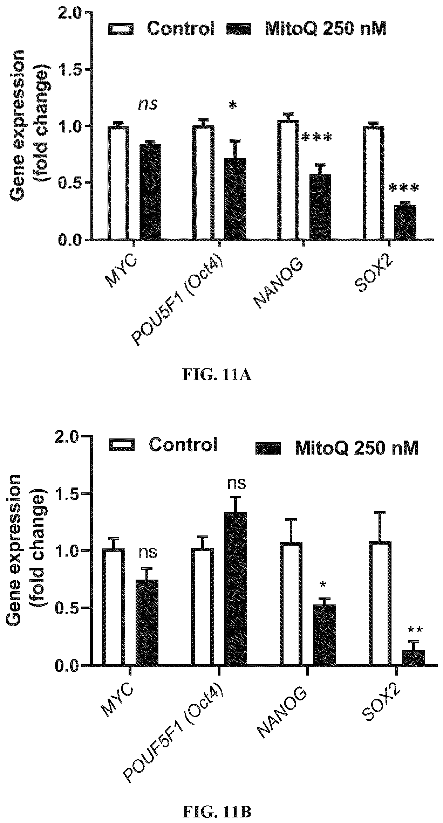

**Figure 11A-B** is a set of graphs showing that MitoQ partially represses breast cancer stemness. Cells were pretreated for 48 h with the indicated doses of MitoQ. mRNA expression of cancer stem cell markers MYC, POU5F1/(Oct4), NANOG and SOX2 in **(A)** MDA-MB-231 spheroids (n = 3-7) and **(B)** SkBr3 spheroids (n = 4-6). All data are shown as means ± SEM. * P < 0.05, ** P < 0.01, *** P < 0.001, ns= non-significant, compared to control; by Student t test.

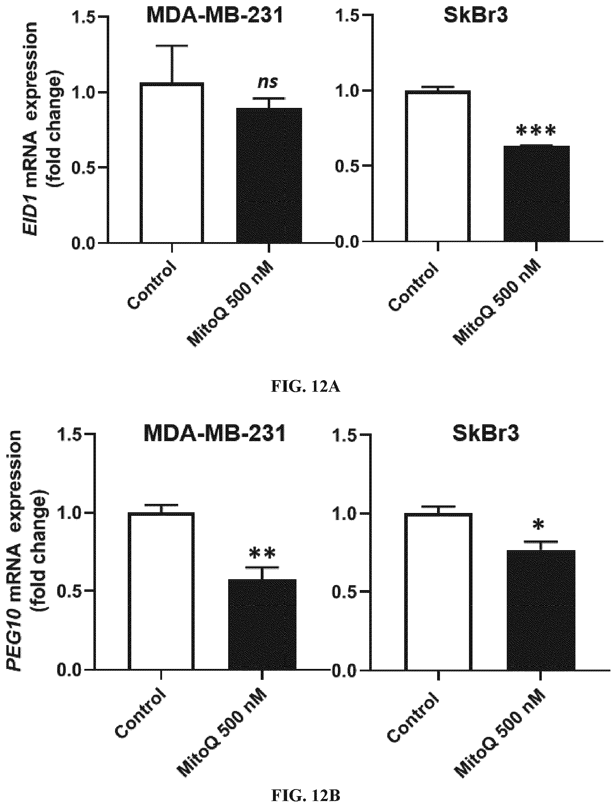

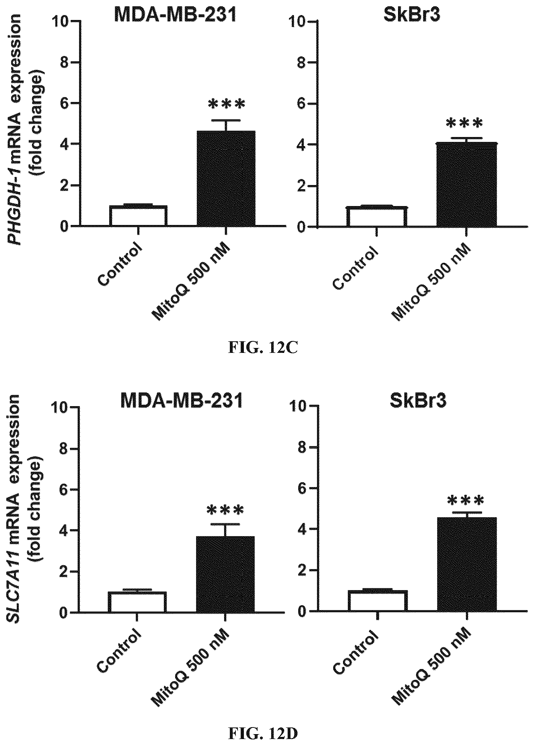

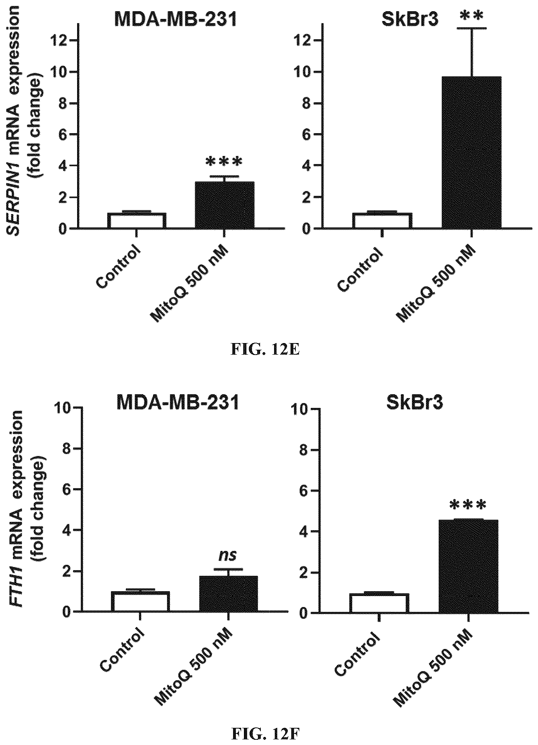

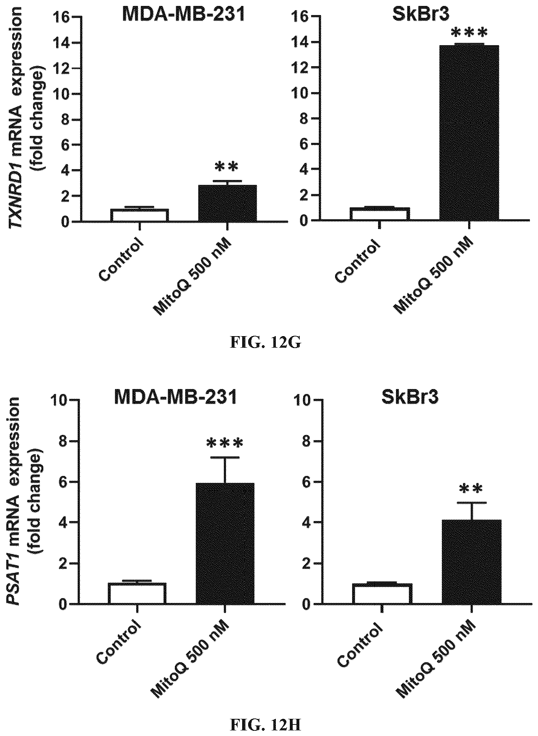

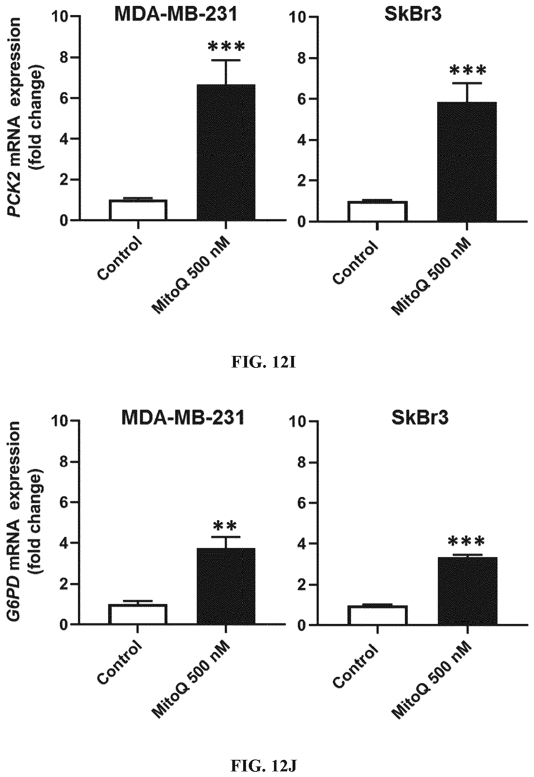

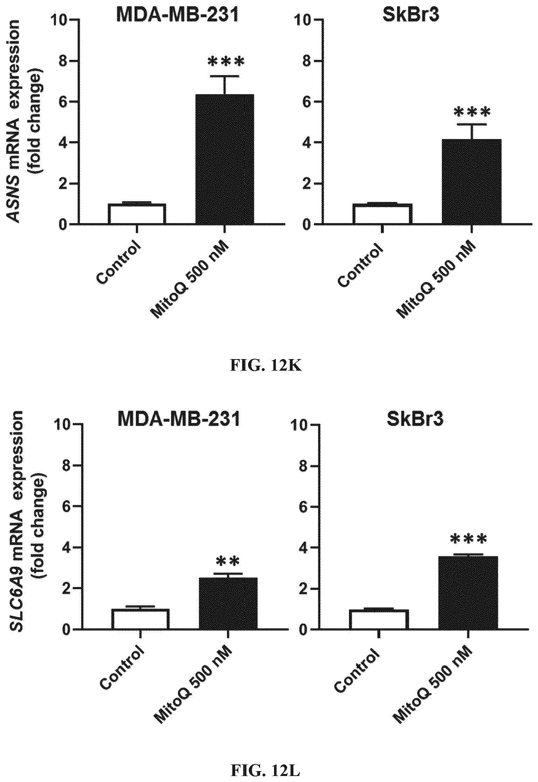

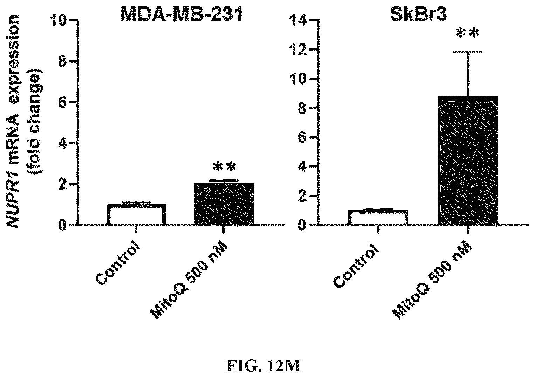

**Figure 12A-M** is a set of graphs showing the MitoQ modulates the transcription of metabolic genes in human breast cancer cells. **(A-M).** MDA-MB-231 (left panels) and SkBR3 (right panels) cells were treated for 48 h ± 500 nM MitoQ, after which mRNA transcripts were analyzed using RNAseq. Thresholds were |log 2 Fold Change| > 1 and P < 0.01 for all analyses. **(A)** EID1 expression (n = 3 all). **(B)** PEG10 expression (n = 3 all). **(C)** PHGDH-1 expression (n = 12 for MDA-MB-231, n = 9 for SkBR3). **(D)** SLC7A11 expression (n = 12 for MDA-MB-231, n = 9 for SkBR3). **(E)** SERPINE1 expression (n = 10-12 for MDA-MB-231, n = 6-9 for SkBR3). **(F)** FTH1 expression (n = 3 all). **(G)** TXNRD1 expression (n = 3 all). **(H)** PSAT1 expression (n = 12 for MDA-MB-231, n = 9 for SkBR3). **(I)** PCK2 expression (n = 12 for MDA-MB-231, n = 9 for SkBR3). **(J)** G6PD expression (n = 3 all). **(K)** ASNS expression (n = 12 for MDA-MB-231, n = 9 for SkBR3). **(L)** SCL6A9 expression (n = 3 all). **(M)** NUPR1 expression (n = 3 for MDA-MB-231, n = 3-6 for SkBR3). All data are shown as means ± SEM. * P < 0.05, ** P < 0.01, *** P < 0.001, ns P > 0.05, by Student t test.

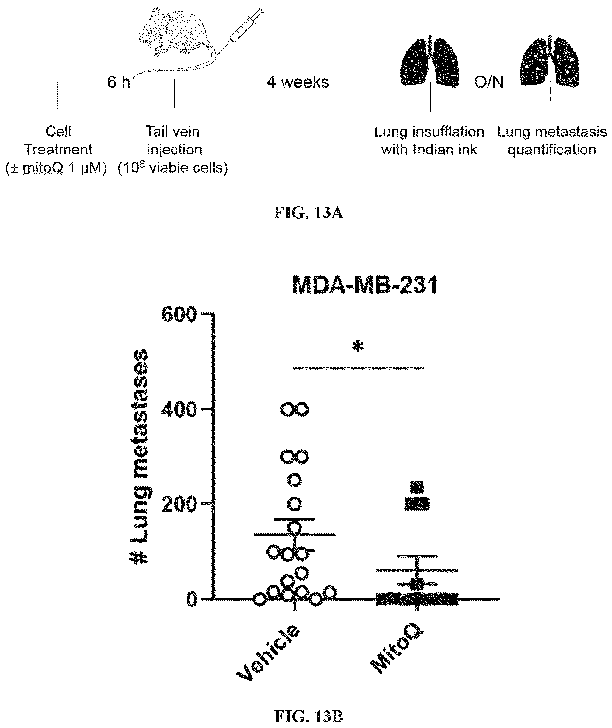

**Figure 13A-B** is a set of graphs showing that MitoQ inhibits the metastatic take of triple-negative human breast cancer cells in mice. **(A)** Experimental protocol for tumor take assays where breast cancer cells were pretreated ± 1 µM MitoQ for 6 h before tail vein injection of viable cells in female NMRI nude mice. **(B)** Metastatic take in the lungs of MDA-MB-231 human breast cancer cells (control group n = 18; MitoQ group n = 11). All data are shown as means ± SEM. * P < 0.05 compared to vehicle, by Mann-Whitney test (B).

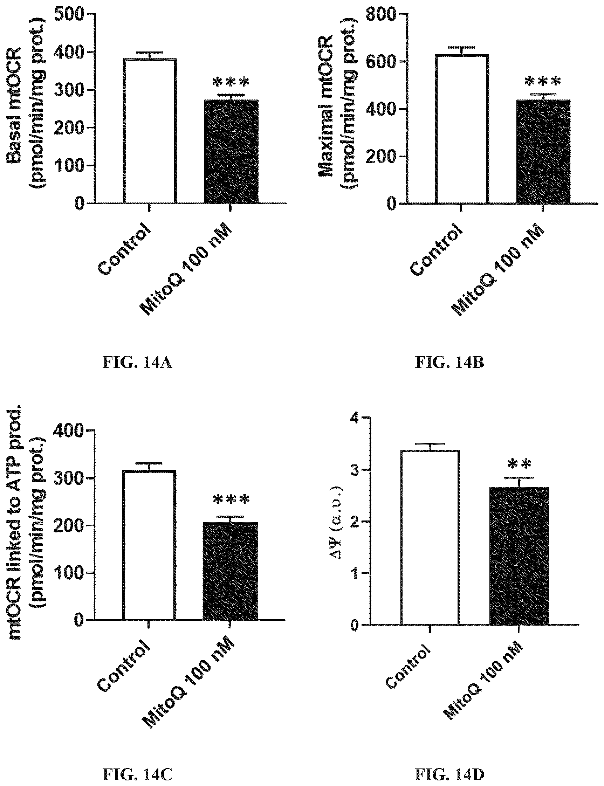

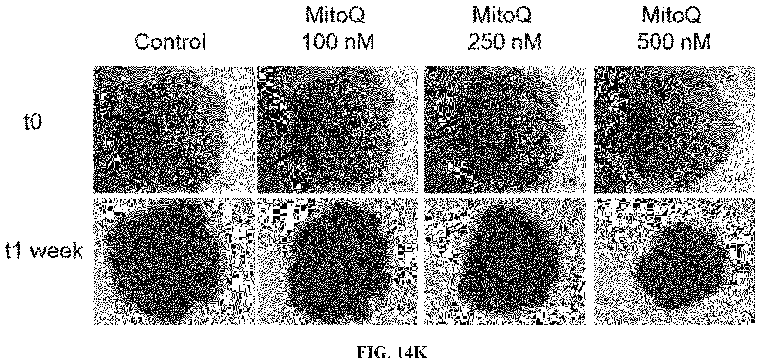

**Figure 14A-K** is a set of graphs and photographs showing that MitoQ reduces the prometastatic traits of human MDA-MB-436 breast cancer cells *in vitro.* **(A-E)** Human MDA-MB-436 cancer cells were treated for 48 h ± the indicated doses of MitoQ. **(A)** Basal, **(B)** maximal and **(C)** ATP-linked oxygen consumption rates (OCRs) were measured using Seahorse oximetry (n =14-16). **(D)** Mitochondrial potential (Δψ) was measured using JC-10 on a SpectraMax i3 spectrophotometer (n = 8). **(E)** Mitochondrial superoxide production was measured using electron paramagnetic resonance (EPR) with MitoTEMPO-H as a specific mitochondrial superoxide sensor ± PEG-SOD2 (n = 4). **(F)** Direct MDA-MB-436 cell counting on a SpectraMax i3 spectrophotometer at the indicated time points after treatment with increasing doses of MitoQ (0 to 500 nM) (n = 7). **(G)** MDA-MB-436 cells were treated for 48 h ± 500 nM MitoQ, and gene expression profile of VIM, SNAI1, SNAI2, ZEB1 and TWIST was assessed (expressed as fold change). **(H)** mRNA expression of EMT markers vimentin (VIM), SNAIL (SNAI1), SLUG (SNAI2), ZEB1 and TWIST1 in cells treated ± 500 nM of MitoQ (n = 6-9). **(I)** Cell migration in a scratch test (n = 6). **(J)** Clonogenic assay on soft agar (n = 3). **(K)** Spheroid formation over 1 week, with cells treated with increasing doses of MitoQ. All data are shown as means ± SEM. * P < 0.05, ** P < 0.01, *** P < 0.001, ns= non-significant, compared to control; by Student t test **(A-E, F, I)** or 2-way ANOVA with Tukey's post-hoc test **(E, H).**

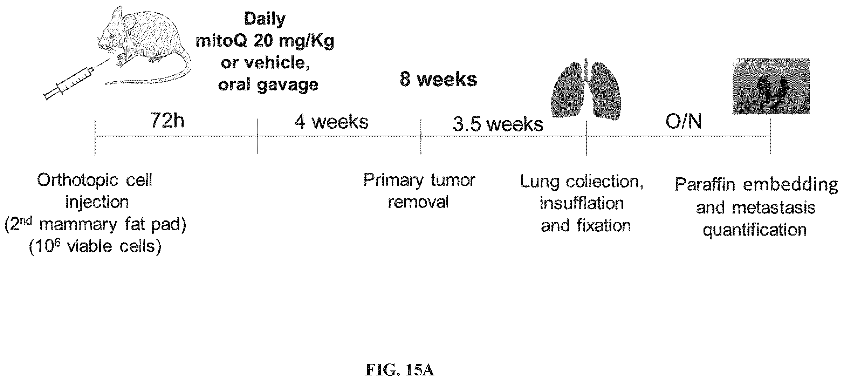

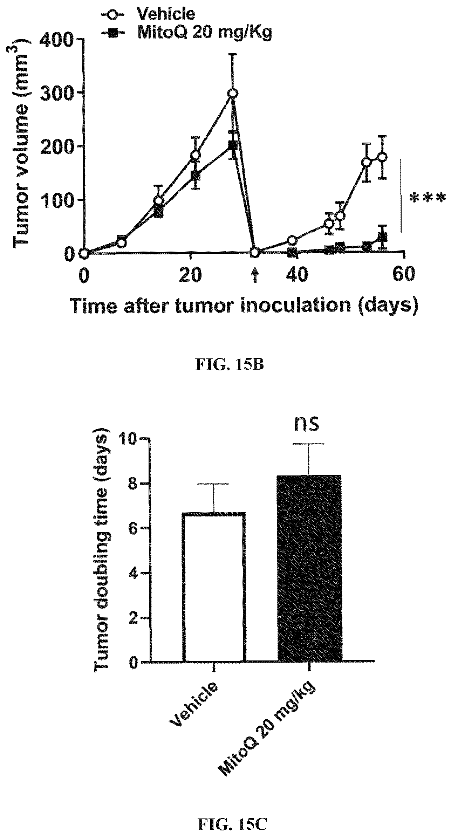

**Figure 15A-E** is a set of schemes and graphs showing that MitoQ prevents primary tumor recurrence and metastatic dissemination of human triple-negative breast cancer in mice. **(A)** Experimental protocol for spontaneous MDA-MB-231 metastasis after orthotopic injection in the mammary fat pad of female NMRI nude mice. **(B-C)** Primary tumor growth in mice using the protocol shown in **(A)** (n = 10 per group until the day of surgery; n = 7 in the vehicle-treated group and n = 8 in the MitoQ-treated group after surgery). **(D)** Kaplan-Meier graph showing recurrence-free mouse survival after surgery. Mice were all from **(B-C)** (n = 7 in the vehicle-treated group and n = 8 in the MitoQ-treated group). **(E)** At the end of the protocol shown in **(A),** mouse lungs were removed, sliced, stained for cytokeratine 19 (CK19), counterstained with hematoxylin and eosin, and analyzed for the presence of metastases and metastasis quantification was performed. Data are for 2 independent experiments, including mice from **(B-C)** (n = 21 in the control group and n = 19 in the MitoQ group). All data are shown as means ± SEM. * P < 0.05, ** P < 0.01, *** P < 0.001, ns= non-significant, compared to vehicle; by 2-way ANOVA with Sidak's multiple comparisons test post-hoc test **(B-C),** Log-Rank (Mantel-Cox) test **(D),** or Student t test **(E).**

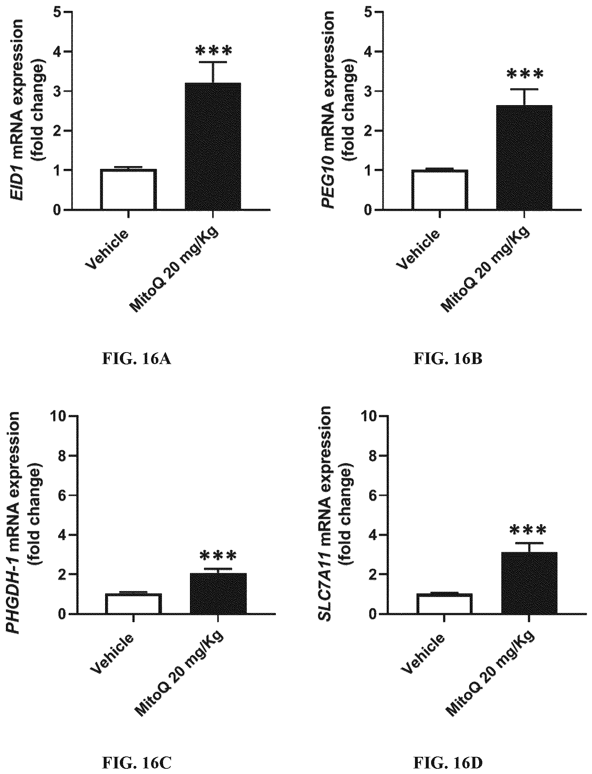

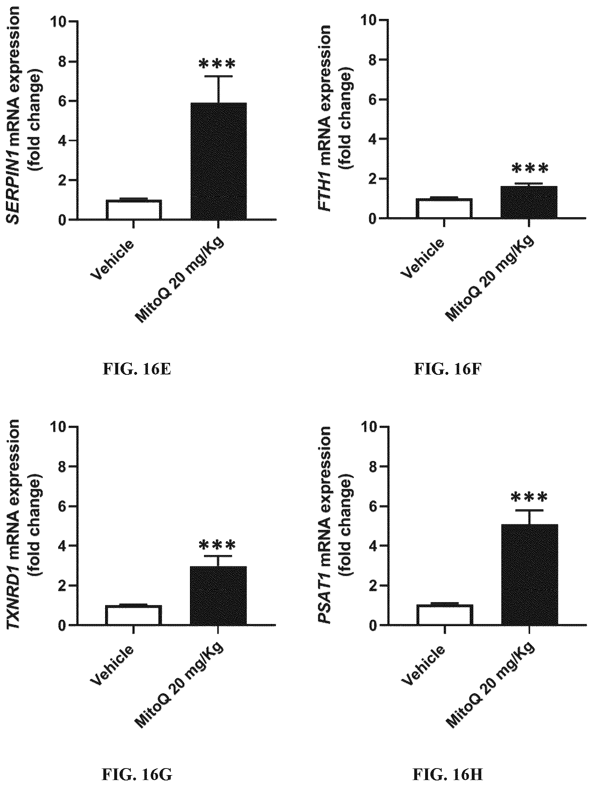

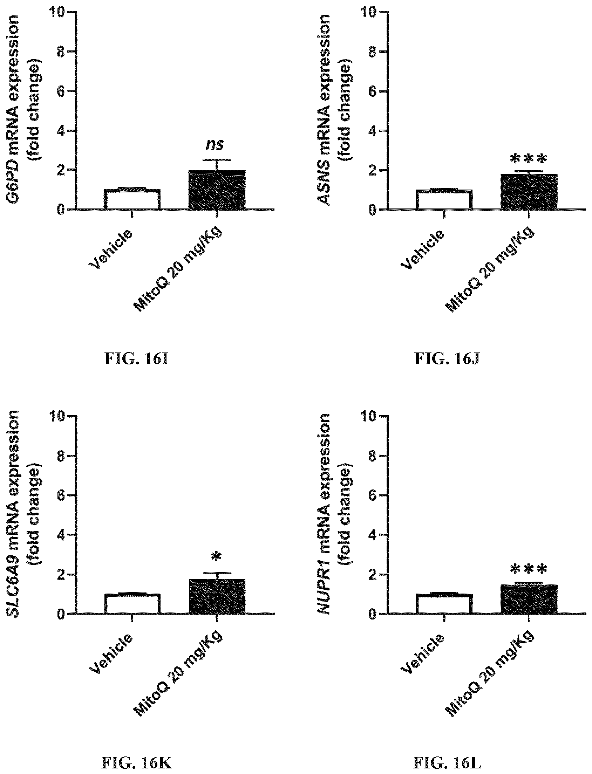

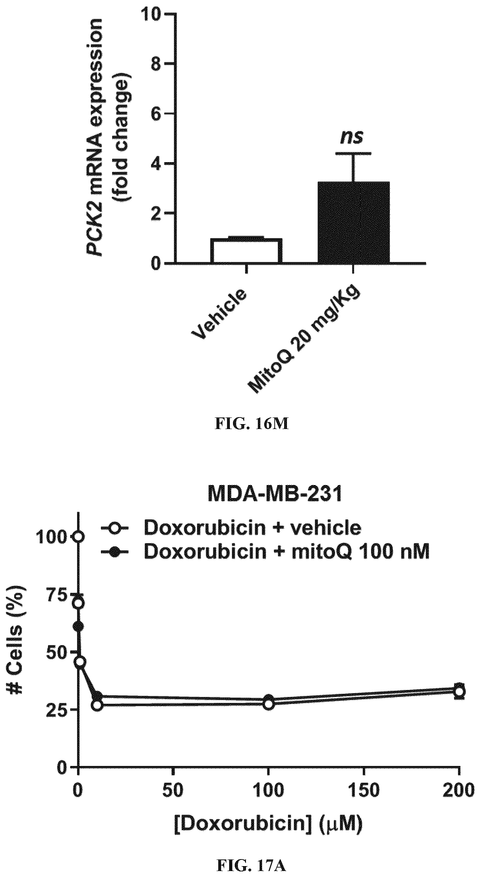

**Figure 16 A-M** is a set of graphs showing the *in vitro* validation of a potential mRNA signature of human breast cancer cell response to mitoQ. Bulk primary human MDA-MB-231 tumors resected on day +32 in the protocol depicted in **Figure 16A****,** were analyzed using RT-qPCR for **(A)** EID1 (n = 28-30), **(B)** PEG10 (n = 30), **(C)** PHGDH-1 (n = 29), **(D)** SLC7A1 1 (n = 29-30), **(E)** SERPINE1 (n = 29-30), **(F)** FTH1 (n = 29-30), **(G)** TXNRD1 (n = 30), **(H)** PSAT1 (n = 29-30), **(I)** G6PD (n = 28-30), **(J)** ASNS (n = 29), **(K)** SLC6A9 (n = 30), **(L)** NUPR1 (n = 30) and **(M)** PCK2 (n = 30) mRNA expression. All data are shown as means ± SEM. * P < 0.05, *** P < 0.001, ns P > 0.05 compared to control, by Student t test.

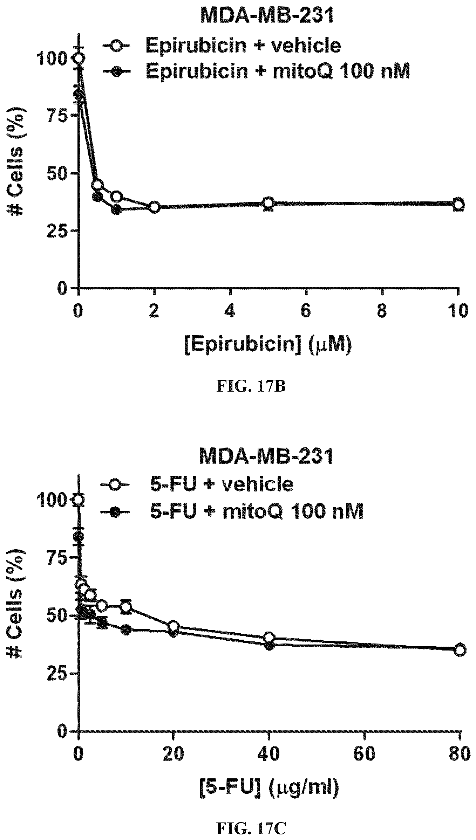

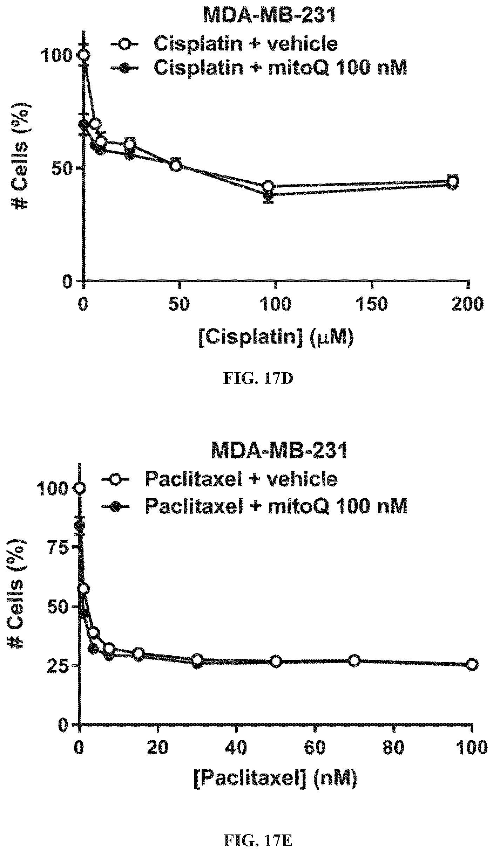

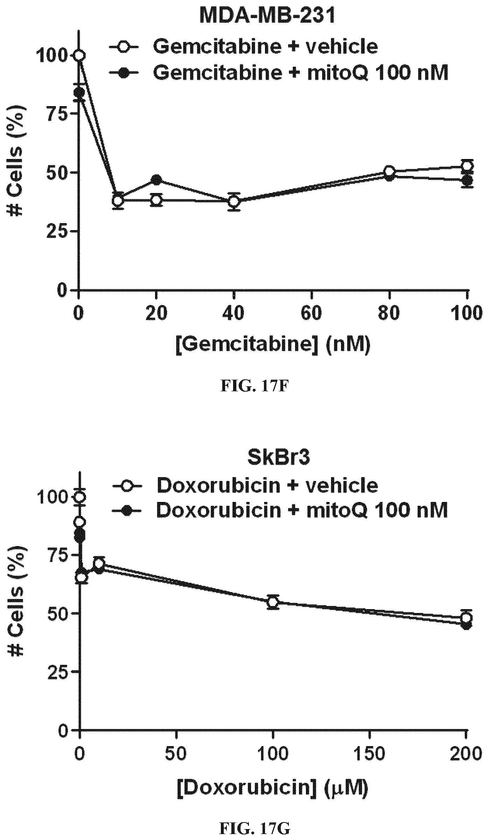

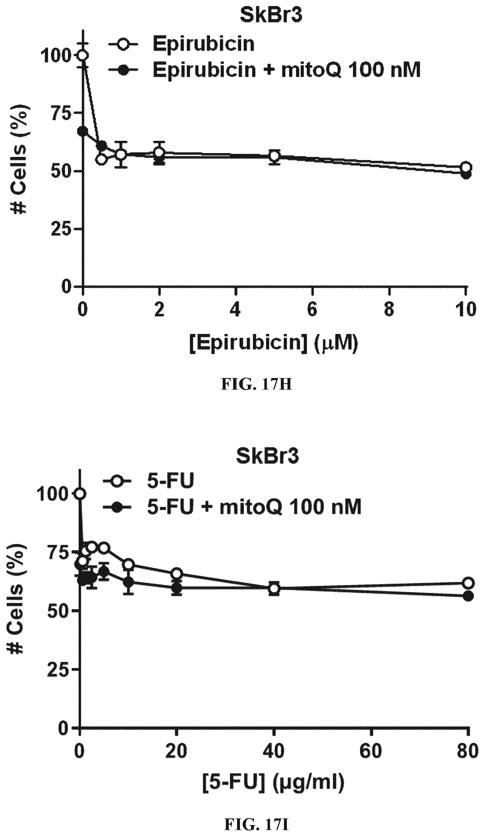

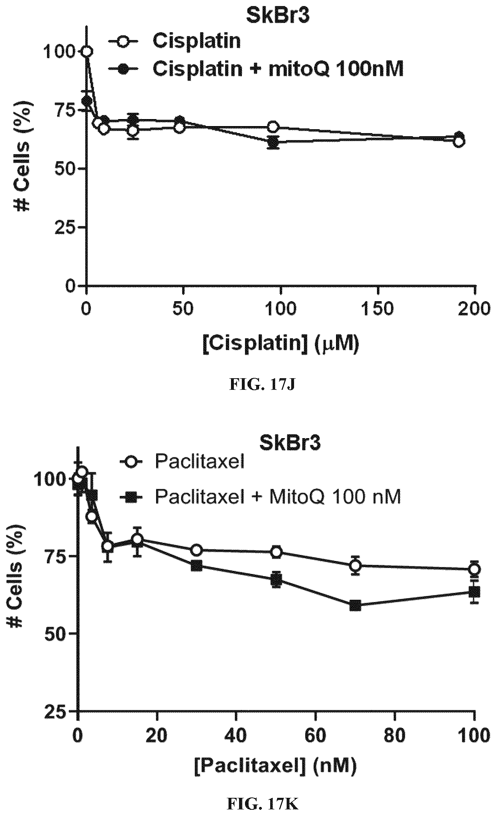

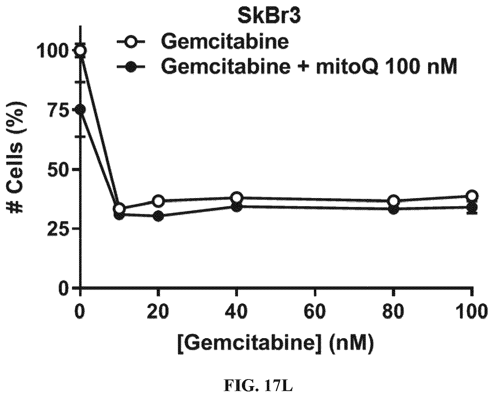

**Figure 17A-L** is a set of graphs showing that MitoQ does not interfere with conventional chemotherapies used to treat breast cancer. **(A-F)** Direct counting of MDA-MB-231 human breast cancer cells treated with increasing doses of chemotherapy on a SpectraMax i3 spectrophotometer. **(A)** Cell treated for 48 h with doxorubicin ± 100 nM mitoQ (n = 3). **(B)** Cell treated for 24 h with epirubicin ± 100 nM mitoQ (n = 3). **(C)** Cell treated for 48 h with 5-fluorouracil (5-FU) ± 100 nM mitoQ (n = 3). **(D)** Cell treated for 48 h with cisplatin ± 100 nM mitoQ (n = 3). **(E)** Cell treated for 48 h with paclitaxel ± 100 nM mitoQ (n = 3). **(F)** Cell treated for 72 h with gemcitabine ± 100 nM mitoQ (n = 3). **(G-L)** Direct counting of SkBr3 human breast cancer cells treated with increasing doses of chemotherapy. **(G)** Cell treated for 48 h with doxorubicin ± 100 nM mitoQ (n = 3). **(H)** Cell treated for 24 h with epirubicin ± 100 nM mitoQ (n = 3). **(I)** Cell treated for 48 h with 5-fluorouracil (5-FU) ± 100 nM mitoQ (n = 3). **(J)** Cell treated for 48 h with cisplatin ± 100 nM mitoQ (n = 3). **(K)** Cell treated for 48 h with paclitaxel ± 100 nM mitoQ (n = 3-4). **(L)** Cell treated for 72 h with gemcitabine ± 100 nM mitoQ (n = 3).

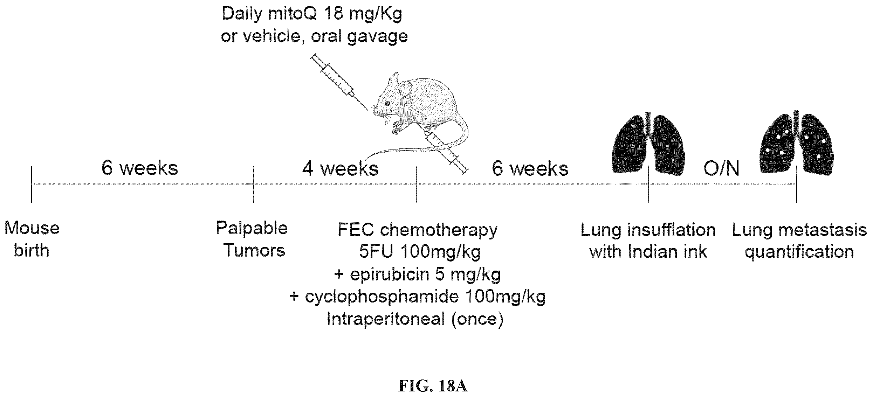

**Figure 18A-C** is a scheme and a set of graphs showing that MitoQ prevents metastatic dissemination in MMTV-PyMT mice that spontaneously develop metastatic breast cancer. **(A)** Experimental protocol for MMTV-PyMT mouse treatment. Note that all mice were treated with FEC chemotherapy. **(B)** Total weight of primary tumors collected on the day of sacrifice of MMTV-PyMT mice treated as depicted in **(A)** (n = 11-17). **(C)** Number of surface lung metastases in MMTV-PyMT mice treated as depicted in **(A)** (n = 15-21). All data are shown as means ± SEM. * P < 0.05, ns= non-significant, compared to vehicle, by Mann-Whitney test **(B-C).**

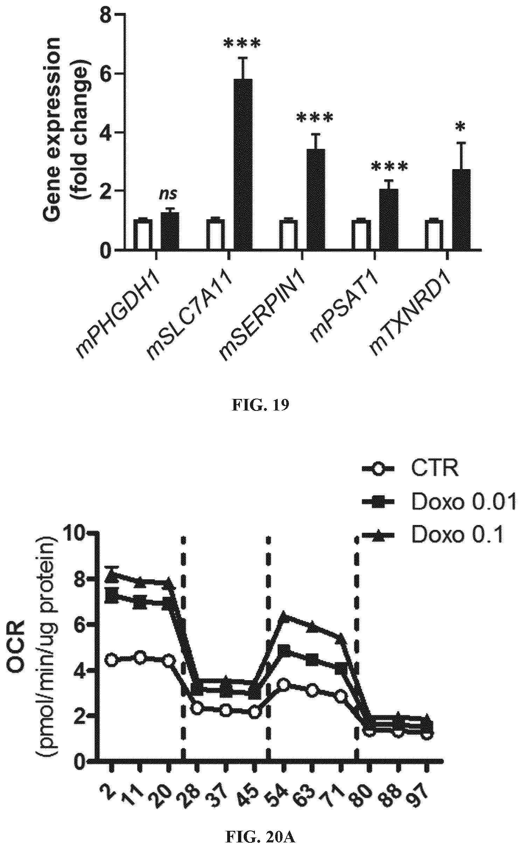

**Figure 19** is a graph showing the validation in MMTV-PyMT mice of the biomarker signature of MitoQ response in primary breast cancer. Primary breast tumors that spontaneously developed in MMTV-PyMT mice were collected at the end of the experiment depicted in **Figure 18A****.** They were analyzed using RT-qPCR for the expression of mouse transcripts of PHGDH-1, SLC7A11, SERPINE1, PSAT1 and TXNRD1 (n = 24-33). All data are shown as means ± SEM. ** P < 0.01, *** P < 0.001, ns P > 0.05 compared to control, by Student t test.
**Figure 20A-C** is a set of graphs showing that low doses of doxorubicin induces proton leak and mitochondrial superoxide production in breast cancer cells. **(A)** Human MCF7 breast cancer cells were treated for 24 h with increasing doses of doxorubicin (DXR), after which oxygen consumption rate (OCR) was measured with a Seahorse XF96 bioanalyzer. Representative Seahorse OCR traces are shown on the left, and the graph on the right reports on mitochondrial and non-mitochondrial OCRs normalized by total protein content (n = 7). **(B-C)** MCF7 cells were treated for 24 h with 0.1 µg/mL of DXR, after which glucose uptake and lactate release were measured enzymatically. Data were normalized by total protein content (n = 5-6). All data are shown as means ± SEM. * P < 0.05, ** P < 0.01, *** P < 0.005, ns: P < 0.05 compared to control; by one-way ANOVA with Dunnett's post-hoc test **(A, B)** or Student's t test **(C).**

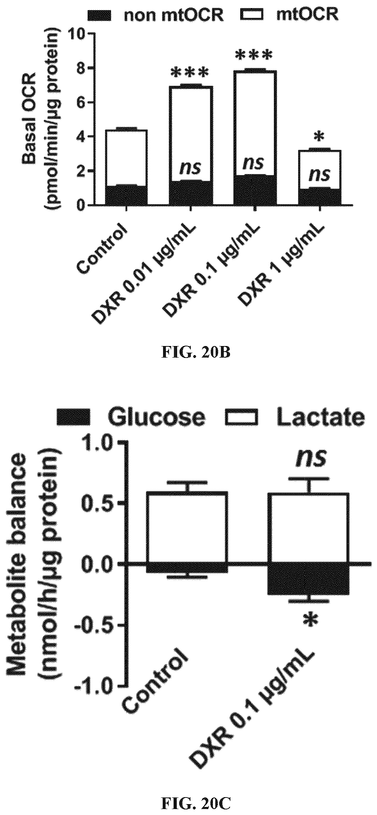

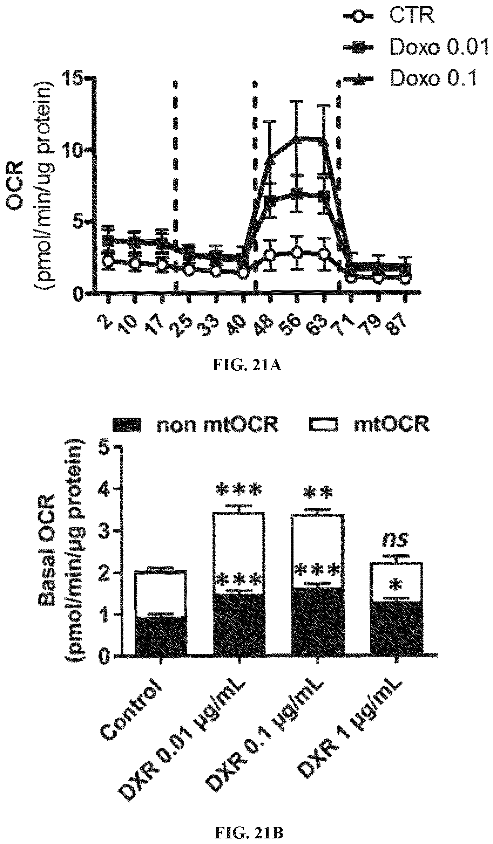

**Figure 21A-C** is a set of graphs showing that low doses of doxorubicin induces proton leak and mitochondrial superoxide production in breast cancer cells. **(A)** As in **Figure 20A****,** but using mouse 4T1 breast cancer cells (n = 7). (B-C) As in **Figure 20B-****C,** but using 4T1 cells (n = 5). All data are shown as means ± SEM. * P < 0.05, ** P < 0.01, *** P < 0.005, ns: P < 0.05 compared to control; by one-way ANOVA with Dunnett's post-hoc test **(A, B)** or Student's t test (C).

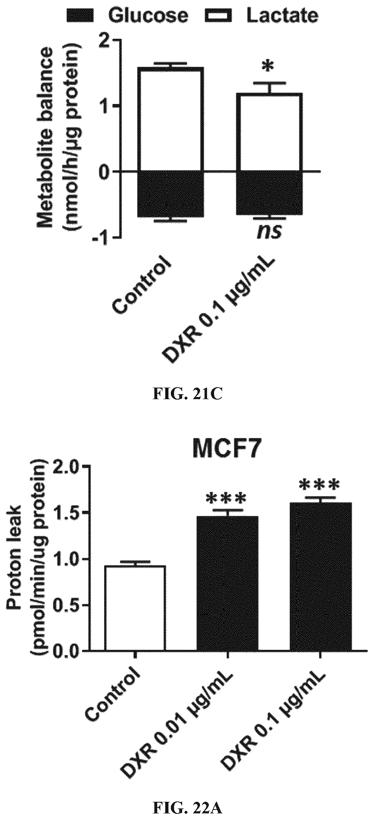

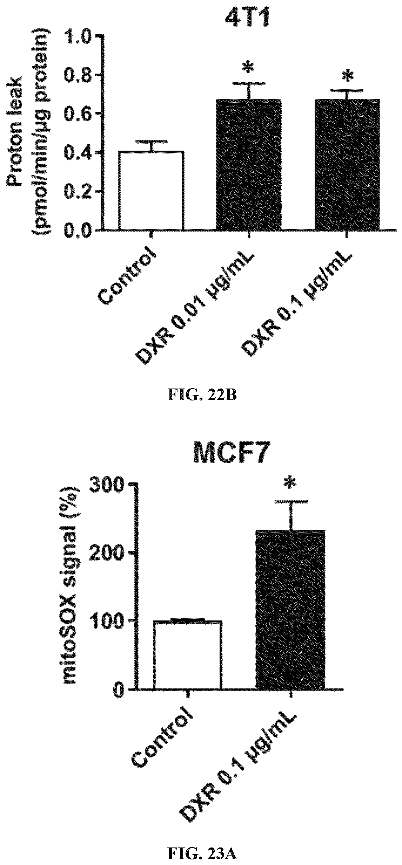

**Figure 22A-B** is a set of graphs showing that low doses of doxorubicin induces proton leak and mitochondrial superoxide production in breast cancer cells. Cells were treated for 24 h with increasing doses of DXR, after which proton leak at the electron transport chain of **(A)** MCF7 (n = 8) and **(B)** 4T1 (n = 5-7) cells was measured with a Seahorse XF96 bioanalyzer. All data are shown as means ± SEM. * P < 0.05, ** P < 0.01, *** P < 0.005, ns: P < 0.05 compared to control; by one-way ANOVA with Dunnett's post-hoc test **(A, B).**

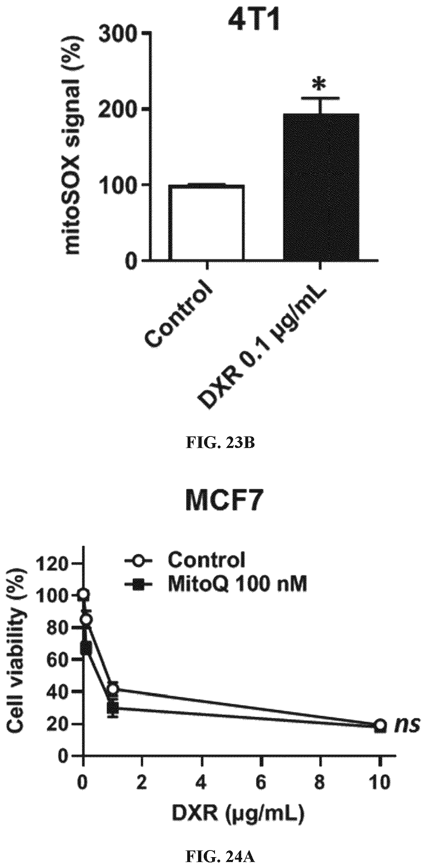

**Figure 23A-B** is a set of graphs showing mitochondrial superoxide levels in breast cancer cells after treatment with doxorubicin (DXR). **(A)** MCF7 (n = 5) and **(B)** 4T1 (n = 3) cells were treated for 24 h with 0.1 µg/mL of DXR, after which mitochondrial superoxide levels were measured by FACS analysis using mitochondria-targeted fluorescent probe mitoSOX. Representative graphs on the left show data plotted as population distribution, and graphs on the right the mean fluorescent intensity (MFI) normalized to vehicle-treated cells (control). All data are shown as means ± SEM. * P < 0.05, ** P < 0.01, *** P < 0.005, ns: P < 0.05 compared to control; by Student's t test (A, **B).**

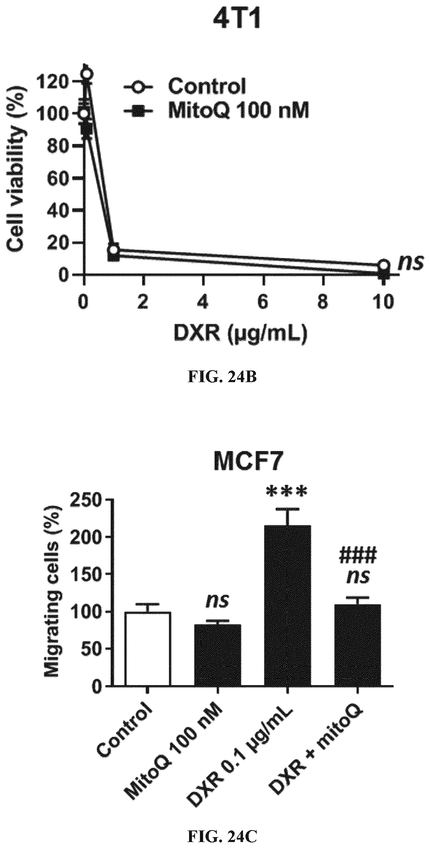

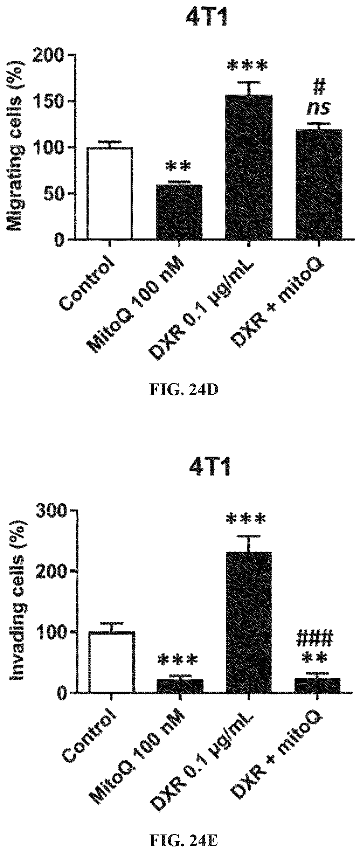

**Figure 24A-E** is a set of graphs showing that MitoQ inhibits doxorubicin-induced cancer cell migration and invasion. **(A)** Human MCF7 (n = 11-24) and **(B)** murine 4T1 (n = 11-12) breast cancer cells were treated for 48 h with increasing doses of DXR ± 100 nM of MitoQ, after which cell viability was determined using a CellTiter-Glo Luminescent Cell Viability assay. **(C-E)** Cells were pretreated with the indicated amounts drugs for 16 h, and left to recover for 6 h. **(C)** MCF7 (n = 6) and **(D)** 4T1 (n = 5-6) migration towards 0.5% serum after treatment with 0.1 µg/mL of DXR ± 100 nM of MitoQ. **(E)** As in **(C-D)** but reporting on invasion (4T1, n = 5-8). All data are shown as means ± SEM normalized to control. * P < 0.05, ** P < 0.01, *** P < 0.005, ns: P < 0.05 compared to control; # P < 0.05, ## P < 0.01, ### P < 0.005 compared to DXR treatment alone; by two-way ANOVA **(A-B)** or one-way ANOVA with Dunnett's post-hoc test **(C-E).**

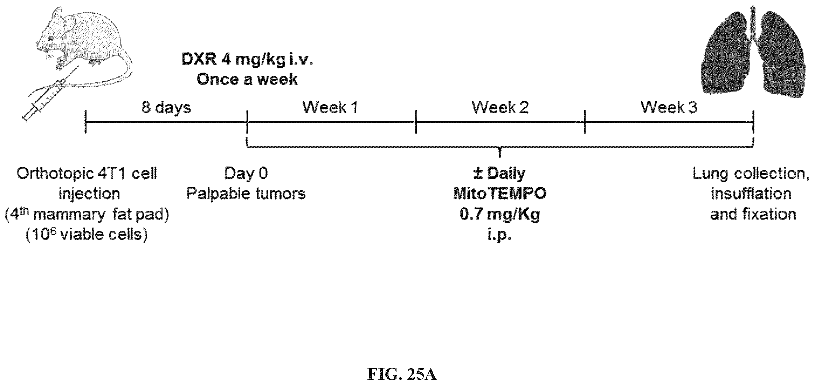

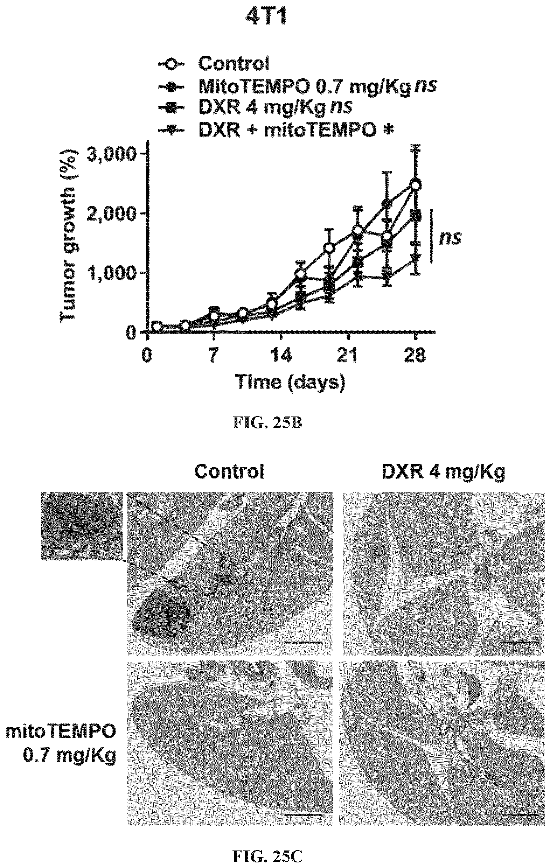

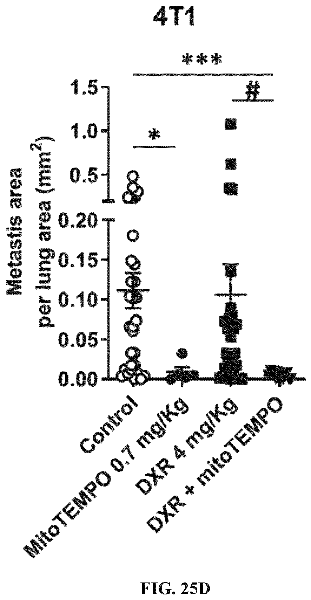

**Figure 25A-D** a scheme and a set of graphs showing that mitochondrial superoxide scavenging synergizes with doxorubicin in retarding breast tumor growth. **(A)** Scheme depicting the treatment of 4T1-bearing mice. **(B)** Primary tumor growth rate shown as volume fold change starting from day 0 (n = 12-16 mice per group). **(C)** Photographs of representative H&E-stained lung sections (left). Bars = 500 µm. **(D)** Determination of the metastasis-positive areas normalized to whole analyzed areas from (C) (n = 5-30 mice per group) using a Leica SCN400 slide scanner. All data are shown as means ± SEM. * P < 0.05, *** P < 0.005, ns: P < 0.05 compared to control; # P < 0.05 compared to DXR treatment alone; by two-way ANOVA **(B)** or Kruskal-Wallis test with Dunn's post-hoc test **(D).**

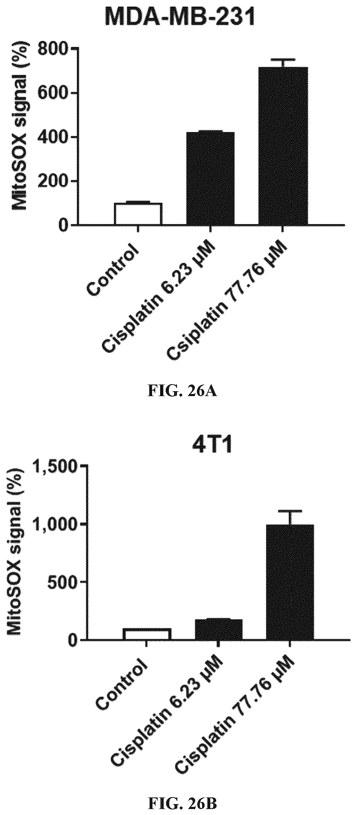

**Figure 26A-B** is a set of graphs showing that low doses of cisplatin stimulate mitochondrial superoxide production. **(A)** Human MDA-MB-231 (n = 2-3) and **(B)** murine 4T1 (n = 2-3) breast cancer cells were treated for 48 h with increasing doses of cisplatin, after which mitochondrial superoxide levels were measured by FACS analysis using mitochondria-targeted fluorescent probe mitoSOX. Representative graphs on the left show data plotted as population distribution, and graphs on the right the mean fluorescent intensity (MFI) normalized to vehicle-treated cells (control). All data are shown as means ± SEM normalized to control.

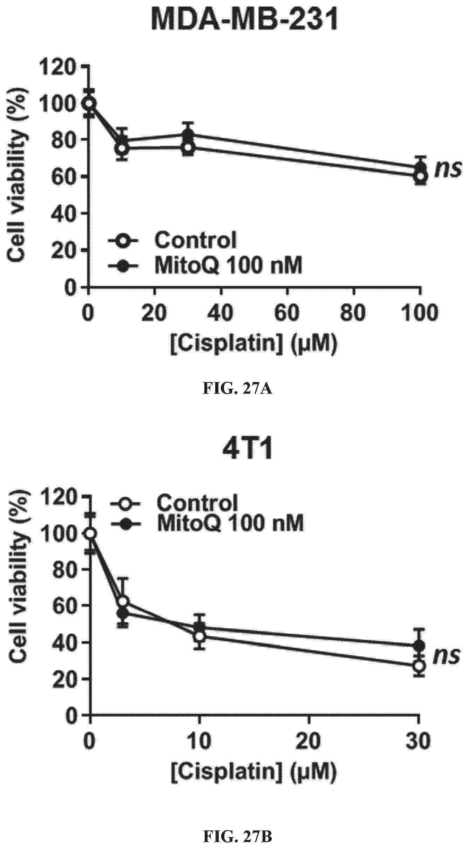

**Figure 27A-D** is a set of graphs showing that MitoQ inhibits cisplatin-induced cancer cell migration. **(A)** Human MDA-MB-231 (n = 18-20) and **(B)** murine 4T1 (n = 20) breast cancer cells were treated for 48 h with increasing doses of cisplatin ± 100 nM of MitoQ, after which cell viability was determined using a Crystal Violet Cell Viability assay. **(C-D)** Cells were pretreated with the indicated amounts drugs for 16 h, and left to recover for 6 h. MDA-MB-231 **(C,** n = 6) and 4T1 **(D,** n = 8-18) migration towards 0.5% serum after treatment with the indicated doses of cisplatin ± 100 nM of MitoQ. All data are shown as means ± SEM normalized to control. * P < 0.05, ** P < 0.01, *** P < 0.005, ns: P < 0.05 compared to control; ### P < 0.005 compared to Cisplatin 6,23 µM alone; by two-way ANOVA **(A)** or one-way ANOVA with Dunnett's post-hoc test **(B).**

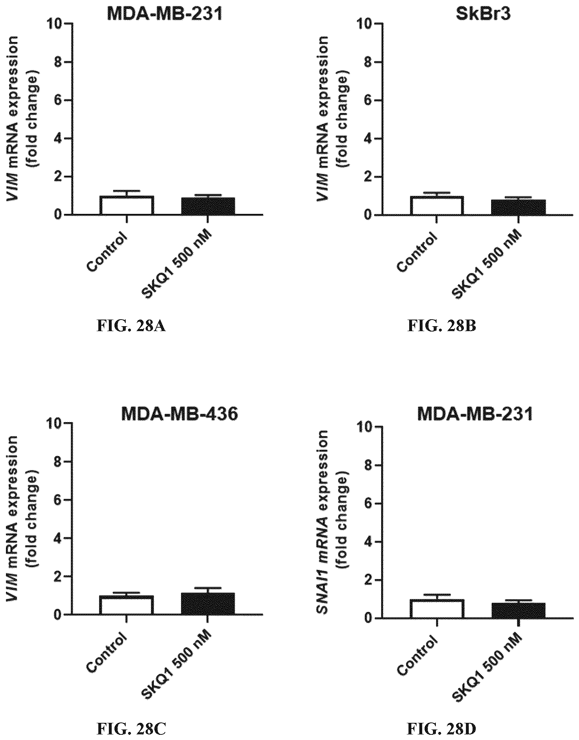

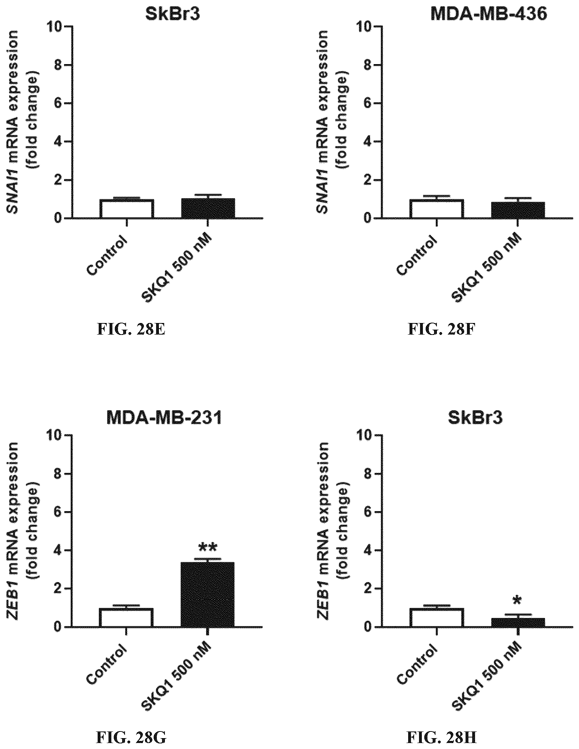

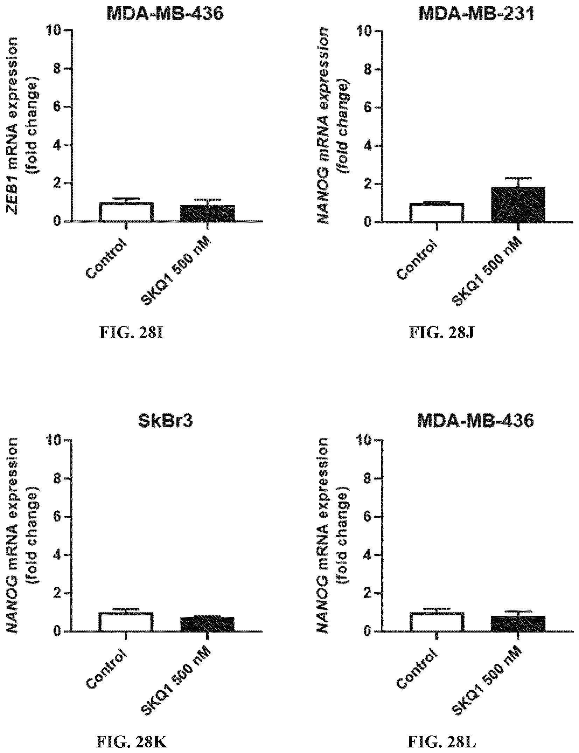

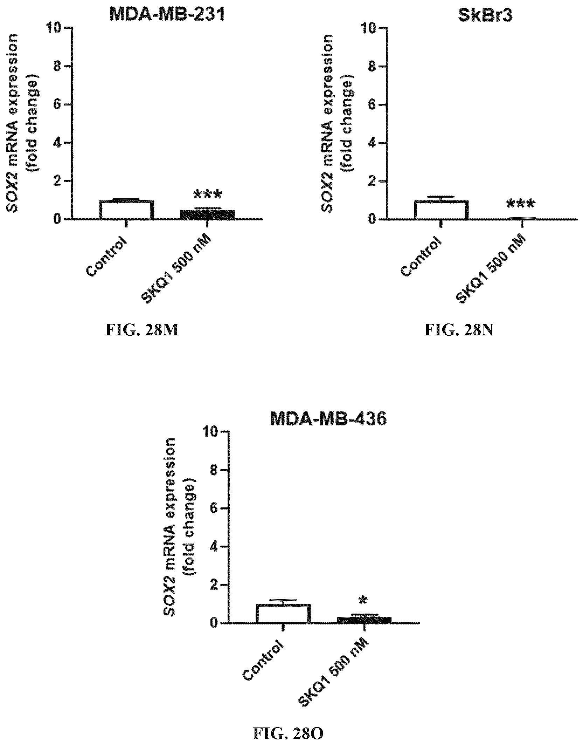

**Figure 28A-O** is a set of graphs showing that SKQ1 modulates the transcription of EMT- and stemness-related genes in human breast cancer cells. **(A-O)** MDA-MB-231 (top panels), SkBR3 (middle panels) and MDA-MB-436 (bottom panels) cells were treated for 48 h ± 500 nM SKQ1, after which mRNA transcripts were analyzed using RT-qPCR. **(A-C)** vimentin (VIM) expression (n = 4-6 for MDA-MB-231, n = 7 for SkBR3, n = 6 for MDA-MB-436). **(D-F)** SNAIL (SNAI1) (n = 6 for MDA-MB-231, n = 5 for SkBR3, n = 6 for MDA-MB-436). **(G-I)** ZEB1 (n = 6 for MDA-MB-231, n = 5-6 for SkBR3, n = 5-6 for MDA-MB-436). **(J-L)** NANOG (n = 5 for MDA-MB-231, n = 5-6 for SkBR3, n = 5-6 for MDA-MB-436). **(M-O)** SOX2 (n = 5 for MDA-MB-231, n = 5 for SkBR3, n = 5-6 for MDA-MB-436). * P < 0.05, ** P < 0.01, *** P < 0.005 compared to control; by Student t test.

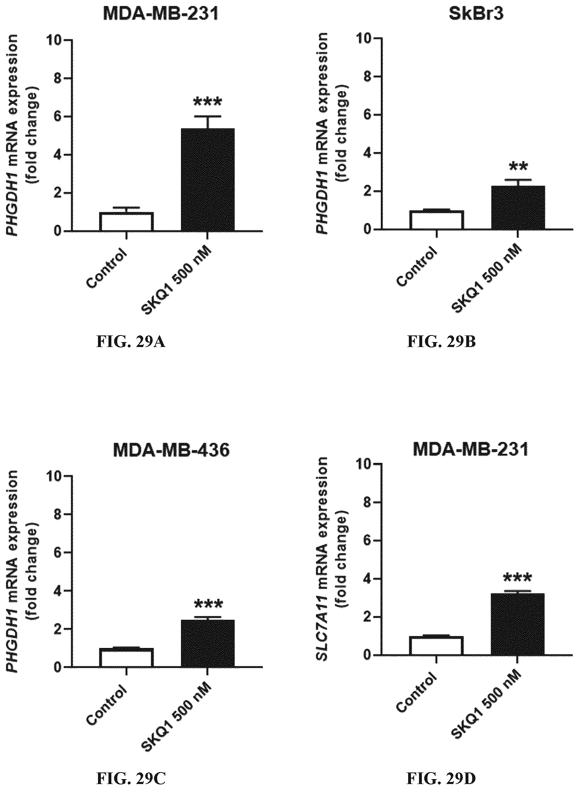

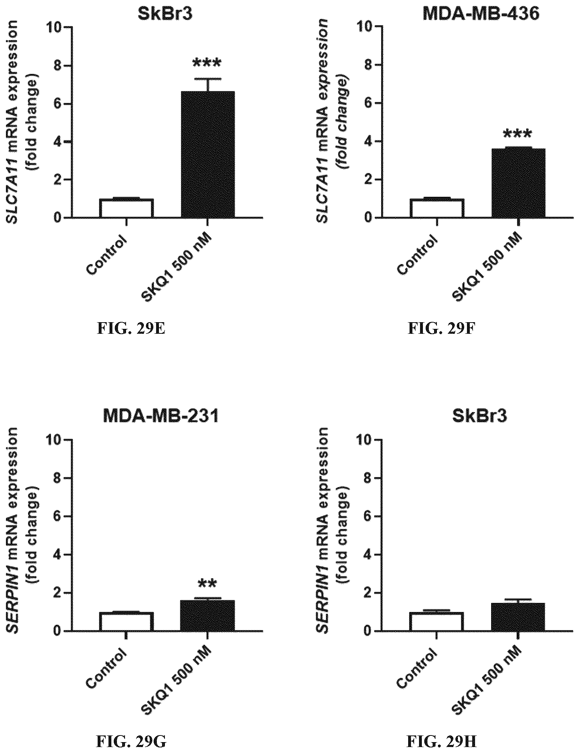

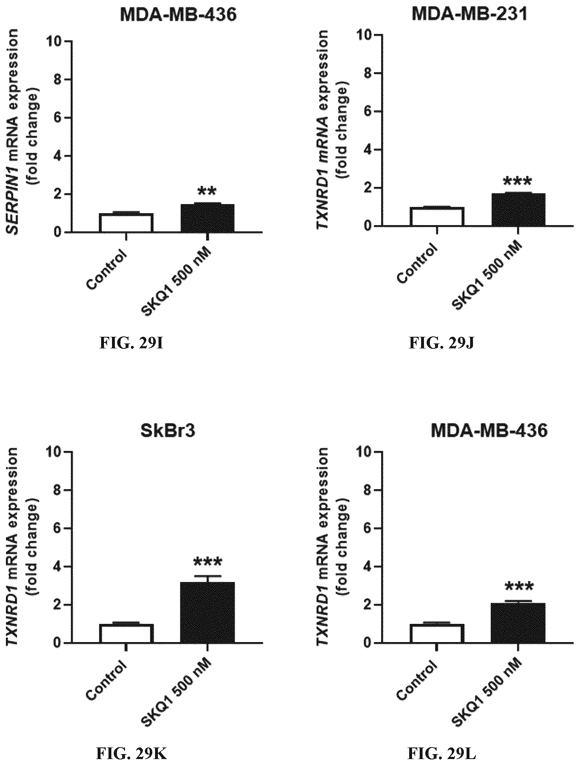

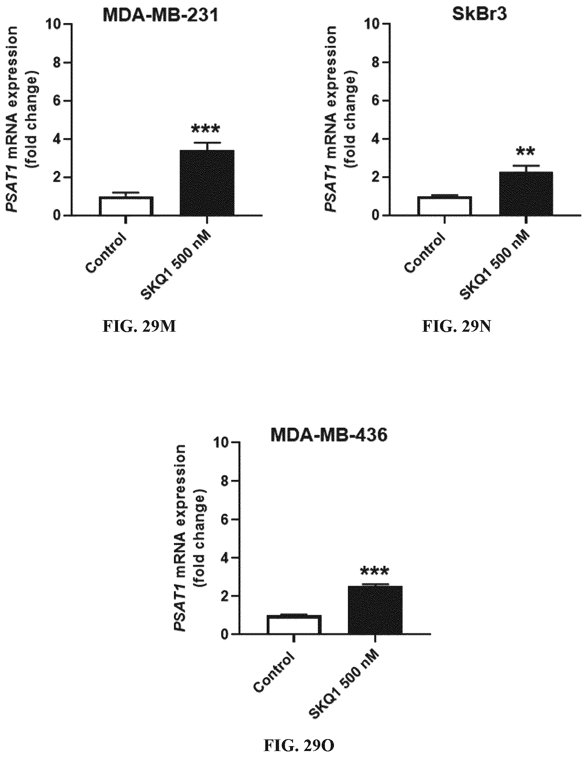

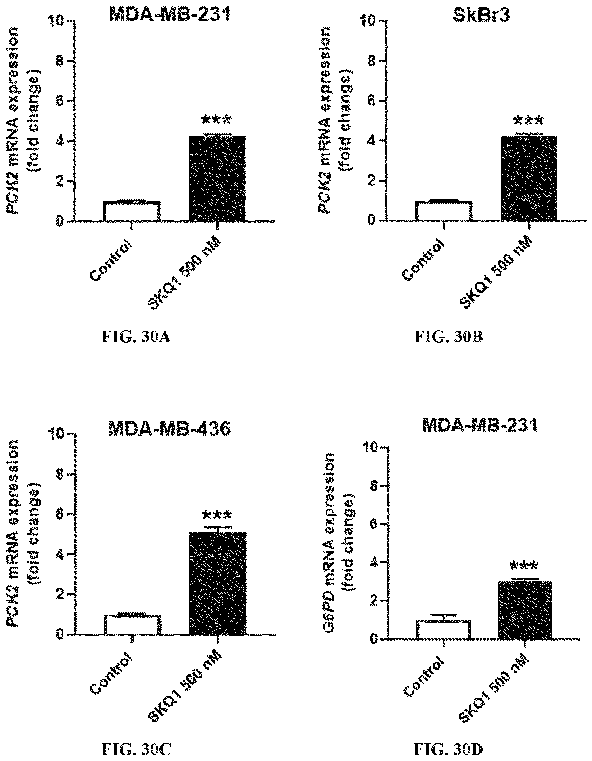

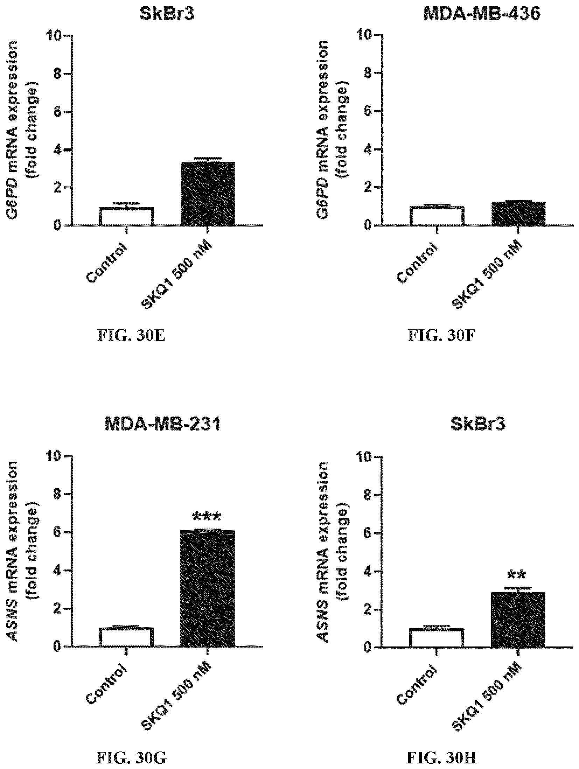

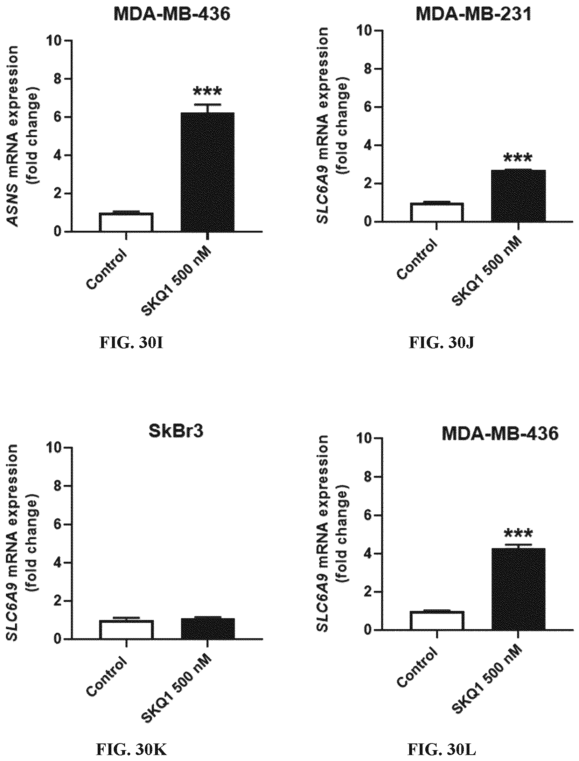

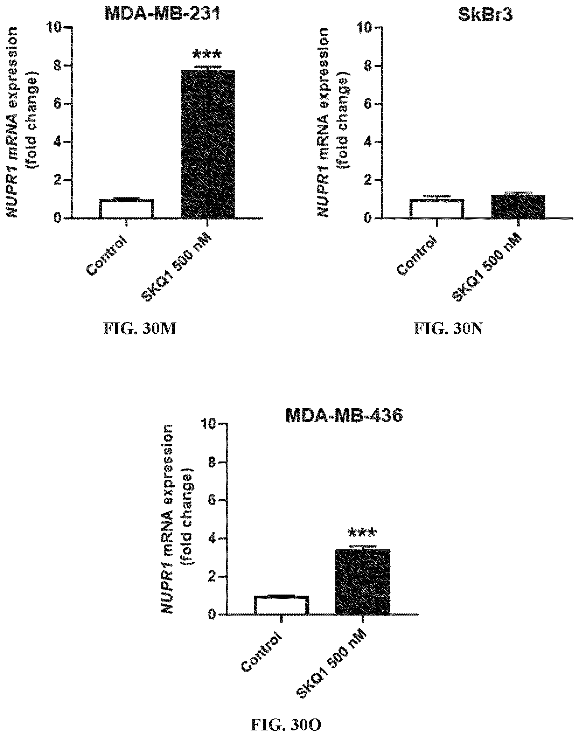

**Figure 29A-O** **and** **30A-O** is a set of graphs showing that SKQ1SkQ1 modulates the transcription of metabolic genes in human breast cancer cells. MDA-MB-231 (top panels), SkBR3 (middle panels) and MDA-MB-436 (bottom panels) cells were treated for 48 h ± 500 nM SKQ1, after which mRNA transcripts were analyzed using RT-qPCR. **(29A-B)** PHGDH-1 expression (n = 5-6 for MDA-MB-231, n = 5-6 for SkBR3, n = 3 for MDA-MB-436). **(29D-F)** SLC7A11 expression (n = 3 for MDA-MB-231, n = 6 for SkBR3, n = 3 for MDA-MB-436). **(29G-I)** SERPINE1 expression (n = 3 for MDA-MB-231, n = 6 for SkBR3, n = 3 for MDA-MB-436). **(29J-L)** TXNRD1 expression (n = 3 for MDA-MB-231, n = 6 for SkBR3, n = 3 for MDA-MB-436). **(29M-O)** PSAT1 expression (n = 5-6 for MDA-MB-231, n = 6 for SkBR3, n = 3 for MDA-MB-436). **(30A-C)** PCK2 expression (n = 3 all). **(30D-F)** G6PD expression (n = 5-6 for MDA-MB-231, n = 2 for SkBR3, n = 3 for MDA-MB-436). **(30G-I)** ASNS expression (n = 3 all). **(30J-L)** SLC6A9 expression (n = 3 all). **(30M-O)** NUPR1 expression (n = 3 all). * P < 0.05, ** P < 0.01, *** P < 0.005 compared to control; by Student t test.

### EXAMPLES

The present disclosure and invention are further illustrated by the following examples.

### Example 1

### 1. Materials and Methods

### 1.1. Chemicals

Mitoquinol methanesulfonate (mitoQ) was dissolved in DMSO at a stock concentration of 10 mM. doxorubicin (2 mg/mL), epirubicin (2 mg/mL), 5-fluorouracil (5-FU; 50 mg/mL), cisplatin (1mg/mL), gemcitabine (38 mg/mL), paclitaxel (1 mg/mL) and cyclophosphamide (20 mg/ml) were kindly provided by the Central Pharmacy of the Cliniques Universitaires Saint-Luc, Brussels, Belgium. Unless stated otherwise, all other chemicals were from Sigma-Aldrich^{®}. Equal volumes of solvent (DMSO) were used in control experiments.

### 1.2. Cells and cell culture

Human triple-negative MDA-MB-231 breast adenocarcinoma cancer cells were from Caliper^{®} (cat# 119369). Human HER2+ SkBr3 breast adenocarcinoma cancer cells (ATCC^{®} HTB-30^{™}), human triple-negative MDA-MB-436 breast adenocarcinoma cancer cells (ATCC^{®} HTB-130^{™}) and human MCF10A nonmalignant breast epithelial cells were from ATCC (ATCC^{®} CRL-10317^{™}). All cancer cell lines were originally derived from pleural effusions. MDA-MB-231 and SkBr3 cells were routinely cultured in DMEM containing 4.5 g/L glucose and GlutaMax^{™} (Gibco^{®}, cat# 10566016) with 10% FBS; MDA-MB-436 in IMDM containing GlutaMax^{™} (Gibco^{®}, cat# 31980030) with 20% FBS; and MCF10A in DMEM:F-12 (Gibco^{®}, cat# 11320033) with 5% horse serum, 1 mM CaCl₂, 10 mM HEPES, 10 µg/mL insulin, 20 ng/mL epithelial growth factor (EGF) and 0.5 µg/mL hydrocortisone. All cell cultures were routinely maintained at a sub-confluent state in a humidified atmosphere (air) with 5% CO₂, 37°C. Cell authenticities were routinely verified with a short tandem repeat (STR) test (Eurofins Genomics).

### 1.3. Metabolic assays

Oxygen consumption rates (OCRs) were determined on a Seahorse XF96 bioenergetic analyzer using the XF cell Mito stress kit (Agilent Technologies^{®}), according to manufacturer's recommendations. Briefly, MDA-MB-231 (10⁴ cells/well), SkBr3 (10⁴ cells/well), MDA-MB-436 (10⁴ cells/well) or MCF10A (10⁵ cells/well) were plated on XF96 culture plates 16 h before experiments in DMEM containing 10% FBS, and treated ± MitoQ for 48 h. On the day of analysis, culture media were replaced by DMEM containing 10 mM glucose, 2 mM glutamine, 1.85 g/L NaCl, 3 mg/L phenol red, pH 7.4. Cells were incubated for 1 h in a CO₂-free incubator before analysis. Sequentially, basal OCR was acquired without treatment; ATP-linked OCR after the addition of 1 µM of ATP synthase inhibitor oligomycin; maximal OCR after mitochondrial potential disruption using 1 µM of ionophore carbonyl cyanide-4-(trifluoromethoxy) phenylhydrazone (FCCP); and non-mitochondrial OCR after the addition of 0.5 µM of Complex I inhibitor rotenone together with 0.5 µM of Complex III inhibitor antimycin A. All data were normalized by total protein content (Bio-Rad^{®} Protein Assay; catalogue #5000006) measured right after oximetry. Mitochondrial OCRs (mtOCRs) were calculated by subtracting non-mitochondrial OCRs to the corresponding basal, maximal and ATP-linked OCRs.

Mitochondrial superoxide levels were determined using electron paramagnetic resonance (EPR) with MitoTEMPO-H as a specific mitochondrial superoxide probe ± PEG-SOD2 to specifically assign signals to mitochondrial superoxide. EPR measurements were performed using a Bruker EMX-Plus spectrometer, operating in X-band (9.85 GHz) and equipped with a PremiumX ultra low noise microwave bridge and a SHQ high sensitivity resonator. Typical settings were: microwave power: 20 mW; attenuation: 10 dB; modulation frequency: 100 kHz; modulation amplitude: 0.1 mT; time constant: 20.48 ms; conversion time: 22.58 ms; sweep width: 0.15 mT. Briefly, MDA-MB-231, SkBr3, MCF10A or MDA-MB-436 cells were treated ± MitoQ for 48 h, harvested and resuspended in PBS at 2 × 10⁶ cells/mL. The experimental mixture was prepared with cell suspension, 1 mM DTPA and 150 µM MitoTEMPO-H. It was then aspirated into a gas-permeable polytetrafluoroethylene (PTFE) tubing that was placed in a quartz tube opened at both ends. The tube was directly inserted inside the EPR cavity that was heated at 310°K with air during all experiments. EPR measurements were performed 3 min after incorporation of the probe within the cell mixture, and acquisitions were continued until 15 min. To specifically measure superoxide contribution to the EPR signal, each experiment was repeated replacing 2.5 µL PBS by 2.5 µL of PEG-SOD2 (2,000 U/mL). Computer simulations were performed using the Bruker Xenon Spin fit program. All data were normalized by cell numbers (trypan blue assay). Net superoxide production was calculated by subtracting the double integration values of the CMH spectrum with PEG-SOD2 to the control value at timepoint 15 min.

Glucose and lactate concentrations were measured in cell supernatants collected after 48 h of culture ± MitoQ, using specific enzymatic assays on a CMA600 analyzer (Aurora Borealis^{®}), as previously described in Sonveaux et al. (J Clin Invest; 2008; 118, 3930-3942). All data were normalized by total protein content (Bio-Rad^{®} Protein Assay).

Intracellular ATP levels were measured after 48 h of treatment ± MitoQ, using the CellTiter-Glo Luminescent Viability Assay (Promega^{®}) on a Glomax 96 microplate luminometer (Promega^{®}) following manufacturer's instructions. Results were normalized to cell number.

### 1.4. Mitochondrial potential

The mitochondrial potential (ΔΨ) was measured using JC-10 Mitochondrial Membrane Potential Assay Kit (Abcam^{®}, #ab112134), according to manufacturer's recommendations. Briefly, MDA-MB-231 (10⁴ cells/well), SkBr3 (10⁴ cells/well), MDA-MB-436 (10⁴ cells/well) or MCF10A (10⁵ cells/well) cells were seeded in 96-well plates and treated for 48 h ± MitoQ. Cells were then washed twice and incubated with JC-10 (1× solution) for 45 min. Fluorescence intensities were measured at 490/525 nm and 540/525 nm of absorbance using a SpectraMax i3 spectrophotometer equipped with a MiniMax imaging cytometer (Molecular Devices^{®}).

### 1.5. Western blotting

For western blotting (WB), whole-protein extracts from MDA-MB-231, SkBr3 and MDA-MB-436 cells were prepared using RIPA buffer (50 mM Tris pH 7.4, 150 mM NaCl, 1% Triton-X-100, 0.05% sodium deoxycholate, 1 mM EDTA, 0.1% SDS, protease inhibitor cocktail and PhosSTOP Phosphatase Inhibitor Cocktail (Roche^{®})), centrifuged at 10,000×g for 10 min, and quantified using the Bio-Rad^{®} Protein Assay. Proteins (50 µg for MDA-MB-231 and 100 µg for SkBr3 and MDA-MB-436 cells) were loaded into each lane of a 10-12% polyacrylamide gel in the presence of SDS, and were allowed to separate at 120 V for 90 min. The transfer of proteins onto nitrocellulose membranes was performed using an iBlot 2 Dry Blotting System (Thermo Fisher Scientific^{®}) with the 7 min P0 program. Membranes were then blocked using 5% (w/v) powdered milk for 1 h, and incubated overnight at 4°C with a primary antibody **(Table 4).**

**Table 4: Antibodies used for western blotting**

| **Protein name** | **Antibody** | **Company and Cat#** | **Dilution** |
|---|---|---|---|
| P-T172-AMPK | rabbit anti-phospho-AMPKα | Cell signaling^{®}, #2531 | 1:1,000 |
| AMPK | rabbit anti-AMPKα | Cell signaling^{®}, #2532 | 1:1,000 |
| VIMENTIN | rabbit anti-vimentin | Cell signaling^{®}, #5741 | 1:1,000 |
| SNAIL | rabbit anti-snail | Cell signaling^{®}, #3879 | 1:1,000 |
| SLUG | rabbit anti-slug | Cell signaling^{®}, #9585 | 1:1,000 |
| ZEB1 | rabbit anti-zeb1 | Cell signaling^{®}, #3396 | 1:1,000 |
| TWIST1 | rabbit anti-twist1 | Cell signaling^{®}, #46702 | 1:1,000 |
| E-cadherin | rabbit anti-E-cadherin | Cell signaling^{®}, #3194 | 1:1,000 |
| β-actin | mouse anti-β-actin | Sigma-Aldrich^{®}, #A5441 | 1:1,000 |

β-actin served as a loading control. Immunodetection was performed at room temperature for 1 h using as secondary antibodies a horseradish peroxidase-conjugated goat anti-rabbit (Jackson^{®}, Cat# 111-035-003) and goat anti-mouse (Jackson^{®}, Cat# 115-035-003) in PBS-Tween containing 5% (w/v) milk or BSA. Detection was performed with ECL reagent (Amersham^{®}, Cat# RPN2209), and protein bands were visualized and captured using ECL imager 600 (Amersham^{®}). They were analyzed using the Image J software (Java).

### 1.6. Cell numbers

To probe the effects of MitoQ alone on cell numbers, MDA-MB-231, SkBr3, MDA-MB-436 or MCF10A (2,500-10,000 cells/well) cells were plated in 96-well plates and treated with increasing concentrations of MitoQ. At each time point, the number of cells per well was measured using a SpectraMax i3 spectrophotometer equipped with a MiniMax^{™} imaging cytometer.

To test the effects of combination treatments on cell numbers, MDA-MB-231 or SkBr3 cancer cells were plated in 96-well plates and treated with increasing concentrations of chemotherapeutic drug ± MitoQ for 24 h (epirubicin), 48 h (doxorubicin, 5-FU, cisplatin, paclitaxel) or 72 h (gemcitabine). Cells were then fixed in 11% glutaraldehyde and stained with crystal violet. The dye retained by the cells was solubilized in 10% acetic acid, and the optical density (570 nm) was measured using a SpectraMax i3 spectrophotometer equipped with a MiniMax^{™} imaging cytometer.

### 1.7. Cell cycle

Cells were seeded in 6-well plates at a density of 500,000 cells/well and allowed to adhere overnight. Cells were then starved in 0.1% FBS medium for 24 h and treated for 48 h ± MitoQ. Afterwards, they were detached with trypsin-EDTA (0.05 %) (Gibco^{®}, cat# 25300054) and centrifuged at 1,200 rpm for 5 min. Culture medium was discarded, and the pellets washed. Cells were fixed by adding 700 µL of ice-cold ethanol 100% in 300 µL of cell suspension in PBS. Cells were then washed twice with 1 mL of Tris buffer with 0.2% (v/v) Triton X-100, and finally resuspended in 300 µL of PBS with RNase (0.2 mg/mL) and propidium iodide (5 µg/mL). At least 10⁴ events were acquired using a FACSCalibur^{™} flow cytometer (Becton Dickinson^{®}), and the FlowJo cell cycle analysis tool was used to analyze data according to DNA content.

### 1.8. Electron microscopy

Electron microscopy images of MDA-MB-231 and SkBr3 cells treated ± MitoQ for 48 h were acquired on a TECNAI G² 20 LaB6 transmission microscope using a previously disclosed protocol (Piret et al.; Nanotoxicology, 2012; 6, 789-803).

### 1.9. Immunocytochemistry

To visualize cell morphology, MDA-MB-231, SkBr3 and MDA-MB-436 cells were seeded in 24-well plates and treated ± MitoQ for 48 h, fixed in 100% methanol for 10 min stained with hematoxylin and eosin (H&E) for 10 min each, washed with water and dried. Pictures were acquired on an Axiovert 40 CFL microscope (Zeiss^{®}) equipped with a MRC camera (Zeiss^{®}).

### 1.10. Real-time quantitative PCR

Total mRNA from MDA-MB-231, SkBr3 and MDA-MB-436 cells treated ± MitoQ for 48 h was extracted using the NucleoSpin RNA Kit (Macherey-Nagel^{®}). Total mRNA from primary tumors was extracted with Tri reagent (Molecular Research Center^{®}, Cat# TR118). mRNAs were quantified by the Qubit BR dsRNA assay kit (Thermo Fisher Scientific^{®}), and mRNA integrity was evaluated on an Agilent^{®} 2100 Bioanalyzer with the RNA 6000 nano kit (Agilent^{®}). It was reverse transcribed in complementary DNA using the High-Capacity cDNA Reverse Transcription Kit (Applied Biosystems^{®}, cat#4368814) according to manufacturer's protocol. Complementary DNA (500 ng) was amplified by real time quantitative PCR (RT-qPCR) using the low ROX SYBR Master Mix dTTP Blue (Eurogentec^{®}) and primers listed in **Table 5** and **Table 6** on a ViiA 7 Real-Time PCR System (Thermo Fisher Scientific^{®}). Gene expression was normalized to β-actin gene expression.

**Table 5: Forward primers used in qPCR**

| **Gene name** | **Forward 5' - 3'** | **SEQUENCE ID NO:** |
|---|---|---|
| **Human genes** | | |
| *VIM* | CGGGAGAAATTGCAGGAGGA | **1** |
| *SNAI1* | AATCCAGAGTTTACCTTCCAGCA | **2** |
| *SNAI2* | GAACTGGACACACATACAGTGAT | **3** |
| *ZEB1* | GCCCAAACTGCAAGAAACGC | **4** |
| *TWIST1* | TTCTCGGTCTGGAGGATGGA | **5** |
| *c-MYC* | ATGAAAAGGCCCCCAAGGTA | **6** |
| *OCT4* | CATCAAAGCTCTGCAGAAAGAACT | **7** |
| *NANOG* | AATACCTCAGCCTCCAGCAGATG | **8** |
| *SOX2* | CGAGTGGAAACTTTTGTCGGA | **9** |
| *EID1* | GCGGGTTTCAGATGCATTAT | **10** |
| *PEG10* | CAGGCCTGAAAAGAAAGTGC | **11** |
| *PHGDH-1* | CACGACAGGCTTGCT GAATGA | **12** |
| *SLC7A11* | GCGTGGGCATGTCTCTGAC | **13** |
| *SERPINE1* | CACAAATCAGACGGCAGCACT | **14** |
| *FTH1* | AAGCTGCAGAACCAACGAGG | **15** |
| *TXNRD1* | CCACTGGTGAAAGACCACGTT | **16** |
| *PSAT1* | GGCCAGTTCAGTGCTGTCC | **17** |
| *PCK2* | TGCCAGGCTGGAAAGTGGAGTGT | **18** |
| *G6PD* | AACAGAGTGAGCCCTTCTTAA | **19** |
| *ASNS* | CACTCCGCGACTCCCTTTT | **20** |
| *SLC6A9* | GAGGATCAGCCCCATGTTCA | **21** |
| *NUPR1* | AGGACTTATTCCCGCTGACTGA | **22** |
| *β-ACTIN* | CCCGCGAGCACAGAGC | **23** |

| **Mouse genes** | | |
|---|---|---|
| *PHGDH-1* | ATGGCCTTCGCAAATCTGC | **24** |
| *SLC7A11* | ATGCAGTGGCAGTGACCTTT | **25** |
| *SERPINE1* | TCATCAATGACTGGGTGGAA | **26** |
| *PSAT1* | ACGCCAAAGGAGACGAAGCT | **27** |
| *TXNRD1* | GTGGCGACTTGGCTAATC | **28** |
| *GAPDH* | ACCCCAGCAAGGACACTGAGCAAG | **29** |

**Table 6: Reverse primers used in qPCR**

| **Gene name** | **Reverse 5' - 3'** | **SEQ ID NO:** |
|---|---|---|
| **Human genes** | | |
| *VIM* | AAGGTCAAGACGTGCCAGAG | **30** |
| *SNAI1* | TCCCAGATGAGCATTGGCAG | **31** |
| *SNAI2* | ACAGTGATGGGGCTGTATGC | **32** |
| *ZEB1* | GTCGCCCATTCACAGGTATCA | **33** |
| *TWIST1* | TCTCTGGAAACAATGACATCTAGG | **34** |
| *c-MYC* | TTTCCGCAACAAGTCCTCTTC | **35** |
| *OCT4* | CTGAATACCTTCCCAAATAGAACCC | **36** |
| *NANOG* | TGCGTCACACCATTGCTATTCTTC | **37** |
| *SOX2* | TGTGCAGCGGCTCGCAG | **38** |
| *EID1* | AGTTGGGTCCCTCCTCAAGT | **39** |
| *PEG10* | AATGCTTTGTGGAAGCCATC | **40** |
| *PHGDH-1* | CTTCCGTAAACACGTCC AGTG | **41** |
| *SLC7A11* | GCTGGTAATGGACCAAAGACTTC | **42** |
| *SERPINE1* | CATCGGGCGTGGTGAACTC | **43** |
| *FTH1* | AGTCACACAAATGGGGGTCATT | **44** |
| *TXNRD1* | AGGAGAAAAGATCATCACTGCTGAT | **45** |
| *PSAT1* | GCTCCTGTCACCACATAGTCA | **46** |
| *PCK2* | GCAACCCCAAAGAAGCCGTTCTCA | **47** |
| *G6PD* | GGAGGCTGCATCATCGTACT | **48** |
| *ASNS* | ACCATTTCCACGGATGCAA | **49** |
| *SLC6A9* | AAGGCGATGCAGATGACCAC | **50** |
| *NUPR1* | TGCCGTGCGTGTCTATTTATTG | **51** |
| *β-ACTIN* | TCATCATCCATGGTGAGCTGG | **52** |

| ***Mouse* genes** | | |
|---|---|---|
| *PHGDH-1* | AGTTCAGCTATCAGCTCCTCC | **53** |
| *SLC7A11* | GGCAACAAAGATCGGAACTG | **54** |
| *SERPINE1* | TGCTGGCCTCTAAGAAAGGA | **55** |
| *PSAT1* | ATGTTGAGTTCTACCGCCTTGTC | **56** |
| *TXNRD1* | ACCAGGAGAGACACTCAC | **57** |
| *GAPDH* | TGGGGGTCTGGGATGGAAATTGTG | **58** |

### 1.11. Cell migration

A scratch test was performed on cells treated ± MitoQ for 48 h (Tamura et al.; Science, 1998; 280, 1614-1617). For each condition and each time point, pictures were taken in the same field, and the distance between the wound edges was analyzed using the Image J software (Java^{®}). The percentage of scratch closure was calculated in respect to wounded area at 0 h.

### 1.12. Cell invasion

Cell chemotaxis was assayed in a 48-well micro-chemotaxis chamber (Neuroprobe^{®} AP48) with polycarbonate porous membrane (8-µm diameter, Neuroprobe^{®} PFB8) coated with 5 µg/mL of fibronectin, according to manufacturer's instructions. Briefly, MDA-MB-231 (20,000 cells /well), SkBr3 (40,000 cells/well) or MDA-MB-436 (80,000 cells/well) cells were seeded in the upper compartment in culture media deprived of FBS, and 0.2% (v/v) FBS-containing medium (for MDA-MB-231 and MDA-MB-436 cells) or 10% (v/v) FBS-containing medium supplemented with 10 ng/mL of human EGF (PrepoTech^{®}, cat#AF-100-15) (for SkBr3 cells) were used as chemo-attractants. Cells were allowed to invade through the membrane overnight. Membranes were then washed with PBS, fixed in methanol, and stained with crystal violet (0.23% v/v). Pictures were acquired under an Axiovert^{™} 40 CFL microscope (Zeiss^{®}) equipped with a MRC camera (Zeiss^{®}). Quantification was performed using the Image J software.

### 1.13. Clonogenic assays

For adherent assays, MDA-MB-231 and SKBR3 cells were pre-treated for 48 h ± MitoQ, and seeded (1,000 to 2,000 cells/well) in 6-well plates. After colony formation (2 weeks), cells were fixed and stained with 0.5% crystal violet in a 10% ethanol solution, for 30 min. Colonies were washed with water and counted. Results are expressed as surviving fraction (SF), where SF = #colonies/plating efficiency (PE).

For soft agar colony formation assays, 0.4% Seaplaque soft agar (Lonza^{®}) was diluted with DMEM containing 10% FBS and was covered by a second 0.3% soft agar layer in which 500 MDA-MB-231 or SKBR3 cells were embedded. Complete DMEM Culture media ± MitoQ was added with 2× the final concentration. After 20 days, colonies were counted using an Axiovert^{™} 40 CFL microscope equipped with a MRC camera.

### 1.14. Spheres

3D models were used to obtain an enriched population in stem cells. For that, 10⁴ MDA-MB-231, SkBr3 or MDA-MB-436 cancer cells were grown in suspension using 10 cm dishes coated with Polyhema in stem-cell medium: DMEM/F-12 (Gibco^{®}, cat# 11320033) supplemented with 100 units/mL penicillin, 100 µg/mL streptomycin, 20 µL/mL B27 (Gibco^{®}, cat# 17504044), 20 ng/mL human recombinant epithelial growth factor (EGF) (Peprotech^{®}, cat# AF-100-15), 10 ng/mL basic fibroblast growth factor (bFGF; Peprotech^{®}, cat# 100-18B) and 2.5 mg/mL insulin. When spheres reached around 100 µm in diameter, they were washed with PBS and dissociated with accutase (Stem Cell Technology^{®}, cat# 07920) in order to have single cells again. At the third passage, dissociated spheres were treated ± MitoQ for 96 h. Images were captured on Axiovert^{™} 40 CFL microscope equipped with a MRC camera. At the end of the treatment, living cells were collected for RNA extraction.

Stem like-spheroids were prepared by seeding 5,000 MDA-MB-231, SkBr3 or MDA-MB-436 dissociated spheres/well in ultra-Low attachment 96-well plates (Corning^{®}, cat# 7007) in stem cell medium supplemented as described above. After overnight formation, spheroids were treated ± MitoQ for 96 h. Spheroid growth was monitored using an Axio Observer live-cell phase contrast microscope (Zeiss^{®}).

### 1.15. RNA sequencing

MDA-MB-231 and SkBr3 cancer cells were treated ± MitoQ for 48 h. Total mRNA was then extracted using the NucleoSpin RNA kit (Macherey-Nagel^{®}), including DNAse treatment in the intermediary steps. mRNAs were quantified using the Qubit BR dsRNA assay kit (Thermo Fisher Scientific^{®}), and mRNA integrity was evaluated on the Agilent 2100 Bioanalyzer using the RNA 6000 nano kit (Agilent^{®}). All samples had RNA integrity number values ≥ 7.5. Libraries from control and treated samples were prepared starting from 150 ng of total mRNA using the KAPA RNA HyperPrep Kit with RiboErase (HMR) kit (KAPA Biosystems^{®}) following the manufacturer's recommendations (KR1351 - v1.16). Libraries were equimolarly pooled and sequenced on a single lane of an Illumina^{®} NovaSeq 6000 platform. All libraries were sequenced at a depth of > 50 million paired-end reads (2×100 bp reads) per sample. All sequencing data were analyzed using the Automated Reproducible MOdular workflow for preprocessing and differential analysis of RNAseq data (ARMOR) pipeline. In this pipeline, reads underwent a quality check using FastQC. Quantification and quality control results were summarized in a MultiQC report before being mapped using Salmon to the transcriptome index that was built using all Ensembl cDNA sequences obtained in the Homo_sapiens.GRCh38.cdna.all.fa file. Then, estimated transcript abundances from Salmon were imported into R using the tximeta package, and analyzed for differential gene expression with edgeR. To get a robust signature, only genes with |log 2 Fold Change| > 1 and P < 0.01 were retained.

### 1.16. In Vivo Experiments

All *in vivo* experiments were performed with the approval of UCLouvain Comité d'Ethique pour l'Expérimentation Animale (approval IDs: 2016/UCL/MD/018 and 2020/UCL/MD/033) according to national and European animal care regulations.

For metastasis take assays, cancer cells were pretreated ± MitoQ for 6 h, and 10⁶ viable cells were injected into the tail vein of five weeks-old female Rj:NMRI-Foxn1nu/nu mice (Janvier). After 4 weeks, mice were sacrificed and the lungs insufflated with 3 mL of Indian ink (15% in ddH₂O). Mouse lungs were removed, washed with PBS and incubated overnight in Fekete's solution (700 mL/L 100% ethanol, 32 mL/L 37% formalin, 40 mL/L glacial acetic acid, diluted in ddH₂O). The next morning, lungs were transferred to a 70% ethanol solution and were further examined under a Stemi 2000-C dissection stereomicroscope (Zeiss^{®}) to count the number of metastasis per lung.

For spontaneous metastasis assays using human breast cancer cells, MDA-MB-231, SkBr3 or MDA-MB-436 cells were prepared in medium containing 10% growth factor-reduced Matrigel (Corning^{®}, cat# 734-11-01). Five weeks-old female Rj:NMRI-Foxn1nu/nu mice (Janvier) were injected with 10⁶ cancer cells in 100 µL of solution in the left second mammary fat pad. After tumor take (72 h), mice were daily treated ± 20 mg/Kg MitoQ by oral gavage. Tumor size was measured once per week with an electronic caliper. Four weeks after treatment initiation, primary tumors were surgically removed and processed for RT-qPCR. Tumor recurrence was monitored until recurred tumors in the vehicle group reached about 200 cm³, *i.e.* 24 days after surgery. At this time point, mice were sacrificed and their lungs collected and fixed in PFA 4% for immunocytochemistry.

Female FVB/N-Tg(MMTV-PyMT)634Mul/J (MMTV-PyMT) mice that express the polyomavirus middle T antigen spontaneously develop orthotropic multifocal breast adenocarcinomas that spontaneously disseminate metastases to the lungs during mouse lifetime. For spontaneous metastasis assays, female MMTV-PyMT mice (The Jackson Laboratory) were daily observed for signs of spontaneous primary tumors starting at 6 weeks of age. Once tumors became palpable, 4 additional weeks were waited to mimic a clinical time frame between first tumor signs and treatment initiation. Ten weeks-old mice with primary tumors of ~ 0.8 cm in diameter received a single dose of FEC chemotherapy (100 mg/Kg 5-FU, 5 mg/Kg epirubicin, 100 mg/Kg cyclophosphamide), a clinically relevant neoadjuvant or adjuvant chemotherapeutic combination. Animals were then randomly assigned to a group receiving daily 18 mg/Kg MitoQ or an equal volume of vehicle by oral gavage. They were sacrificed at 16 weeks-old, and the lungs insufflated with Indian ink for metastasis quantification as described above. Primary tumors were collected, weighted, and processed for RT-qPCR.

### 1.17. Immunohistochemistry

Collected lungs were processed for immunohistochemistry as previously shown. They were stained for Cytokeratin 19 (CK19) using a primary rabbit recombinant anti-CK19 antibody (Abcam^{®}, cat# ab76539) and a secondary Envision anti-rabbit antibody coupled to HRP (Dako^{®}, cat# K4003), and counter-stained with H&E. Images of whole lung slides were acquired on a SCN400 Slide Scanner (Leica^{®}) and analyzed with the QuPath Software (0.1.2). Quantification was performed according to Chang and Erler (Adv Exp Med Biol, 2016; 899, 245-251), and the number of lung metastases was proportional to the positive areas of CK19 staining.

### 1.18. Statistics

All results are expressed as means ± standard error of the mean (SEM) for n independent observations. Error bars are sometimes smaller than symbols. Outliers were identified using Dixon's Q test. Data were analyzed using GraphPad Prism 8.4.3. Student's t test, Mann Whitney test, one-way ANOVA with Tukey's post-hoc test, and two-way ANOVA were used where appropriate. A Log-Rank (Mantel-Cox) test was used to analyze the Kaplan-Meier graph. P < 0.05 was considered to be statistically significant.

### 2. Results

### 2.1. MitoQ represses pro-metastatic mitochondrial ROS signaling in human breast cancer cells

To test whether MitoQ can prevent metastasis in breast cancer, human triple-negative MDA-MB-231 breast adenocarcinoma cancer cells and human HER2+ SkBr3 breast adenocarcinoma cancer cells were selected as main models. Both cell lines originally derived from pleural effusion, attesting their metastatic capabilities.

In MDA-MB-231 cells, metabolic characterization showed that MitoQ at low concentration (100 nM, 48h) decreased basal, maximal and ATP-linked oxygen consumption rates (OCRs) **(****Figure 1A-C****),** as well as the mitochondrial potential (Δψ) **(****Figure 1D****).** This was associated with lower mitochondrial superoxide and mitochondrial H₂O₂ production measured using electron paramagnetic resonance (EPR) **(****Figure 1E****).** Similar effects were observed when repeating these assays with SkBr3 cancer cells **(****Figure 2A-E****).** Comparatively, even if MitoQ reduced the OCRs of human MCF10A normal epithelial breast cells **(****Figure 3A-C****),** Δψ was not affected **(****Figure 3D****),** nor the mitochondrial superoxide production **(****Figure 3E****),** revealing selective effects of MitoQ on cancer cells.

Despite 5' AMP-activated protein kinase (AMPK) activation **(****Figure 4A****)** and a metabolic compensation through increasing their glycolytic rate **(****Figure 4B-C****),** MDA-MB-231 cells treated with MitoQ (100 nM, 48h) experienced an overall decrease in ATP production **(****Figure 4D****).** At this dose, the cell cycle was not altered **(****Figure 4E****),** but MitoQ eventually reduced MDA-MB-231 cell number starting 500 nM **(****Figure 4F****).** MitoQ did not significantly activate AMPK in SkBr3 cells **(****Figure 5A****).** However, similar effects on OCRs, ATP production and cell number were observed in SkBr3 cells **(****Figure 5B-F****),** except that SkBr3 cell numbers started to decrease from a dose of 250 nM of MitoQ. Dose-dependent decreases in cell numbers matched with dose-dependent decreases in Δψ **(****Figure 6A-B****),** while glycolytic compensation saturated **(****Figure 7A-B****)** in both cancer cell lines.

### 2.2. MitoQ induces a mesenchymal to epithelial transition (MET) and repressed the in vitro migration and invasion of human breast cancer cells

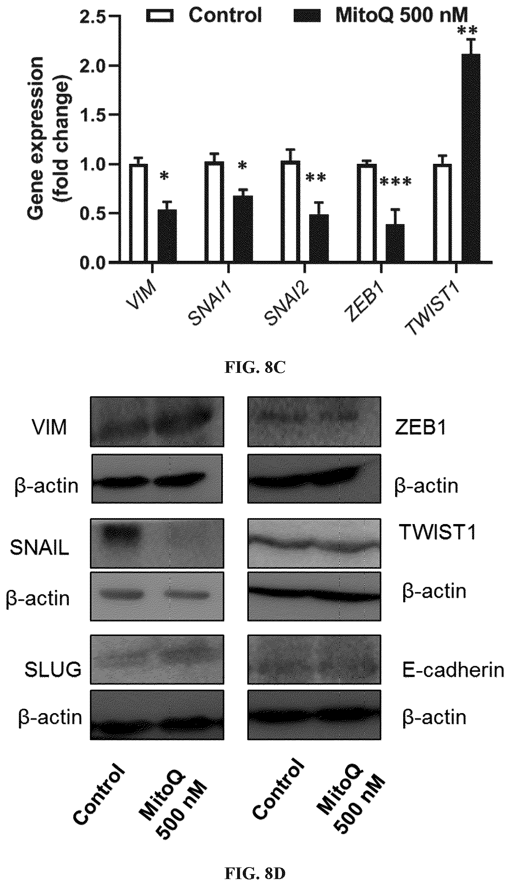

Although as adenocarcinomas both MDA-MB-231 and SkBr3 are of epithelial origin, they have a mesenchymal phenotype, as expected from metastatic breast cancer cells isolated from pleural effusions in patients. It was therefore tested whether MitoQ could reverse the epithelial to mesenchymal transition (EMT) that was acquired in patients. Morphological changes induced by MitoQ from 100 to 500 nM for 48h were not obvious in either MDA-MB-231 or SkBr3 cancer cells. However, MitoQ (500 nM, 48 h) decreased the mRNA expression of EMT markers vimentin (VIM), SNAIL (SNAI1), ZEB1 and TWIST1 in MDA-MB-231 cells **(****Figure 8A****),** whereas SLUG/SNAI2 transcription was not significantly altered. At the same time point, SNAIL, ZEB1, Twist1 and E-cadherin protein expression was decreased **(****Figure 8B****).** In SkBr3 cells, MitoQ (500 nM, 48 h) repressed the mRNA expression of all analyzed EMT inducers **(****Figure 8C****).** At the same time point, SNAIL protein expression was decreased **(****Figure 8D****).** Based on these data, it was concluded that MitoQ has the capacity to induce partial MET in mesenchymal human breast cancer cells having undergone EMT in patients.

After EMT, metastatic cancer cells must acquire migratory and invasive capabilities to metastasize. MDA-MB-231 and SkBr3 cells possess these capabilities, which were highly significantly reduced when the cells were treated with MitoQ (100 nM, 48 h) **(****Figure 9A-D****).**

### 2.3. MitoQ represses breast cancer clonogenicity and stemness

To establish a secondary tumor in a distant organ, mesenchymal, migratory and invasive cancer cells must in addition possess stem cell characteristics. Assays in Petri dishes allowing adhesion showed that MDA-MB-231 and SkBr3 cells were equally clonogenic, which was largely inhibited upon treatment with MitoQ (100 nM, 48 h) **(****Figure 10A-B****). In** suspension on soft agar, MDA-MB-231 were more clonogenic than SkBr3 cells, rendering them less sensitive to MitoQ **(****Figure 10C****).** Yet, MitoQ at a 250 nM concentration completely abrogated the clonogenicity of both cell lines. This dose also inhibited MDA-MB-231 spheroid formation, which was associated to a significant decrease in the mRNA expression of stem cells markers POU5F1/(Oct4), NANOG and SOX2 in MDA-MB-231 spheroids **(****Figure 11A****).** Furthermore, a dose of 100 nM of MitoQ was sufficient to destabilize already formed MDA-MB-231 spheroids. Neither inhibition of spheroid formation not spheroid destabilization was seen using SkBr3 cells, even if NANOG and SOX2 mRNA expression was decreased in SkBr3 spheroids **(****Figure 11B****).** It was therefore concluded that MitoQ has the capacity to inhibit, at least partially, all major metastatic traits (*i.e.,* EMT, migration, invasion, clonogenicity and stemness) in breast cancer cells *in vitro.*

### 2.4. Identification of markers of the response of breast cancer cells to MitoQ

While EMT and stemness markers are usual suspects when testing antimetastatic strategies, it was decided to go deeper in the understanding of the molecular effects of MitoQ, and performed unbiased RNA sequencing (RNAseq) using cells treated for 48 h ± MitoQ (500 nM). Principal component analysis showed a strong clustering of gene transcripts between treated and untreated MDA-MB-231 and between treated and untreated SkBr3 cells. With thresholds set at | log 2 fold change | > 1 and P < 0.01 in order to retain only highly significantly modulated transcripts, 1,943 and 5 downregulated transcripts in MDA-MB-231 and SkBr3 cells were identified, respectively, of which 2 were commonly repressed; and 2,153 and 20 upregulated transcripts in MDA-MB-231 and SkBr3 cells were identified, respectively, of which 11 were commonly induced. As shown in **Figure 12A-M****,** downregulated gene transcripts were EID1 and PEG10, and upregulated gene transcripts were PHGDH-1, SLC7A11, SERPINE1, FTH1, TXNRD1, PSAT1, PCK2, G6PD, ASNS, SLC6A9 and NUPR1. Downregulated transcripts encoded proteins normally required for cellular differentiation, whereas most upregulated transcripts encoded metabolic enzymes and transporters **(Table 7).** On independent samples upon the same conditions (including | log 2 Fold Change | > 1 and P < 0.01), RT-qPCR on the two cell lines validated all genes as potential biomarkers of human breast cancer cell response to MitoQ, to the exception of EID1, PEG10, FTH1 and NUPR1 **(****Figure 12A-M** and **Table 7).**

**Table 7: mRNA biomarkers of the response of human breast cancer cells to MitoQ**

| **Gene** | **Geneban k ID** | **Protein** | **Validate d *in vitro¹*** | **Validate d *in vivo²*** | **Reference** |
|---|---|---|---|---|---|
| **Transcripts downregulated in response to MitoQ** | | | | | |
| *EID1* | 23741 | EP300-interacting inhibitor of differentiation 1 | - | - | Miyake *et al., 2000* |
| *PEG10* | 23089 | Paternally expressed 10 | - | - | Li *et al.,* 2016; Xie *et al.,* 2018 |

| **Transcripts upregulated in response to MitoQ** | | | | | |
|---|---|---|---|---|---|
| *ASNS* | 440 | Asparagine synthetase | + | - | Xiu *et al.,* 2020 |
| *FTH1* | 2495 | Ferritin heavy chain 1 | - | - | Gray *et al.,* 2003; Halpern *et al.,* 2007 |
| *G6PD* | 2539 | Glucose-6-phosphate dehydrogenase | + | - | Yang *et al.,* 2019 |
| *NUPR1* | 26471 | Nuclear protein 1 | - | - | Chowdhur y et al., 2009 |
| *PCK2* | 5106 | Phosphoenolpyruvat e carboxykinase 2 (PEPCK) | + | - | Mendez-Lucas *et al.,* 2014; Leithner et al., 2014; Zhao et al., 2017 ; Luo *et al.,* 2017 |
| *PHGDH-1* | 26227 | Phosphoglycerate dehydrogenase-1 | + | + | Zhao et al., 2020 |
| *PSAT1* | 29968 | Phosphoserine aminotransferase 1 | + | + | Wang *et al.,* 2020 |
| *SERPINE* 1 | 5054 | Serpin Family E Member 1 | + | + | Li *et al.,* 2018 |
| *SLC6A9* | 6536 | Solute carrier family 6 member 9 | + | - | No references |
| *SLC7A11* | 23657 | xCT | + | + | Koppula et al., 2018 |
| *TXNRD1* | 7296 | Thioredoxin reductase 1 | + | + | Hayashi *et al.,* 1993; Ueno *et al.,* 1999 |

| | | | | | |
|---|---|---|---|---|---|
| ¹ Repressed or induced by ≥ 2-fold with P < 0.01 in both MDA-MB-231 and SkBr3 cells in culture. ² Repressed or induced by ≥ 2-fold with P < 0.01 and MDA-MB-231 primary tumors in mice. ALL: Acute Lymphoblastic Leukemia; TNBC: Triple Negative Breast Cancer; GSH : Glutathione; TICs: tumor-initiating cells | | | | | |

### 2.5. MitoQ inhibits human breast cancer recurrence and metastasis in mice

Altogether, the *in vitro* results indicated that MDA-MB-231 and SkBr3 cells are well equipped to induce MitoQ-sensitive metastasis, which hypothesis was tested *in vivo* in mice. Metastatic take assays used the protocol depicted in **Figure 13A****,** where cancer cells were pretreated for 6 h ± 1 µM MitoQ before tail vain injection. Four weeks after the injection of 10⁶ viable MDA-MB-231 cells, mice that had received MitoQ-treated cells developed significantly less metastasis than mice that had received vehicle-treated cells **(****Figure 13B****).** In the control group, 11.1% of mice were metastasis-free and 88.9% presented > 2 lung metastases at necropsy. In the MitoQ group, 36.4% of mice were metastasis-free, 27.2% had ≤ 2 metastasis, and 36.4% > 2 lung metastases.

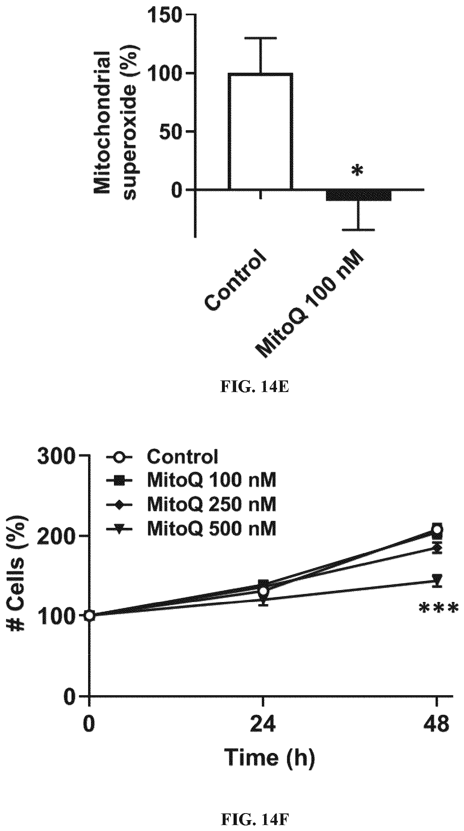

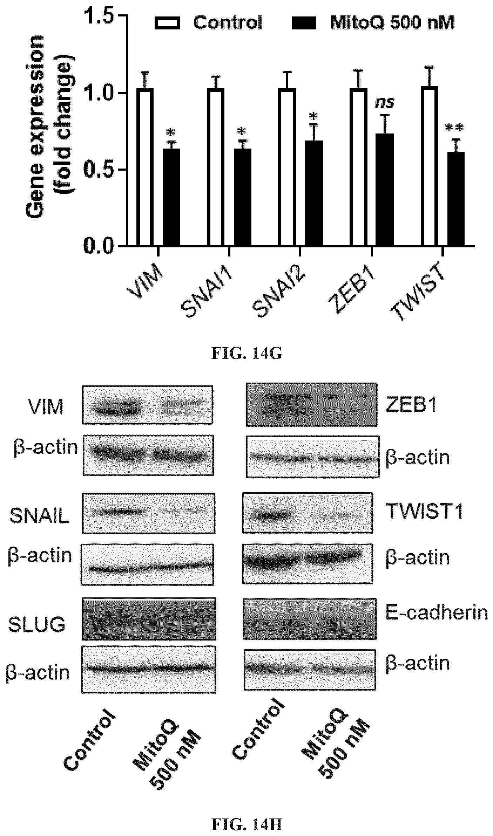

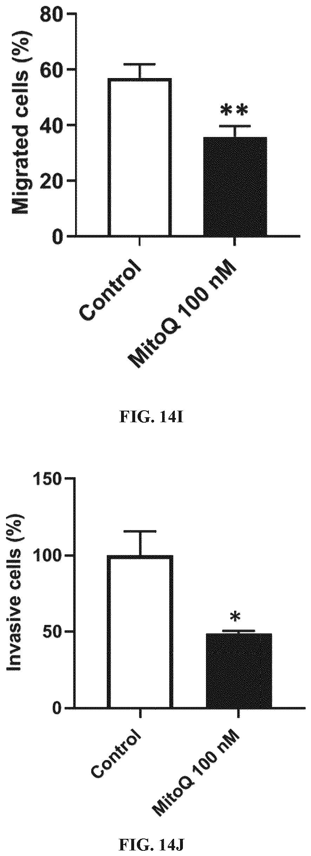

SkBr3 did not generate metastases in mice up to 6 weeks after tail vein injection, so it was envisioned to use as a third model triple-negative MDA-MB-436 breast adenocarcinoma cancer cells that were initially retrieved from pleural effusion. MDA-MB-436 responded similarly than MDA-MB-231 and SkBr3 to MitoQ *in vitro.* Accordingly, MitoQ (100 nM, 48 h), repressed basal, maximal and ATP-linked OCRs **(****Figure 14A-C****)** as well as Δψ **(****Figure 14D****)** in MDA-MB-436 cells. It dose-dependently reduced cell numbers **(****Figure 14E****).** MitoQ (100 nM, 48 h) further downregulated the mRNA expression of EMT markers VIM (vimentin), SNAI1, SNAI2 and TWIST1 **(****Figure 14F****).** A higher dose of 500 nM for 48 h was necessary to reveal a decrease in the protein expression of Vimentin, SNAI1, SNAI2 (SLUG), ZEB1 and TWIST1 **(****Figure 14G****).** At the phenotypic level, MitoQ (100 nM, 48 h) inhibited MDA-MB-436 cell migration **(****Figure 14H****)** and invasion **(****Figure 14I****).** It dose-dependently retarded spheroid formation **(****Figure 14J****).** It was therefore considered that these elements were sufficient to validate the model *in vitro.* However, similar to SkBr3, MDA-MB-436 turned out to be unable to form metastases in metastatic take assays following the protocol of **Figure 13A** (not shown).

The capability of MitoQ to prevent tumor metastasis was tested in an orthotopic model of MDA-MB-231-bearing mice using the protocol depicted in **Figure 15A****.** The protocol involved a time lapse of 3 days between tumor implantation and the beginning of treatment in order not to interfere with primary tumor take, a daily oral administration of 20 mg/kg of MitoQ (or an equivalent volume of vehicle). Direct measurements revealed that primary tumor growth was not significantly affected (P = 0.3150) **(****Figure 15B-C****).**

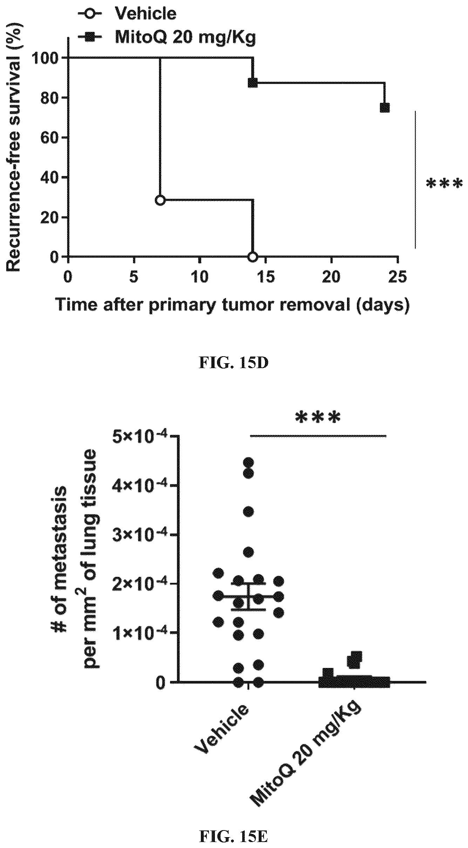

Primary tumor removal was performed on day +32 to unravel metastatic growth, as previously reported. Three mice died on the day of surgery in the control group and two in the MitoQ group. After surgery, primary tumors relapsed in 100% of the animals in the control group but only in 25% in the MitoQ group **(****Figure 15D****).** MitoQ thus significantly improved recurrence-free survival.

After mouse sacrifice on day +56, lung inspection using microscopy revealed that MitoQ had highly significantly prevented metastatic dissemination **(****Figure 15E****).** In the MitoQ group, 75.0% (2/16) of mice had no metastasis, whereas only 9.5% (2/21) of mice were metastasis-free in the control group.

Primary tumor samples were assayed to test the relevance of the biomarkers identified above. Of those, PHGDH-1, SLC7A11, SERPINE1, TXNRD1 and PSAT1 each independently qualified, with fold induction ≥ 2-fold and P < 0.01 **(****Figure 16A-M** and **Table 7).** Taken individually, PSAT1 **(****Figure 16H****)** was the most robust, as its expression was induced in 100% of tumor samples.

### 2.6. MitoQ is compatible with common breast cancer chemotherapies and prevents metastasis in spontaneous metastatic breast cancer in MMTV-PyMT mice

For the final validation of the antimetastatic activity of MitoQ in mice, the MMTV-PyMT mouse model of spontaneous breast cancer was selected, which is known to spontaneously metastasize. To set the ground for future clinical trials, standard of care + vehicle versus standard of care + MitoQ were compared. However, several chemotherapies are known to increase ROS production in cancer cells, which could participate in their anticancer activities. It was therefore important to first test whether MitoQ interfered with these treatments. To do so, cells treated *in vitro* with increasing doses of chemotherapy ± 100 nM MitoQ were counted. Treatment times were adapted to drug activities. In MDA-MB-231 and SkBr3 cells, MitoQ did not alter the cytostatic/cytotoxic effects of doxorubicin, epirubicin, 5-fluorouracil (5-FU), cisplatin, paclitaxel and gemcitabine **(****Figure 17A-L****).**

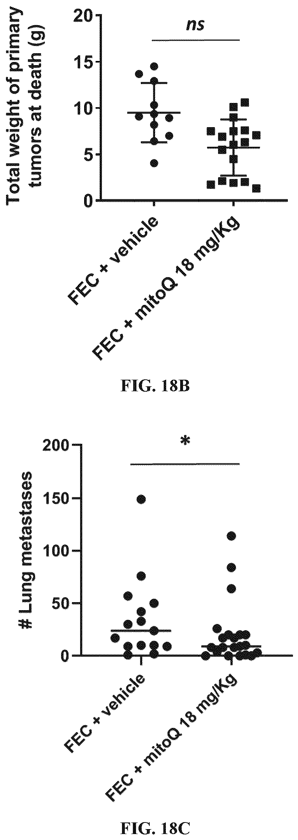

To test the advantage of combining MitoQ with chemotherapy in a spontaneous model of metastatic breast cancer, hemizygous female MMTV-PyMT mice were treated as shown in **Figure 18A****,** using FEC (5-FU 100 mg/kg, epirubicin 5 mg/kg, cyclophosphamide 100 mg/kg) as a standard of care ± a daily oral administration of MitoQ (18 mg/kg). After 6 weeks of treatment, total primary tumor weight (several tumors per animal) was not altered in animals having received FEC + MitoQ compared to FEC + vehicle (P = 0.1395) **(****Figure 18B****).** However, MitoQ significantly repressed the number of surface lung metastases **(****Figure 18C****).** In the FEC + control group, all 15 mice had metastases, whereas 4 of 21 mice were metastasis-free in the FEC + MitoQ group. Primary tumor samples were used to test the relevance of the biomarkers identified retained until here. Of those, SLC7A11, SERPINE1 and TXNRD1 each independently qualified, with fold induction ≥ 2-fold and P < 0.01 **(****Figure 19****).**

### 3. Conclusions

In this study, it was first validated that MitoQ can be used to prevent metastasis of human breast cancer cells in immunodeficient mice. It was further discovered that MitoQ can prevent human breast cancer recurrence after surgery. It was also found that MitoQ does not interfere with the cytotoxic effects of all common chemotherapies used to treat breast cancer. Using *in vitro* and *in vivo* assays, a molecular mRNA signature of the response of human breast cancer cells to MitoQ was finally validated. The signature consists in the changed expression, upon MitoQ treatment, of the following mRNAs: decreased: VIM, SNAI1, ZEB1, NANOG, SOX2; increased: PHGDH-1, SLC7A11, SERPINE1, TXNRD1, PSAT1, PCK2, G6PD, ASNS, NUPR1 and SLC6A9. Within this signature, strongest biomarkers that were also validated *in vivo* were: increased: PHGDH-1, SLC7A11, SERPINE1, TXNRD1 and PSAT1. Among these, the strongest biomarkers that were also validated in all *in vitro* and *in vivo* assays were: increased: SLC7A11, SERPINE1 and PSAT1. This signature can be used as a biomarker of the response of breast tumors to MitoQ therapy in order to evidence early those patients responding to the treatment versus those patients that do not respond. This offers an advantage for initial Phase II clinical trials, as an alternative to metastasis-free survival and recurrence-free survival that usually occur several months up to years after surgery. In later Phase II and III clinical trials, the biomarker could also be used to select patients at enrolment, thus continuing MitoQ treatments on those with a positive biomarker response and halting treatment for those without a positive biomarker response.

### Example 2

### 1. Materials and methods

### 1.1. Cells and cell culture

Human MCF7 breast cancer cells (ATCC^{®}, catalogue #HTB-22), human MDA-MB-231 breast cancer cells (Caliper^{®}, catalogue #119369) and murine 4T1 breast cancer cells (kind gift of Prof. Fred R. Miller, Karmanos Cancer Institute, Detroit, MI) were routinely grown in DMEM containing GlutaMax^{®} and 4.5 g/L glucose supplemented with 10% FBS (Gibco^{®}, #11965092) in a humidified atmosphere (air) with 5% CO₂ at 37°C. All *in vitro* assays were performed in this medium unless stated otherwise.

### 1.2. Metabolic measurements

Cellular oxygen consumption rates (OCRs) were assessed on a Seahorse XF96^{®} bioenergetic analyzer using the Mito Stress^{®} Kit following manufacturer's instructions (Agilent Technologies^{®}). Briefly, 40,000 cells were plated and left to adhere for 24 h before treatment with the indicated doses DXR. Basal mitochondrial oxygen consumption (mtOCR) was identified as the portion of OCR sensitive to 0.5 µM of Complex I inhibitor rotenone together with 0.5 µM of Complex III inhibitor antimycin A. Proton leak corresponded to the portion of mtOCR resistant to 1 µM of ATP synthase inhibitor oligomycin. Maximal OCR was determined after mitochondrial potential disruption using 1 µM of ionophore carbonyl cyanide-4-(trifluoromethoxy)phenylhydrazone (FCCP). At the end of the experiment, cells were lysed in 30 µl of NaOH 0.5 M, and proteins were quantified with the Bradford method in order to normalize data by total protein content. Lactate and glucose concentrations were measured on deproteinized cell supernatants using an ISCUSflex CMA600^{™} analyzer (Aurora Borealis^{®}), as previously described (Sonveaux et al.; PLoS. One 2012;7, e33418).

### 1.3. Mitochondrial superoxide measurements

Mitochondrial superoxide was measured in cells loaded with 3 µM of MitoSOX (Invitrogen^{®}, catalogue #M36008) for 10 min at 37°C. Cells were gently detached with accutase^{®} (Thermo Fisher Scientific^{®}, catalogue #A1110501), resuspended in PBS containing 10 mM of D-Glucose and 2% FBS, and immediately analyzed by flow cytometry using a FACSCantoII flow cytometer (BD Biosciences^{®}).

### 1.4. Cell viability

Cells were plated in 96-well plates and left to adhere for 16 hours. In order to avoid reaching full confluence at the endpoint, starting cell count was defined according to doubling time and the duration of the experiment. Following adhesion, cells were treated with the indicated compounds for 48 h, after which staining was done with a CellTiter-Glo Luminescent Cell Viability assay (Promega^{®}, catalogue #G7570), according to supplier's instructions. This method is based on the quantification of intracellular ATP levels.

Alternatively, following adhesion, cells were treated with the indicated compounds for 24 hours, and stained with 100 µl of a 0.23% crystal violet solution (Sigma-Aldrich^{®}, #C0775) for 10 min. The solution was carefully removed and plates were washed twice with water. After drying protected by light, cells were resuspended in 50 µl DMSO and the plates were read with a Victor X4 plate reader (Perkin^{®}).

### 1.5. Cell migration and invasion

Previous to migration and invasion assays, cells were pretreated with the indicated amounts of drugs for 16 h, and left to recover for 6 h in full culture medium. Migration assays were performed in a NeuroProbe^{®} standard 48-well chemotaxis chamber according to manufacturer's instructions. Briefly, the bottom chamber was filled with DMEM containing 0.5% serum as a chemo-attractant. Fifty thousand cells were seeded in the upper chamber in the same medium but without serum ± MitoQ where indicated, and allowed to migrate overnight through an 8-µm pore-size polycarbonate membrane. A similar stimulation protocol was used for invasion assays, with the difference of using Matrigel-coated transwells (Corning^{®}). With MCF7 cells, membranes were previously incubated for 30 min with a solution of Vitronectin 10 µg/ml (Sigma-Aldrich^{®}, #SRP3186). Migrated/invaded cells were fixed and stained with 100 µl of a 0.23% crystal violet solution (Sigma-Aldrich^{®}, #C0775) for 10 min before counting on a SpectraMax^{™} i3 spectrophotometer equipped with a MiniMax imaging cytometer, using the SoftMax Pro software. Data were normalized to vehicle-treated cells (control).

### 1.6. In vivo assays

*In vivo* experiments were conducted under approval of the Université catholique de Louvain (UCL) authorities (Comité d'Ethique Facultaire pour l'Expérimentation Animale) according to national animal care regulations. Specific approval IDs for this study were UCL/MD/2010/11 and 2016/UCL/MD/018.

On day -8, nine-week-old female Balb/cJRj mice (Janvier^{®}) were injected with 200,000 viable 4T1 cells into the fourth mammary fat pad. When tumors reached an average diameter of 4 mm (day 0) and became palpable in all mice, an intravenous injection of doxorubicin (4 mg/kg) was administered once per week during 3 weeks (30) ± daily intraperitoneal injections of MitoTEMPO (0.7 mg/kg) or the same volume of vehicle (DMSO) until the end of the experiment. Tumor volume was determined three times a week using an electronic caliper and normalized to tumor volume at day 0. At the end-point (day +21), mice were sacrificed and their lungs were fixed in formalin. Tissues were stained with hematoxylin & eosin (H&E). Pictures of whole lung slides were acquired with a slide scanner (SCN400, Leica^{®}) and analyzed with the Digital Image Hub software (DIH, Leica^{®}). The area occupied by metastases was normalized to the lung area analyzed.

### 1.7. Statistics

All data are shown as means ± SEM for n independent observations. Error bars are sometimes smaller than symbols. Outliers were identified using Dixon's Q test. Data were analyzed using GraphPad Prism. One-way ANOVA with Dunnett's post-hoc test and two-way ANOVA with Sidak's post-hoc test were used as indicated. P < 0.05 was considered to be statistically significant.

### 2. Results

In previous examples, it was shown that MitoQ can prevent cell migration, invasion and metastasis dependent on mitochondrial superoxide. Here, it was further hypothesized that chemotherapy could induce these phenotypic traits. Accordingly, it was observed that doxorubicin (DXR), a chemotherapeutic agent used for breast cancer treatment in the clinics, increased the mitochondrial oxygen consumption rate (mtOCR) of human MCF7 breast cancer cells when used at low doses (0.1 to 0.01 µg/mL, **Figure 20A-B****).** Within this range, DXR had little effect on glycolysis, still significantly increasing glucose uptake **(****Figure 20C****).** Low-dose DXR also increased mtOCR in murine 4T1 cells **(****Figure 21A-B****).** At 0.1 µg/mL, DXR had little effect on glycolysis, still significantly decreasing lactate release **(****Figure 21C****).**

Interestingly, DXR also induced a dose dependent leak of protons at the electron transport chain (ETC) of both human MCF and murine 4T1 cancer cell lines **(****Figure 22A-B****).** At a dose of DXR of 0.1 µg/mL, it went along with an increased production of mitochondrial superoxide at the ETC of both cancer cell lines, as measured using mitochondria-targeted superoxide-selective probe mitoSOX in FACS assays **(****Figure 23A-B****).**

In cell survival assays using the CellTiter-Glo reporter, MitoQ did not interfere with DXR-induced cell killing **(****Figure 24A-B****).** At low dose, DXR stimulated MCF7 and 4T1 cell migration **(****Figure 24C-D****)** as well as 4T1 cell invasion **(****Figure 24E****),** and these phenotypes were inhibited by 100 nM of MitoQ **(****Figure 24C-E****).**

Together, these results demonstrate that increased cancer cell migration and invasion are side effects of DXR chemotherapy, and that these deleterious side effects can be inhibited by MitoQ. It was therefore hypothesized that MitoQ can inhibit cancer metastasis in breast cancer-bearing animals treated with DXR, which was demonstrated in **Figure 25A-D** with another mitochondria-targeted superoxide scavenger, MitoTEMPO.

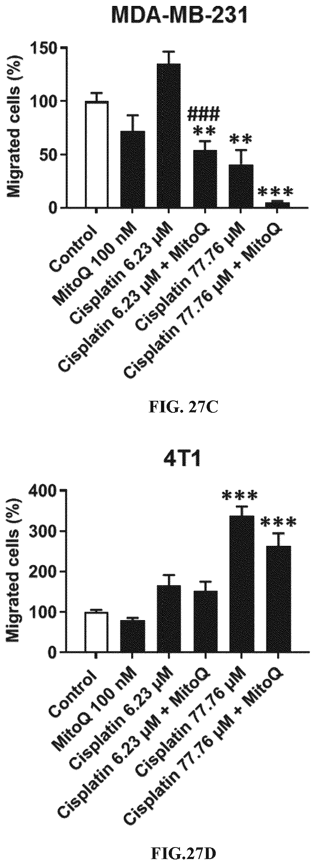

It was next hypothesized that the above observations could be shared by other types of chemotherapies. For the demonstration, cisplatin was chosen, which is another chemotherapeutical agent used for breast cancer treatment. It was found that, similar to DXR, cisplatin can induce mitochondrial superoxide production at the ETC of human MDA-MB-231 breast cancer cell line and murine 4T1 breast cancer cell line **(****Figure 26A-B****).** Combining cisplatin with MitoQ did not interfere with the cancer cell killing activity of MitoQ using a cell viability assay based on crystal violet incorporation **(****Figure 27A-B****).** Interestingly, cisplatin induced cancer cell migration, for example at a dose of 77.76 µM in 4T1 cells **(****Figure 27D****).** In the presence of cisplatin, MitoQ repressed breast cancer cell migration **(****Figure 27C-D****).**

Together, these results indicate that one can anticipate that increased cancer cell migration, invasion and metastasis are common side effects of different types of chemotherapeutic drugs, and that these side effects may also be countered by using MitoQ or an analogue thereof.

### Example 3

### 1. Materials and methods

### 1.1. Chemicals

[10-(4,5-dimethyl-3,6-dioxo-1,4-cyclohexadien-1-yl)decyl]triphenylphosphonium, monobromide (SKQ1 bromide; also known as Visomitin^{®}) (Selleck Chemicals^{®}, cat #S9729) was dissolved in DMSO at a stock concentration of 10 mM. Unless stated otherwise, all other chemicals were from Sigma-Aldrich^{®}. Equal volumes of solvent (DMSO) were used in control experiments.

### 1.2. Cells and cell culture

Human triple-negative MDA-MB-231 breast adenocarcinoma cancer cells were from Caliper^{®} (cat# 119369). Human HER2+ SkBr3 breast adenocarcinoma cancer cells (ATCC^{®} HTB-30^{™}), human triple-negative MDA-MB-436 breast adenocarcinoma cancer cells (ATCC^{®} HTB-130^{™}) and human MCF10A nonmalignant breast epithelialcells were from ATCC (ATCC^{®} CRL-10317^{™}). All cancer cell lines were originally derived from pleural effusions. MDA-MB-231 and SkBr3 cells were routinely cultured in DMEM containing 4.5 g/L glucose and GlutaMaxTM (Gibco^{®}, cat# 10566016) with 10% FBS; MDA-MB-436 in IMDM containing GlutaMaxTM (Gibco^{®}, cat# 31980030) with 20% FBS; and MCF10A in DMEM:F-12 (Gibco^{®}, cat# 11320033) with 5% horse serum, 1 mM CaCl₂, 10 mM HEPES, 10 µg/mL insulin, 20 ng/mL epithelial growth factor 25 (EGF) and 0.5 µg/mL hydrocortisone. All cell cultures were routinely maintained at a sub-confluent state in a humidified atmosphere (air) with 5% CO₂, 37°C. Cell authenticities were routinely verified with a short tandem repeat (STR) test (Eurofins Genomics).

### 1.3. Real-time quantitative PCR

Total mRNA from MDA-MB-231 or SkBr-3 human breast cancer cells treated ± 500 nM SKQ1 for 48 h (Alexandr V. et al. Int. J. Cancer 2016;139, 130-139) was extracted using the Tri reagent (Brunschwig Chemie^{®}, cat #TR118). mRNAs were quantified by NanoDrop TM 1000 (Thermo Fisher Scientific^{®}), and were reverse-transcribed in complementary DNAs using the high-capacity cDNA Reverse Transcription Kit (Thermo Fisher Scientific^{®}, cat #4368814) according to manufacturer's protocol. Complementary DNAs (500 ng per sample) were amplified by RT-qPCR using the ROX SYBR Master Mix dTTP Blue (Eurogentec^{®}) and primers listed in **Table 5** and **Table 6** on a CFX96 Real Time PCR system (BioRad^{®}). Gene expression was normalized to β-actin gene expression.

### 1.4. Statistics

All data are shown as means ± SEM for n independent observations. Error bars are sometimes smaller than symbols. Outliers were identified using Dixon's Q test. Data were analyzed using GraphPad Prism. Student t test was used in all experiments. P < 0.05 was considered to be statistically significant.

### 2. Results

Finally, it was tested whether the biomarker signature of response to MitoQ identified above could be used to assess the response of human breast cancer cells MDA-MB-231, SkBR3 and MDAMB-436 to other mitochondria-targeted antioxidants. SKQ1, an analogue of MitoQ, was chosen.

Similar to MitoQ, SKQ1 comprises an antioxidant moiety (in this case plastoquinone) coupled to a mitochondria-targeting triphenylphosphonium cation with a decane linker. It was used at a concentration of 500 nM according to Alexandr V et al. (Alexandr V. et al. Int. J. Cancer 2016;139, 130-139) and, as for MitoQ, cell responses were detected by using RT-qPCR 48 h after the treatment.

**Figure 28A-O** reports on the expression on the expression of EMT-related genes (VIM, SNAI1, ZEB1; **Figure 28A-I****)** and stemness related genes (NANOG, SOX2; **Figure 28J-O**), for which a decrease in gene expression in response to SKQ1 was expected. **Figures 29A-O** **and** **30A-O** reports on the expression of the other genes (PHGDH-1, SLC7A11, SERPINE1, TXNRD1, PSAT1, PCK2, G6PD, ASNS, SLC6A9 and NUPR1). Similar to assays with MitoQ, the expression of this second panel of genes was expected to increase in response to SKQ1.

The core biomarker signature determined using MitoQ comprised SLC7A11, SERPINE1, and PSAT1. As expected, the expression of all 3 genes was increased in all 3 cell lines following a 48 h treatment with 500 nM of SKQ1 **(****Figure 29D-F****,** **Figure 29G-I** and **Figure 29M-O****).** In MDA-MB-231 cell, SLC7A11 expression was increased by 2.35-fold (P < 0.005), SERPINE1 expression by 1.63-fold (P < 0.01) and PSAT1 expression by 3.43-fold (P < 0.005). In SkBR3 cells, SLC7A11 expression was increased by 6.65-fold (P < 0.005), SERPINE1 expression by 1.45-fold (P = 0.0732) and PSAT1 expression by 2.26-fold (P < 0.01). In MDA-MB-436 cells, SLC7A11 expression was increased by 3.60-fold (P < 0.01), SERPINE1 expression by 1.46-fold (P < 0.01) and PSAT1 expression by 2.53-fold (P < 0.005). These changes validated the capability of this set of genes to evidence a molecular response to SKQ1 in human breast cancer cells.

The extended biomarker signature determined using MitoQ comprised SLC7A11, SERPINE1, PSAT1, PHGDH-1, and TXNRD1. As expected, the expression of both PHGDH-1PHGDH1 and TXNRD1 was increased in all 3 cell lines following a 48 h treatment with 500 nM of SKQ1 **(****Figure 29A-C** and **Figure 29J-L****).** PHGDH-1PHGDH1 and TXNRD1 expression was increased by 5.36-fold (P < 0.005) and 1.69-fold (P < 0.005) in MDA-MB-231 cells, respectively; by 2.26-fold (P < 0.01) and 3.20-fold (P < 0.005) in SkBR3 cells (P < 0.005), respectively; and by 2.51 (P < 0.005) fold and 2.11-fold (P < 0.005) in MDA-MB-436 cells, respectively. These changes validated the capability of this extended set of genes to evidence a molecular response to SKQ1 in human breast cancer cells.

The full biomarker signature determined using MitoQ comprised SLC7A11, SERPINE1, PSAT1, PHGDH-1, TXNRD1, VIM, SNAI1, ZEB1, NANOG, SOX2, PCK2, G6PD, ASNS, NUPR1, and SLC6A9. In addition to the above results, among expected responses, decreased gene expression of *SNAI1* was detected (although not significant, P = 0.4575) **(****Figure 28D-F****),** SOX2 (P < 0.005) (**Figure 28M-O**) as well as increased gene expression of PCK2 (P < 0.005) **(****Figure 30A-C****),** G6PD (P < 0.005) **(****Figure 30D-F****),** ASNS (P < 0.005) **(****Figure 29G-I****),** SLC6A9 (P < 0.005) **(****Figure 29J-****L)** and NUPR1 (P < 0.005) (**Figure 29M-O**) in MDA-MB-231 cells. In SkBR3 cells, decreased gene expression of VIM was detected (although not significant, P = 0.3557) **(****Figure 28A-B****),** ZEB1 (P < 0.05) **(****Figure 28G-I****),** NANOG (although not significant, P = 0.1539) **(****Figure 28J-L****)** and SOX2 (P < 0.005), as well as increased gene expression of PCK2 (P < 0.005), G6PD, ASNS (P < 0.01), and NUPR1 (although not significant, P = 0.2615). In MDA-MB-436 cells, decreased gene expression of SNAI1 was detected (although not significant, P = 0.6013), ZEB1 (although not significant, P = 0.6890), NANOG (although not significant, P = 0.5663) and SOX2 (P < 0.05), as well as increased gene expression of PCK2 (P < 0.005), G6PD (although not significant, P = 0.0974), ASNS (P < 0.005), SLC6A9 (P < 0.005) and NUPR1 (P < 0.005).

### 3. Conclusions

Overall, the sets of experiments of **Example 3** demonstrate that the biomarker signature comprising the gene expression of SLC7A11, SERPINE1, PSAT1, PHGDH-1, TXNRD1, VIM, SNAI1, ZEB1, NANOG, SOX2, PCK2, G6PD, ASNS, NUPR1, and SLC6A9 is valid to determine the response of human breast cancer cells to SKQ1, *i.e.,* another mitochondria-targeted antioxidant than MitoQ.

With this example, it can therefore be anticipated that this biomarker signature is valid for any other mitochondria-targeted antioxidants than MitoQ and SKQ1.

In support of this conclusion, for MDA-MB-231 cells, SKQ1 elicited the expected gene expression changes in 12 of the 15 genes of the complete biomarker signature. Among expected changes in the expression of these 12 genes, 11 were statistically significant. For the 12 genes, the amplitude of the changes varied from -21% (SNAI1) to -52% (SOX2) for genes whose expression was expected to decrease, and from +63% (SERPIN1) to +676% (NUPR1) for genes whose expression was expected to increase.

In further support of this conclusion, for SkBR3 cells, SKQ1 elicited the expected gene expression changes in 13 of the 15 genes of the complete biomarker signature. Among expected changes in the expression of these 13 genes, 8 were statistically significant and 1 (G6PD) comprised only 2 replicates, preventing final conclusion with respect to statistics. For the 13 genes, the amplitude of the changes varied from -19% (VIM) to -95% (SOX2) for genes whose expression was expected to decrease, and from +25% (NUPR1) to +565% (SLC7A11) for genes whose expression was expected to increase.

Even in further support of this conclusion, for MDA-MB-436 cells, SKQ1 elicited the expected gene expression changes in 14 of the 15 genes of the complete biomarker signature. Among expected changes in the expression of these 14 genes, 10 were statistically significant. For the 14 genes, the amplitude of the changes varied from -12% (SNAI1) to -67% (SOX2) for genes whose expression was expected to decrease, and from +22% (G6PD) to +526% (ASNS) for genes whose expression was expected to increase.

The core version of the biomarker signature (SLC7A11, SERPIN1 and PSAT1) was very robust, providing 100% of the expected results, and 8 of the 9 tests provided statistically significant data.

The extended version of the biomarker signature (SLC7A11, SERPINE1, PSAT1, PHGDH-1 and TXNRD1) was very robust too, providing 100% of the expected results, and 14 of the 15 tests provided statistically significant data.

The complete biomarker signature (SLC7A11, SERPINE1, PSAT1, PHGDH-1, TXNRD1, VIM, SNAI1, ZEB1, NANOG, SOX2, PCK2, G6PD, ASNS, NUPR1, and SLC6A9) was less robust, providing 87% (39/45) of the expected results, and 29 of the 39 successful tests (74%) provided statistically significant data.

Of important note, the complete biomarker signature has important added value, as gene expression changes did not always affect the same panel of genes in the 3 tested human breast cancer cells lines, and the extremes with respect to the amplitude of the changes most often affected different genes in the different cell lines.

Overall, based on the results of **Example 3,** it can be reasonably concluded in the validity and usefulness of the complete biomarker signature to detect a response of human breast cancer cells to mitochondria-targeted antioxidants.

## Claims

1. A method for identifying an individual with cancer, in particular breast cancer, as being susceptible to respond to a treatment by at least one mitochondria-targeted antioxidant, the method comprising:
a. assessing the expression levels of at least three biomarkers selected from the group consisting of SLC7A11, SERPINE1, PSAT1, PHGDH-1, TXNRD1, VIM, SNAI1, ZEB1, NANOG, SOX2, PCK2, G6PD, ASNS, NUPR1 and SLC6A9, in samples obtained from said individual before and after treatment with said at least one mitochondria-targeted antioxidant; and
b. comparing the expression levels of the at least three biomarkers in the samples obtained after treatment with said at least one mitochondria-targeted antioxidant with their respective reference levels in the samples obtained before treatment with said at least one mitochondria-targeted antioxidant;
wherein a significant variation of the expression levels of at least three biomarkers as compared to their respective reference levels represents a molecular signature indicative of the individual with cancer, in particular breast cancer, as being susceptible to respond to a treatment by the at least one mitochondria-targeted antioxidant.

2. The method according to claim 1, wherein the significant variation comprises a variation of at least 1.2-fold of the expression levels of at least three biomarkers as compared to their respective reference levels, and optionally a statistical relevance of P < 0.05.

3. The method according to any one of claim **1** or **2,** wherein the at least three biomarkers include SLC7A11, and/or SERPINE1 and/or PSAT1, preferably SLC7A11, SERPINE1 and PSAT1.

4. The method according to any one of claims **1** to **3,** wherein the at least three biomarkers include SLC7A11, and/or SERPINE1, and/or PSAT1, and/or PHGDH-1 and/or TXNRD1, preferably SLC7A11, SERPINE1, PSAT1, PHGDH-1 and TXNRD 1.

5. The method according to any one of claims **1 to 4,** wherein the biomarkers are SLC7A11, SERPINE1, PSAT1, PHGDH-1, TXNRD1, VIM, SNAI1, ZEB1, NANOG, SOX2, PCK2, G6PD, ASNS, NUPR1 and SLC6A9.

6. The method according to any one of claims **1** to **5,** wherein the cancer is a metastatic cancer or a cancer susceptible to undergo metastasis, in particular a metastatic breast cancer or a breast cancer susceptible to undergo metastasis.

7. The method according to any one of claims **1** to **6,** wherein the at least one mitochondria-targeted antioxidant is selected from the group consisting of MitoQ, MitoTEMPO, MitoTEMPOL, MitoE, MitoVitE, MitoSOD, MitoSNO, SKQ1, SKQR1, SKQ2, SKQ3, SKQ4, SKQ5, SKQBerb, SKQPalm, C12TPP, melatonin, dimethyl malonate, methylene blue, Mn-porphyrin-oligopeptide conjugate, M40401, SS20, SS31, XJB-5-125, XJB-5-131 and XJB-5-197, preferably the at least one mitochondria-targeted antioxidant is MitoQ or SKQ1 or a functional derivative thereof.

8. Use of the expression levels of at least three biomarkers selected from the group consisting of SLC7A11, SERPINE1, PSAT1, PHGDH-1, TXNRD1, VIM, SNAI1, ZEB1, NANOG, SOX2, PCK2, G6PD, ASNS, NUPR1 and SLC6A9 as a molecular signature for identifying an individual with cancer, in particular breast cancer, as being susceptible to respond to a treatment by at least one mitochondria-targeted antioxidant.

9. The use according to claim **8,** wherein the molecular signature comprises:
a. at least three biomarkers including SLC7A11, and/or SERPINE1, and/or PSAT1, preferably biomarkers SLC7A11, SERPINE1 and PSAT1; or
b. at least three biomarkers including SLC7A11, and/or SERPINE1, and/or, PSAT1 and/or PHGDH-1 and/or TXNRD1, preferably biomarker SLC7A11, SERPINE1, PSAT1, PHGDH-1and TXNRD1; or
c. biomarkers being SLC7A11, SERPINE1, PSAT1, PHGDH-1, TXNRD1, VIM, SNAI1, ZEB 1, NANOG, SOX2, PCK2, G6PD, ASNS, NUPR1 and SLC6A9.

10. The use according to claim **8** or **9,** wherein the expression levels of the at least three biomarkers are compared to their respective reference levels, and wherein a significant variation of the expression levels of the at least three biomarkers as compared to their respective reference levels is indicative of the individual with cancer being susceptible to respond to a treatment by the at least one mitochondria-targeted antioxidant.

11. The use according to any one of claims **8** to **10,** wherein the cancer is a metastatic cancer or a cancer susceptible to undergo metastasis, in particular a metastatic breast cancer or a breast cancer susceptible to undergo metastasis.

12. A mitochondria-targeted antioxidant for use in a method for preventing and/or treating cancer, in particular breast cancer, in an individual identified by the method according to any one of claims **1** to **7.**

13. A mitochondria-targeted antioxidant for use in a method for preventing and/or treating metastasis of cancer, in particular breast cancer, in an individual identified by the method according to any one of claims **1** to **7.**

14. A mitochondria-targeted antioxidant for use in a method for preventing and/or treating cancer recurrence before, concomitantly or after a surgery intended to remove all or part of the tumor, in particular breast cancer recurrence, in an individual identified by the method according to any one of claims **1** to **7.**

15. The mitochondria-targeted antioxidant for use according to any one of claims **12** to **14,** wherein the antioxidant is further combined with another cancer treatment, preferably selected in the group consisting of chemotherapy, radiotherapy, hormonal therapy, immunotherapy, anti-angiogenic therapy, a surgery intended to remove all or part of the tumor, in particular the breast tumor, at least another mitochondria-targeted antioxidant, and any combination thereof.

## Patentansprüche

1. Verfahren zur Identifizierung eines Individuums mit Krebs, insbesondere Brustkrebs, als anfällig für das Ansprechen auf eine Behandlung mit mindestens einem mitochondrienzielenden Antioxidans, wobei das Verfahren umfasst:
a. die Bestimmung der Expressionsniveaus von mindestens drei Biomarkern, ausgewählt aus der Gruppe bestehend aus SLC7A11, SERPINE1, PSAT1, PHGDH-1, TXNRD1, VIM, SNAI1, ZEB1, NANOG, SOX2, PCK2, G6PD, ASNS, NUPR1 und SLC6A9, in Proben, die von dem Individuum vor und nach der Behandlung mit dem mindestens einen mitochondrienzielenden Antioxidans gewonnen wurden; und
b. den Vergleich der Expressionsniveaus der mindestens drei Biomarker in den nach der Behandlung mit dem mindestens einen mitochondrienzielenden Antioxidans gewonnenen Proben mit ihren jeweiligen Referenzniveaus in den vor der Behandlung mit dem mindestens einen mitochondrienzielenden Antioxidans gewonnenen Proben;
wobei eine signifikante Variation der Expressionsniveaus von mindestens drei Biomarkern im Vergleich zu ihren jeweiligen Referenzniveaus eine molekulare Signatur darstellt, die darauf hinweist, dass das Individuum mit Krebs, insbesondere Brustkrebs, anfällig für das Ansprechen auf eine Behandlung mit dem mindestens einen mitochondrienzielenden Antioxidans ist.

2. Verfahren nach Anspruch 1, wobei die signifikante Variation eine Variation von mindestens dem 1,2-Fachen der Expressionsniveaus von mindestens drei Biomarkern im Vergleich zu ihren jeweiligen Referenzniveaus und gegebenenfalls eine statistische Relevanz von P < 0,05 umfasst.

3. Verfahren nach einem der Ansprüche 1 oder 2, wobei die mindestens drei Biomarker SLC7A11 und/oder SERPINE1 und/oder PSAT1, vorzugsweise SLC7A11, SERPINE1 und PSAT1, umfassen.

4. Verfahren nach einem der Ansprüche 1 bis 3, wobei die mindestens drei Biomarker SLC7A11 und/oder SERPINE1 und/oder PSAT1 und/oder PHGDH-1 und/oder TXNRD1 umfassen, vorzugsweise SLC7A11, SERPINE1, PSAT1, PHGDH-1 und TXNRD1.

5. Verfahren nach einem der Ansprüche 1 bis 4, wobei die Biomarker SLC7A11, SERPINE1, PSAT1, PHGDH-1, TXNRD1, VIM, SNAI1, ZEB1, NANOG, SOX2, PCK2, G6PD, ASNS, NUPR1 und SLC6A9 sind.

6. Verfahren nach einem der Ansprüche 1 bis 5, wobei der Krebs ein metastasierender Krebs oder ein Krebs ist, der anfällig dafür ist, eine Metastasierung zu durchlaufen, insbesondere ein metastasierender Brustkrebs oder ein Brustkrebs, der anfällig dafür ist, eine Metastasierung zu durchlaufen.

7. Verfahren nach einem der Ansprüche 1 bis 6, wobei das mindestens eine mitochondrienzielende Antioxidans ausgewählt ist aus der Gruppe bestehend aus MitoQ, MitoTEMPO, MitoTEMPOL, MitoE, MitoVitE, MitoSOD, MitoSNO, SKQ1, SKQR1, SKQ2, SKQ3, SKQ4, SKQ5, SKQBerb, SKQPalm, C12TPP, Melatonin, Dimethylmalonat, Methylenblau, Mn-Porphyrin-Oligopeptid-Konjugat, M40401, SS20, SS31, XJB-5-125, XJB-5-131 und XJB-5-197, vorzugsweise ist das mindestens eine mitochondrienzielende Antioxidans MitoQ oder SKQ1 oder ein funktionelles Derivat davon.

8. Verwendung der Expressionsniveaus von mindestens drei Biomarkern, ausgewählt aus der Gruppe bestehend aus SLC7A11, SERPINE1, PSAT1, PHGDH-1, TXNRD1, VIM, SNAI1, ZEB1, NANOG, SOX2, PCK2, G6PD, ASNS, NUPR1 und SLC6A9, als molekulare Signatur zur Identifizierung eines Individuums mit Krebs, insbesondere Brustkrebs, als anfällig dafür, auf eine Behandlung mit mindestens einem mitochondrienzielenden Antioxidans zu reagieren.

9. Verwendung nach Anspruch 8, wobei die molekulare Signatur umfasst:
a. mindestens drei Biomarker, die SLC7A11 und/oder SERPINE1 und/oder PSAT1 umfassen, vorzugsweise Biomarker SLC7A11, SERPINE1 und PSAT1; oder
b. mindestens drei Biomarker, die SLC7A11 und/oder SERPINE1 und/oder, PSAT1 und/oder PHGDH-1 und/oder TXNRD1 umfassen, vorzugsweise Biomarker SLC7A11, SERPINE1, PSAT1, PHGDH-1 und TXNRD1; oder
c. Biomarker, die SLC7A11, SERPINE1, PSAT1, PHGDH-1, TXNRD1, VIM, SNAI1, ZEB1, NANOG, SOX2, PCK2, G6PD, ASNS, NUPR1 und SLC6A9 sind.

10. Verwendung nach Anspruch 8 oder 9, wobei die Expressionsniveaus der mindestens drei Biomarker mit ihren jeweiligen Referenzniveaus verglichen werden, und wobei eine signifikante Variation der Expressionsniveaus der mindestens drei Biomarker im Vergleich zu ihren jeweiligen Referenzniveaus darauf hinweist, dass das Individuum mit Krebs anfällig ist, auf eine Behandlung mit dem mindestens einen mitochondrienzielenden Antioxidans zu reagieren.

11. Verwendung nach einem der Ansprüche 8 bis 10, wobei der Krebs ein metastasierender Krebs oder ein Krebs ist, der anfällig dafür ist, eine Metastasierung zu durchlaufen, insbesondere ein metastasierender Brustkrebs oder ein Brustkrebs, der anfällig dafür ist, eine Metastasierung zu durchlaufen.

12. Ein mitochondrienzielendes Antioxidans zur Verwendung in einem Verfahren zur Vorbeugung und/oder Behandlung von Krebs, insbesondere Brustkrebs, bei einem Individuum, das durch das Verfahren nach einem der Ansprüche 1 bis 7 identifiziert wurde.

13. Ein mitochondrienzielendes Antioxidans zur Verwendung in einem Verfahren zur Vorbeugung und/oder Behandlung von Metastasierung von Krebs, insbesondere Brustkrebs, bei einem Individuum, das durch das Verfahren nach einem der Ansprüche 1 bis 7 identifiziert wurde.

14. Ein mitochondrienzielendes Antioxidans zur Verwendung in einem Verfahren zur Vorbeugung und/oder Behandlung von Krebsrezidiv vor, gleichzeitig mit oder nach einer Operation, die darauf abzielt, den gesamten oder einen Teil des Tumors zu entfernen, insbesondere Brustkrebsrezidiv, bei einem Individuum, das durch das Verfahren nach einem der Ansprüche 1 bis 7 identifiziert wurde.

15. Das mitochondrienzielende Antioxidans zur Verwendung nach einem der Ansprüche 12 bis 14, wobei das Antioxidans ferner mit einer anderen Krebsbehandlung kombiniert wird, vorzugsweise ausgewählt aus der Gruppe bestehend aus Chemotherapie, Radiotherapie, hormoneller Therapie, Immuntherapie, antiangiogener Therapie, einer Operation, die darauf abzielt, den gesamten oder einen Teil des Tumors zu entfernen, insbesondere den Brusttumor, mindestens einem weiteren mitochondrienzielenden Antioxidans und jeder Kombination davon.

## Revendications

1. Procédé d'identification d'un individu atteint de cancer, en particulier de cancer du sein, comme étant susceptible de répondre à un traitement par au moins un antioxydant ciblant les mitochondries, le procédé comprenant :
a. l'évaluation des niveaux d'expression d'au moins trois biomarqueurs sélectionnés dans le groupe constitué de SLC7A11, SERPINE1, PSAT1, PHGDH-1, TXNRD1, VIM, SNAI1, ZEB1, NANOG, SOX2, PCK2, G6PD, ASNS, NUPR1 et SLC6A9, dans des échantillons obtenus à partir dudit individu avant et après traitement par ledit au moins un antioxydant ciblant les mitochondries ; et
b. la comparaison des niveaux d'expression des au moins trois biomarqueurs dans les échantillons obtenus après traitement par ledit au moins un antioxydant ciblant les mitochondries avec leurs niveaux de référence respectifs dans les échantillons obtenus avant traitement par ledit au moins un antioxydant ciblant les mitochondries ;
dans lequel une variation significative des niveaux d'expression d'au moins trois biomarqueurs par rapport à leurs niveaux de référence respectifs représente une signature moléculaire indicative de l'individu atteint de cancer, en particulier de cancer du sein, comme étant susceptible de répondre à un traitement par l'au moins un antioxydant ciblant les mitochondries.

2. Procédé selon la revendication 1, dans lequel la variation significative comprend une variation d'au moins 1,2 fois des niveaux d'expression d'au moins trois biomarqueurs par rapport à leurs niveaux de référence respectifs, et éventuellement une pertinence statistique de P < 0,05.

3. Procédé selon l'une quelconque des revendications 1 ou 2, dans lequel les au moins trois biomarqueurs incluent SLC7A11, et/ou SERPINE1 et/ou PSAT1, de préférence SLC7A11, SERPINE1 et PSAT1.

4. Procédé selon l'une quelconque des revendications 1 à 3, dans lequel les au moins trois biomarqueurs incluent SLC7A11, et/ou SERPINE1, et/ou PSAT1, et/ou PHGDH-1 et/ou TXNRD1, de préférence SLC7A11, SERPINE1, PSAT1, PHGDH-1 et TXNRD1.

5. Procédé selon l'une quelconque des revendications 1 à 4, dans lequel les biomarqueurs sont SLC7A11, SERPINE1, PSAT1, PHGDH-1, TXNRD1, VIM, SNAI1, ZEB1, NANOG, SOX2, PCK2, G6PD, ASNS, NUPR1 et SLC6A9.

6. Procédé selon l'une quelconque des revendications 1 à 5, dans lequel le cancer est un cancer métastatique ou un cancer susceptible de subir une métastase, en particulier un cancer du sein métastatique ou un cancer du sein susceptible de subir une métastase.

7. Procédé selon l'une quelconque des revendications 1 à 6, dans lequel l'au moins un antioxydant ciblant les mitochondries est sélectionné dans le groupe constitué de MitoQ, MitoTEMPO, MitoTEMPOL, MitoE, MitoVitE, MitoSOD, MitoSNO, SKQ1, SKQR1, SKQ2, SKQ3, SKQ4, SKQ5, SKQBerb, SKQPalm, C12TPP, mélatonine, malonate de diméthyle, bleu de méthylène, conjugué Mn-porphyrine-oligopeptide, M40401, SS20, SS31, XJB-5-125, XJB-5-131 et XJB-5-197, de préférence l'au moins un antioxydant ciblant les mitochondries est MitoQ ou SKQ1 ou un dérivé fonctionnel de ceux-ci.

8. Utilisation des niveaux d'expression d'au moins trois biomarqueurs sélectionnés dans le groupe constitué de SLC7A11, SERPINE1, PSAT1, PHGDH-1, TXNRD1, VIM, SNAI1, ZEB1, NANOG, SOX2, PCK2, G6PD, ASNS, NUPR1 et SLC6A9 en tant que signature moléculaire pour identifier un individu atteint de cancer, en particulier de cancer du sein, comme étant susceptible de répondre à un traitement par au moins un antioxydant ciblant les mitochondries.

9. Utilisation selon la revendication 8, dans laquelle la signature moléculaire comprend :
a. au moins trois biomarqueurs incluant SLC7A11, et/ou SERPINE1, et/ou PSAT1, de préférence les biomarqueurs SLC7A11, SERPINE1 et PSAT1 ; ou
b. au moins trois biomarqueurs incluant SLC7A11, et/ou SERPINE1, et/ou, PSAT1 et/ou PHGDH-1 et/ou TXNRD1, de préférence le biomarqueur SLC7A11, SERPINE1, PSAT1, PHGDH-1 et TXNRD1 ; ou
c. des biomarqueurs étant SLC7A11, SERPINE1, PSAT1, PHGDH-1, TXNRD1, VIM, SNAI1, ZEB1, NANOG, SOX2, PCK2, G6PD, ASNS, NUPR1 et SLC6A9.

10. L'utilisation selon la revendication 8 ou 9, dans laquelle les niveaux d'expression des au moins trois biomarqueurs sont comparés à leurs niveaux de référence respectifs, et dans laquelle une variation significative des niveaux d'expression des au moins trois biomarqueurs par rapport à leurs niveaux de référence respectifs est indicative du fait que l'individu atteint de cancer est susceptible de répondre à un traitement par l'au moins un antioxydant ciblant les mitochondries.

11. L'utilisation selon l'une quelconque des revendications 8 à 10, dans laquelle le cancer est un cancer métastatique ou un cancer susceptible de subir une métastase, en particulier un cancer du sein métastatique ou un cancer du sein susceptible de subir une métastase.

12. Un antioxydant ciblant les mitochondries pour son utilisation dans un procédé de prévention et/ou de traitement du cancer, en particulier du cancer du sein, chez un individu identifié par le procédé selon l'une quelconque des revendications 1 à 7.

13. Un antioxydant ciblant les mitochondries pour son utilisation dans un procédé de prévention et/ou de traitement de la métastase du cancer, en particulier du cancer du sein, chez un individu identifié par le procédé selon l'une quelconque des revendications 1 à 7.

14. Un antioxydant ciblant les mitochondries pour son utilisation dans un procédé de prévention et/ou de traitement de la récidive du cancer avant, concomitamment ou après une chirurgie destinée à retirer tout ou partie de la tumeur, en particulier la récidive du cancer du sein, chez un individu identifié par le procédé selon l'une quelconque des revendications 1 à 7.

15. L'antioxydant ciblant les mitochondries pour son utilisation selon l'une quelconque des revendications 12 à 14, dans lequel l'antioxydant est en outre combiné avec un autre traitement du cancer, de préférence sélectionné dans le groupe constitué par la chimiothérapie, la radiothérapie, la thérapie hormonale, l'immunothérapie, la thérapie anti-angiogénique, une chirurgie destinée à retirer tout ou partie de la tumeur, en particulier la tumeur mammaire, au moins un autre antioxydant ciblant les mitochondries, et toute combinaison de ceux-ci.
